(19) Europäisches Patentamt
European Patent Office
Office européen des brevets

(11) EP 1 536 007 B1

(12) **EUROPEAN PATENT SPECIFICATION**

(45) Date of publication and mention of the grant of the patent:
**30.12.2015 Bulletin 2015/53**

(21) Application number: **03794154.9**

(22) Date of filing: **01.09.2003**

(51) Int Cl.:
*C12N 15/09* (2006.01) *C12N 15/85* (2006.01)
*C12N 5/10* (2006.01) *A61K 48/00* (2006.01)

(86) International application number:
**PCT/JP2003/011134**

(87) International publication number:
**WO 2004/022741 (18.03.2004 Gazette 2004/12)**

(54) **ARTIFICIAL MAMMALIAN CHROMOSOME**
KÜNSTLICHES SÄUGERCHROMOSOM
CHROMOSOME MAMMIFERE ARTIFICIEL

(84) Designated Contracting States:
**AT BE BG CH CY CZ DE DK EE ES FI FR GB GR HU IE IT LI LU MC NL PT RO SE SI SK TR**

(30) Priority: **03.09.2002 JP 2002258114**
**22.11.2002 JP 2002338865**

(43) Date of publication of application:
**01.06.2005 Bulletin 2005/22**

(73) Proprietor: **Japan Science and Technology Agency**
**Kawaguchi-shi,**
**Saitama 332-0012 (JP)**

(72) Inventors:
- **OKAZAKI, Tsuneko,**
**Room 804, Yagoto Family Heights**
**Nagoya-shi, Aichi 466-0815 (JP)**
- **IKENO, Masashi,**
**Room 602, Atre-Haraekimae**
**Nagoya-shi, Aichi 464-0015 (JP)**
- **ITOU, Toshihide,**
**Room 202, Cejuir Midori**
**Nagoya-shi, Aichi 458-0814 (JP)**
- **SUZUKI, Nobutaka**
**Nagoya-shi, Aichi 464-0848 (JP)**

(74) Representative: **Müller Fottner Steinecke Rechtsanwalts- und Patentanwaltspartnerschaft mbB**
**Römerstraße 16 b**
**52428 Jülich (DE)**

(56) References cited:
**WO-A-02/067665 US-A- 6 133 503**
**US-A1- 2002 076 811**

- **MEJIA J E ET AL: "The Assembly of Large BACs by in Vivo Recombination" GENOMICS, ACADEMIC PRESS, SAN DIEGO, US, vol. 70, no. 2, 1 December 2000 (2000-12-01), pages 165-170, XP004437749 ISSN: 0888-7543**
- **MORALLI D ET AL: "Insertion of a loxP site in a size-reduced human accessory chromosome" CYTOGENETICS AND CELL GENETICS, BASEL, CH, vol. 94, no. 3-4, 2001, pages 113-120, XP002956895 ISSN: 0301-0171**
- **KANAME T ET AL: "Simple and efficient vectors for retrofitting BACs and PACs with mammalian neo<R> and EGFP marker genes" GENE, ELSEVIER, AMSTERDAM, NL, vol. 266, no. 1-2, 21 March 2001 (2001-03-21), pages 147-153, XP004233103 ISSN: 0378-1119**
- **VOET THIERRY ET AL: "Efficient male and female germline transmission of a human chromosomal vector in mice" GENOME RESEARCH, vol. 11, no. 1, January 2001 (2001-01), pages 124-136, XP002355812 ISSN: 1088-9051**
- **LARIN Z ET AL: "Advances in human artificial chromosome technology" TRENDS IN GENETICS, ELSEVIER, AMSTERDAM, NL, vol. 18, no. 6, 1 June 2002 (2002-06-01), pages 313-319, XP004357968 ISSN: 0168-9525**

EP 1 536 007 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- PETERSON K R ET AL: "Production of transgenic mice with yeast artificial chromosomes" TRENDS IN GENETICS, ELSEVIER SCIENCE PUBLISHERS B.V. AMSTERDAM, NL, vol. 13, no. 2, February 1997 (1997-02), pages 61-66, XP004034151 ISSN: 0168-9525
- RIVAL S ET AL: "Cloning, transcription and chromosomal localization of the porcine whey acidic protein gene and its expression in HC11 cell line" GENE, ELSEVIER, AMSTERDAM, NL, vol. 267, no. 1, 4 April 2001 (2001-04-04), pages 37-47, XP004235017 ISSN: 0378-1119
- IKENO M ET AL: "Generation of human artificial chromosomes expressing naturally controlled guanosine triphosphate cyclohydrolase I gene" GENES TO CELLS, OXFORD, GB, vol. 7, no. 10, 2 October 2002 (2002-10-02), pages 1021-1032, XP002976596 ISSN: 1356-9597
- LIPPS H J ET AL: "Chromosome-based vectors for gene therapy" GENE: AN INTERNATIONAL JOURNAL ON GENES AND GENOMES, ELSEVIER, AMSTERDAM, NL, vol. 304, 30 January 2003 (2003-01-30), pages 23-33, XP004406349 ISSN: 0378-1119
- GRIMES B.R. ET AL.: 'Stable gene expression from a mammalian artificial chromosome' EMBO REP. vol. 2, no. 10, 2001, pages 910 - 914, XP002973962
- TSUNEKO OKAZAKI: 'Honyurui jinko senshokutai no kaihatsu to kotai no keishitsu tenkan eno riyo' SENRYAKUTEKI KISO KENKYU SUISHIN JIGYO KENKYU NENPO vol. 2000, March 2002, pages 61 - 64, XP002975686
- YOSHIMI KUROIWA ET AL.: 'Hito mini senshokutai vector o mochiita mega base (Mb) size no tokutei hito senshokutai ryoiki no cloning system no kaihatsu' EXPERIMENTAL MEDICINE vol. 19, no. 1, 2001, pages 50 - 52, XP002975687
- HASEGAWA K. ET AL.: 'Insulators prevent transcriptional interference between two promoters in a double gene construct for transgenesis' FEBS LETT. vol. 520, no. 1-3, June 2002, pages 47 - 52, XP004361052
- RIVELLA S. ET AL.: 'The cHS4 insulator increases the probability of retroviral expression at random chromosomal integration sites' J. VIROL. vol. 74, no. 10, 2000, pages 4679 - 4687, XP002249347
- SHIN'ICHI HORIIE ET AL.: 'Bisho kakusaibo yugoho o riyo shita senshokutai kogaku' SAIBO vol. 33, no. 3, 2001, pages 114 - 117, XP002975688
- TSUNEKO OKAZAKI: 'Honyurui jinko senshokutai no kochiku to sono riyo' SEIMEI KATSUDO NO PROGRAM, HEISEI 9 NENDO SAITAKU KENKYU KADAI SHURYO SYMPOSIUM KOEN YOKOSHU November 2002, pages 14 - 18, XP002975689
- CARPENTER M K ET AL: "CHARACTERIZATION AND DIFFERENTIATION OF HUMAN EMBRYONIC STEM CELLS", CLONING AND STEM CELLS, MARY ANN LIEBERT, LARCHMONT, US LNKD- DOI: 10.1089/153623003321512193, vol. 5, no. 1, 1 January 2003 (2003-01-01) , pages 79-88, XP008064757, ISSN: 1536-2302
- DRUKKER MICHA ET AL: "Characterization of the expression of MHC proteins in human embryonic stem cells.", PROCEEDINGS OF THE NATIONAL ACADEMY OF SCIENCES OF THE UNITED STATES OF AMERICA 23 JUL 2002 LNKD- PUBMED:12114532, vol. 99, no. 15, 23 July 2002 (2002-07-23) , pages 9864-9869, ISSN: 0027-8424
- HYUN INSOO ET AL: "New advances in PS cell research do not obviate the need for human embryonicstem cells", CELL STEM CELL, CELL PRESS, US, vol. 1, no. 4, 1 October 2007 (2007-10-01) , pages 367-368, XP009098391, ISSN: 1934-5909
- RICHARDS M ET AL: "Human feeders support prolonged undifferentiated growth of human inner cell masses and embryonic stem cells", NATURE BIOTECHNOLOGY, NATURE PUBLISHING GROUP, NEW YORK, NY, US LNKD- DOI:10.1038/NBT726, vol. 20, no. 9, 5 August 2002 (2002-08-05) , pages 933-936, XP002979805, ISSN: 1087-0156
- THOMSON J A ET AL: "EMBRYONIC STEM CELL LINES DERIVED FROM HUMAN BLASTOCYSTS", SCIENCE, AMERICAN ASSOCIATION FOR THE ADVANCEMENT OF SCIENCE, WASHINGTON, DC; US LNKD- DOI:10.1126/SCIENCE. 282.5391.1145, vol. 282, 6 November 1998 (1998-11-06), pages 1145-1147, XP002933311, ISSN: 0036-8075
- REUBINOFF BENJAMIN E ET AL: "Embryonic stem cell lines from human blastocysts: Somatic differentiation in vitro", NATURE BIOTECHNOLOGY, NATURE PUBLISHING GROUP, NEW YORK, NY, US LNKD- DOI: 10.1038/74447, vol. 18, no. 4, 1 April 2000 (2000-04-01), pages 399-404, XP002195338, ISSN: 1087-0156
- AMIT M ET AL: "DERIVATION AND SPONTANEOUS DIFFERENTIATION OF HUMAN EMBRYONIC STEM CELLS", JOURNAL OF ANATOMY, CAMBRIDGE UNIVERSITY PRESS, CAMBRIDGE, GB LNKD- DOI:10.1046/J. 1469-7580.2002.00032.X, vol. 200, no. PART 03, 1 March 2002 (2002-03-01), pages 225-232, XP001199527, ISSN: 0021-8782
- "people: James Thomson - A transplant medicine revolutionary", THE BUSINESS JOURNAL, vol. 17, no. 31, 28 April 2000 (2000-04-28), page 43,




(Cont. next page)

- **SRIVASTAVA MADHULIKA ET AL: "H19 and Igf2 monoallelic expression is regulated in two distinct ways by a shared cis acting regulatory region upstream of H19", GENES AND DEVELOPMENT, vol. 14, no. 10, 15 May 2000 (2000-05-15), pages 1186-1195, ISSN: 0890-9369**
- **TAKESHI HARAGUCHI ET AL: "Vectors expressing efficient RNA decoys achieve the long-term suppression of specific microRNA activity in mammalian cells", NUCLEIC ACIDS RESEARCH, OXFORD UNIVERSITY PRESS, GB, vol. 37, no. 6, 1 April 2009 (2009-04-01), pages E43 (1)-E43(13), XP002656483, ISSN: 0305-1048**
- **LIPPS H J ET AL: "Chromosome-based vectors for gene therapy", GENE, ELSEVIER, AMSTERDAM, NL, vol. 304, 30 January 2003 (2003-01-30), pages 23-33, XP004406349, ISSN: 0378-1119, DOI: 10.1016/S0378-1119(02)01215-5**
- **HYUN ET AL.: "New advances in iPS Cell research do not obviate the need for human embryonic stem cells", CELL STEM CELL, vol. 1, 2007, pages 367-368,**


Remarks:

The file contains technical information submitted after the application was filed and not included in this specification

SEQUENCE LISTING

<110> JAPAN SCIENCE AND TECHNOLOGY CORPORATION OKAZAKI, Tsuneko IKENO, Masashi ITOU, Toshihide SUZUKI, Nobukata

<120> Mammalian artificial chromosome

<130> P0203102

<150> JP P2002-258114
<151> 2002-09-03

<150> JP P2002-338865
<151> 2002-11-22

<160> 23

<170> PatentIn version 3.1

<210> 1
<211> 17
<212> DNA
<213> Homo sapiens

<220>
<221> source
<222> (1)..(17)
<223> Human chromosome centromere region

<220>
<221> misc_feature
<222> (1)..(1)
<223> n stands for any base

<220>
<221> misc_feature
<222> (6)..(9)
<223> n stands for any base

<220>
<221> misc_feature
<222> (11)..(12)
<223> n stands for any base

<220>
<221> misc_feature
<222> (17)..(17)
<223> n stands for any base

<400> 1
nttcgnnnna nncgggn 17

<210> 2
<211> 17
<212> DNA
<213> Homo sapiens

<220>
<221> source
<222> (1)..(17)
<223> Human chromosome 21 centromere region

<220>
<221> misc_feature
<222> (1)..(1)
<223> n stands for any base

<400> 2
nttcgttgga aacggga 17

<210> 3
<211> 1868
<212> DNA
<213> Homo sapiens

<220>
<221> source
<222> (1)..(1868)
<223> Human chromosome 21 centromere region

<400> 3

aattcaaata aaaggtagac agcagcattc tcagaaattt ctttctgatg tctgcattca 60

actcatagag ttgaagattg cctttcatag agcaggtttg aaacactctt tctggagtat 120

ctggatgtgg acatttggag cgctttgatg cctacggtgg aaaagtaaat atcttccata 180

aaaacgagac agaaggattc tcagaaacaa gtttgtgatg tgtgtactca gctaacagag 240

tggaaccttt ctttttacag agcagctttg aaactctatt tttgtggatt ctgcaaattg 300

atatttagat tgctttaacg atatcgttgg aaaagggaat atcgtcatac aaaatctaga 360

cagaagcatt ctcacaaact tctttgtgat gtgtgtcctc aactaacaga gttgaacctt 420

tcttttgatg cagcagtttg gaaacactct ttttgtagaa actgtaagtg gatatttgga 480

tagctctaac gatttcgttg gaaacgggaa tatcatcatc taaaatctag acagaagcac 540

tattagaaac tacttggtga tatctgcatt caagtcacag agttgaacat tcccttactt 600

tgagcacgtt tgaaacactc ttttggaaga atctggaagt ggacatttgg agcgctttga 660

ctgcctttgt tgaaaaggaa acgtcttcca ataaaagcca gacagaagca ttctcagaaa 720

cttgttcgtg atgtgtgtac tcaactaaaa gagttgaacc tttctattga tagagcagtt 780

ttgaaacact ctttttgtgg attctgcaag tggatatttg gattgctttg aggatttcgt 840

tggaagcggg aattcgtata aaaactagac agcagcattc ccagaaattt ctttcggata 900

tttccattca actcatagag atgaacatgg cctttcatag agcaggtttg aaacactctt 960

tttgtagttt gtggaagtgg acatttcgat cgccttgacg cctacggtga aaaaggaaat 1020

atcttcccat aaaaatagac agaagcattc tcagaaactt gttggtgata tgtgtctcaa 1080

ctaacagagt tgaactttgc cattgataga gagcagtttt gaaacactct ttttgtggaa 1140

tctgcaagtg gatatttgga tagcttggag gatttcgttg gaagcgggaa ttcaaataaa 1200

aggtagacag cagcattctc agaaatttct ttctgatgac tgcattcaac tcatagagtt 1260

gaacattccc tttcatagag caggtttgaa acactctttc tggagtatct ggatgtggac 1320

atttggagcg ctttgatgcc tatggtgaaa aagtaaatat cttcccataa aaacgagaca 1380

gaaggattct gagaaacaag tttgtgatgt gtgtactcag ctaacagagt ggaacctctc 1440

ttttgatgca gcagtttgga aacactcttt ttgtagaaac tgtaagtgga tatttggata 1500

gctctaatga tttcgttgga aacgggaata tcatcatcta aaatctagac agaagccctc 1560

tcagaaacta ctttgtgata tctgcattca agtcacagag ttgaacattc gctttcttag 1620

agcacgttgg aaacactctt tttgtagtgt ctggaagtgg acatttggag cgctttgatg 1680

cctttggtga aaaagggaat gtcttcccat aaaaactaga cagaagcatt ctcagaaact 1740

tgtttttgat gtgtgtaccc agccaaagga gttgaacatt tctattgata gagcagtttt 1800

gaaacactct ttttgtggaa aatgcaggtg gatatttgga tagcttggag gatttcgttg 1860

gaagcggg 1868

<210> 4
<211> 8286
<212> DNA
<213> Artificial Sequence

<220>
<223> Description of Artificial Sequence:Probe for an arm region of YAC

<400> 4

ttctcatgtt tgacagctta tcatcgataa gctttaatgc ggtagtttat cacagttaaa 60

ttgctaacgc agtcaggcac cgtgtatgaa atctaacaat gcgctcatcg tcatcctcgg 120

caccgtcacc ctggatgctg taggcatagg cttggttatg ccggtactgc cgggcctctt 180

gcgggatatc gtccattccg acagcatcgc cagtcactat ggcgtgctgc tagcgctata 240

tgcgttgatg caatttctat gcgcacccgt tctcggagca ctgtccgacc gctttggccg 300

ccgcccagtc ctgctcgctt cgctacttgg agccactatc gactacgcga tcatggcgac 360

cacacccgtc ctgtggatca attcccttta gtataaattt cactctgaac catcttggaa 420

ggaccggtaa ttatttcaaa tctctttttc aattgtatat gtgttatgtt atgtagtata 480

ctctttcttc aacaattaaa tactctcggt agccaagttg gtttaaggcg caagacttta 540

atttatcact acggaattcc gtaatcttga gatcgggcgt tcgatcgccc cgggagattt 600

ttttgttttt tatgtcttcc attcacttcc cagacttgca agttgaaata tttctttcaa 660

gggaattgat cctctacgcc ggacgcatcg tggccggcat caccggcgcc acaggtgcgg 720

ttgctggcgc ctatatcgcc gacatcaccg atggggaaga tcgggctcgc cacttcgggc 780

tcatgagcgc ttgtttcggc gtgggtatgg tggcaggccc cgtggccggg ggactgttgg 840

gcgccatctc cttgcatgca ccattccttg cggcggcggt gctcaacggc ctcaacctac 900

tactgggctg cttcctaatg caggagtcgc ataagggaga gcgtcgaccg atgcccttga 960

gagccttcaa cccagtcagc tccttccggt gggcgcgggg catgactatc gtcgccgcac 1020

ttatgactgt cttctttatc atgcaactcg taggacaggt gccggcagcg ctctgggtca 1080

ttttcggcga ggaccgcttt cgctggagcg cgacgatgat cggcctgtcg cttgcggtat 1140

tcggaatctt gcacgccctc gctcaagcct tcgtcactgg tcccgccacc aaacgtttcg 1200

gcgagaagca ggccattatc gccggcatgg cggccgacgc gctgggctac gtcttgctgg 1260

cgttcgcgac gcgaggctgg atggccttcc ccattatgat tcttctcgct tccggcggca 1320

tcgggatgcc cgcgttgcag gccatgctgt ccaggcaggt agatgacgac catcagggac 1380

agcttcaagg atcgctcgcg gctcttacca gcctaacttc gatcactgga ccgctgatcg 1440

tcacggcgat ttatgccgcc tcggcgagca catggaacgg gttggcatgg attgtaggcg 1500

ccgccctata ccttgtctgc ctccccgcgt tgcgtcgcgg tgcatggagc cgggccacct 1560

cgacctgaat ggaagccggc ggcacctcgc taacggattc accactccaa gaattggagc 1620

caatcaattc ttgcggagaa ctgtgaatgc gcaaaccaac ccttggcaga acatatccat 1680

cgcgtccgcc atctccagca gccgcacgcg gcgcatcccc cccccccttt caattcaatt 1740

catcattttt tttttattct ttttttgat ttcggtttct ttgaaatttt tttgattcgg 1800

taatctccga acagaaggaa gaacgaagga aggagcacag acttagattg gtatatatac 1860

gcatatgtag tgttgaagaa acatgaaatt gcccagtatt cttaacccaa ctgcacagaa 1920

caaaaacctg caggaaacga agataaatca tgtcgaaagc tacatataag gaacgtgctg 1980

ctactcatcc tagtcctgtt gctgccaagc tatttaatat catgcacgaa aagcaaacaa 2040

acttgtgtgc ttcattggat gttcgtacca ccaaggaatt actggagtta gttgaagcat 2100

taggtcccaa aatttgttta ctaaaaacac atgtggatat cttgactgat ttttccatgg 2160

agggcacagt taagccgcta aaggcattat ccgccaagta caatttttta ctcttcgaag 2220

acagaaaatt tgctgacatt ggtaatacag tcaaattgca gtactctgcg ggtgtataca 2280

gaatagcaga atgggcagac attacgaatg cacacggtgt ggtgggccca ggtattgtta 2340

gcggtttgaa gcaggcggca gaagaagtaa caaaggaacc tagaggcctt ttgatgttag 2400

cagaattgtc atgcaagggc tccctatcta ctggagaata tactaagggt actgttgaca 2460

ttgcgaagag cgacaaagat tttgttatcg gctttattgc tcaaagagac atgggtggaa 2520

gagatgaagg ttacgattgg ttgattatga cacccggtgt gggtttagat gacaagggag 2580

acgcattggg tcaacagtat agaaccgtgg atgatgtggt ctctacagga tctgacatta 2640

ttattgttgg aagaggacta tttgcaaagg gaagggatgc taaggtagag ggtgaacgtt 2700

acagaaaagc aggctgggaa gcatatttga gaagatgcgg ccagcaaaac taaaaaactg 2760

tattataagt aaatgcatgt atactaaact cacaaattag agcttcaatt taattatatc 2820

agttattact cgggcgtaat gatttttata atgacgaaaa aaaaaaaatt ggaaagaaaa 2880

gggggggggg gcagcgttgg gtcctggcca cgggtgcgca tgatcgtgct cctgtcgttg 2940

aggacccggc taggctggcg gggttgcctt actggttagc agaatgaatc accgatacgc 3000

gagcgaacgt gaagcgactg ctgctgcaaa acgtctgcga cctgagcaac aacatgaatg 3060

gtcttcggtt tccgtgtttc gtaaagtctg gaaacgcgga agtcagcgcc ctgcaccatt 3120

atgttccgga tctgcatcgc aggatgctgc tggctaccct gtggaacacc tacatctgta 3180

ttaacgaagc gctggcattg accctgagtg atttttctct ggtcccgccg catccatacc 3240

gccagttgtt taccctcaca acgttccagt aaccgggcat gttcatcatc agtaacccgt 3300

atcgtgagca tcctctctcg tttcatcggt atcattaccc ccatgaacag aaattccccc 3360

ttacacggag gcatcaagtg accaaacagg aaaaaaccgc ccttaacatg gcccgcttta 3420

tcagaagcca gacattaacg cttctggaga aactcaacga gctggacgcg gatgaacagg 3480

cagacatctg tgaatcgctt cacgaccacg ctgatgagct ttaccgcagc caagcttatc 3540

cctcgagggc tgcctcgcgc gtttcggtga tgacggtgaa aacctctgac acatgcagct 3600

cccggagacg gtcacagctt gtctgtaagc ggatgccggg agcagacaag cccgtcaggg 3660

cgcgtcagcg ggtgttggcg ggtgtcgggg cgcagccatg acccagtcac gtagcgatag 3720

cggagtgtat actggcttaa ctatgcggca tcagagcaga ttgtactgag agtgcaccat 3780

atgcggtgtg aaataccgca cagatgcgta aggagaaaat accgcatcag gcgctcttcc 3840

gcttcctcgc tcactgactc gctgcgctcg gtcgttcggc tgcggcgagc ggtatcagct 3900

cactcaaagg cggtaatacg gttatccaca gaatcagggg ataacgcagg aaagaacatg 3960

tgagcaaaag gccagcaaaa ggccaggaac cgtaaaaagg ccgcgttgct ggcgtttttc 4020

cataggctcc gcccccctga cgagcatcac aaaaatcgac gctcaagtca gaggtggcga 4080

aacccgacag gactataaag ataccaggcg tttccccctg gaagctccct cgtgcgctct 4140

cctgttccga ccctgccgct taccggatac ctgtccgcct ttctcccttc gggaagcgtg 4200

gcgctttctc atagctcacg ctgtaggtat ctcagttcgg tgtaggtcgt tcgctccaag 4260

ctgggctgtg tgcacgaacc ccccgttcag cccgaccgct gcgccttatc cggtaactat 4320

cgtcttgagt ccaacccggt aagacacgac ttatcgccac tggcagcagc cactggtaac 4380

aggattagca gagcgaggta tgtaggcggt gctacagagt tcttgaagtg gtggcctaac 4440

tacggctaca ctagaaggac agtatttggt atctgcgctc tgctgaagcc agttaccttc 4500

ggaaaaagag ttggtagctc ttgatccggc aaacaaacca ccgctggtag cggtggtttt 4560

tttgtttgca agcagcagat tacgcgcaga aaaaaaggat ctcaagaaga tcctttgatc 4620

ttttctacgg ggtctgacgc tcagtggaac gaaaactcac gttaagggat tttggtcatg 4680

agattatcaa aaaggatctt cacctagatc cttttaaatt aaaaatgaag ttttaaatca 4740

atctaaagta tatatgagta aacttggtct gacagttacc aatgcttaat cagtgaggca 4800

cctatctcag cgatctgtct atttcgttca tccatagttg cctgactccc cgtcgtgtag 4860

ataactacga tacgggaggg cttaccatct ggccccagtg ctgcaatgat accgcgagac 4920

ccacgctcac cggctccaga tttatcagca ataaaccagc cagccggaag ggccgagcgc 4980

agaagtggtc ctgcaacttt atccgcctcc atccagtcta ttaattgttg ccgggaagct 5040

agagtaagta gttcgccagt taatagtttg cgcaacgttg ttgccattgc tgcaggcatc 5100

gtggtgtcac gctcgtcgtt tggtatggct tcattcagct ccggttccca acgatcaagg 5160

cgagttacat gatcccccat gttgtgcaaa aaagcggtta gctccttcgg tcctccgatc 5220

gttgtcagaa gtaagttggc cgcagtgtta tcactcatgg ttatggcagc actgcataat 5280

tctcttactg tcatgccatc cgtaagatgc ttttctgtga ctggtgagta ctcaaccaag 5340

tcattctgag aatagtgtat gcggcgaccg agttgctctt gcccggcgtc aacacgggat 5400

aataccgcgc cacatagcag aactttaaaa gtgctcatca ttggaaaacg ttcttcgggg 5460

cgaaaactct caaggatctt accgctgttg agatccagtt cgatgtaacc cactcgtgca 5520

cccaactgat cttcagcatc ttttactttc accagcgttt ctgggtgagc aaaaacagga 5580

aggcaaaatg ccgcaaaaaa gggaataagg gcgacacgga aatgttgaat actcatactc 5640

ttcctttttc aatattattg aagcatttat cagggttatt gtctcatgag cggatacata 5700

tttgaatgta tttagaaaaa taaacaaata ggggttccgc gcacatttcc ccgaaaagtg 5760

ccacctgacg tctaagaaac cattattatc atgacattaa cctataaaaa taggcgtatc 5820

acgaggccct ttcgtcttca agaattaatt cggtcgaaaa aagaaaagga gagggccaag 5880

agggagggca ttggtgacta ttgagcacgt gagtatacgt gattaagcac acaaaggcag 5940

cttggagtat gtctgttatt aatttcacag gtagttctgg tccattggtg aaagtttgcg 6000

gcttgcagag cacagaggcc gcagaatgtg ctctagattc cgatgctgac ttgctgggta 6060

ttatatgtgt gcccaataga aagagaacaa ttgacccggt tattgcaagg aaaatttcaa 6120

gtcttgtaaa agcatataaa aatagttcag gcactccgaa atacttggtt ggcgtgtttc 6180

gtaatcaacc taaggaggat gttttggctc tggtcaatga ttacggcatt gatatcgtcc 6240

aactgcatgg agatgagtcg tggcaagaat accaagagtt cctcggtttg ccagttatta 6300

aaagactcgt atttccaaaa gactgcaaca tactactcag tgcagcttca cagaaacctc 6360

attcgtttat tcccttgttt gattcagaag caggtgggac aggtgaactt ttggattgga 6420

actcgatttc tgactgggtt ggaaggcaag agagccccga aagcttacat tttatgttag 6480

ctggtggact gacgccagaa aatgttggtg atgcgcttag attaaatggc gttattggtg 6540

ttgatgtaag cggaggtgtg gagacaaatg gtgtaaaaga ctctaacaaa atagcaaatt 6600

tcgtcaaaaa tgctaagaaa taggttatta ctgagtagta tttatttaag tattgtttgt 6660

gcacttgcct gcaggccttt tgaaaagcaa gcataaaaga tctaaacata aaatctgtaa 6720

aataacaaga tgtaaagata atgctaaatc atttggcttt ttgattgatt gtacaggaaa 6780

atatacatcg caggggggttg acttttacca tttcaccgca atggaatcaa acttgttgaa 6840

gagaatgttc acaggcgcat acgctacaat gacccgattc ttgctagcct tttctcggtc 6900

ttgcaaacaa ccgccggcag cttagtatat aaatacacat gtacatacct ctctccgtat 6960

cctcgtaatc attttcttgt atttatcgtc ttttcgctgt aaaaacttta tcacacttat 7020

ctcaaataca cttattaacc gcttttacta ttatcttcta cgctgacagt aatatcaaac 7080

agtgacacat attaaacaca gtggtttctt tgcataaaca ccatcagcct caagtcgtca 7140

agtaaagatt tcgtgttcat gcagatagat aacaatctat atgttgataa ttagcgttgc 7200

ctcatcaatg cgagatccgt ttaaccggac cctagtgcac ttaccccacg ttcggtccac 7260

tgtgtgccga acatgctcct tcactatttt aacatgtgga attaattcta aatcctcttt 7320

atatgatctg ccgatagata gttctaagtc attgaggttc atcaacaatt ggattttctg 7380

tttactcgac ttcaggtaaa tgaaatgaga tgatacttgc ttatctcata gttaactcta 7440

agaggtgata cttatttact gtaaaactgt gacgataaaa ccggaaggaa gaataagaaa 7500

actcgaactg atctataatg cctattttct gtaaagagtt taagctatga aagcctcggc 7560

attttggccg ctcctaggta gtgctttttt tccaaggaca aaacagtttc tttttcttga 7620

gcaggtttta tgtttcggta atcataaaca ataaataaat tatttcattt atgtttaaaa 7680

ataaaaaata aaaaagtatt ttaaattttt aaaaaagttg attataagca tgtgaccttt 7740

tgcaagcaat taaattttgc aatttgtgat tttaggcaaa agttacaatt tctggctcgt 7800

gtaatatatg tatgctaaag tgaactttta caaagtcgat atggacttag tcaaaagaaa 7860

ttttcttaaa aatatatagc actagccaat ttagcacttc tttatgagat atattataga 7920

ctttattaag ccagatttgt gtattatatg tatttacccg gcgaatcatg gacatacatt 7980

ctgaaatagg taatattctc tatggtgaga cagcatagat aacctaggat acaagttaaa 8040

agctagtact gttttgcagt aattttttc tttttataa gaatgttacc acctaaataa 8100

gttataaagt caatagttaa gtttgatatt tgattgtaaa ataccgtaat atatttgcat 8160

gatcaaaagg ctcaatgttg actagccagc atgtcaacca ctatattgat caccgatata 8220

tggacttcca caccaactag taatatgaca ataaattcaa gatattcttc atgagaatgg 8280

cccaga 8286

<210> 5
<211> 3631
<212> DNA
<213> Homo sapiens

<400> 5

aagaccagat agtacagggc ctggctacaa aaatacaagc ttttactatg ctattgcaat 60

actaaacgat aagcattagg atgttaagtg actcaggaaa taagattttg ggaaaaagta 120

atctgcttat gtgcacaaaa tggattcaag tttgcagata aaataaaata tggatgatga 180

ttcaagggga cagatacaat ggttcaaacc caagaggagc agtgagtctg tggaattttg 240

aaggatggac aaaggtgggg tgagaaagac atagtattcg acctgactgt gggagatgag 300

aaggaagaag gaggtgataa atgactgaaa gctcccagac tggtgaagat aacaggagga 360

aaccatgcac ttgaccctgg tgactctcat gtgtgaaggg tagagggata ttaacagatt 420

tactttttag gaagtgctag attggtcagg gagttttgac cttcaggtct tgtgtctttc 480

atatcaagga acctttgcat tttccaagtt agagtgccat attttggcaa atataacttt 540

attagtaatt ttatagtgct ctcacattga tcagactttt tcctgtgaat tacttttgaa 600

tttggctgta tatatccaga atatgggaga gagacaaata attattgtag ttgcaggcta 660

tcaacaatac tggtctctct gagccttata acctttcaat atgccccata aacagagtaa 720

acaggatta ttcatggcac taaatatttt cacctaggtc agtcaacaaa tggaggcaat 780

gtgcattttt tgatacatat ttttatatat ttatggggca tgtgatactt acatgcctag 840

aacatgtgac tgattaagtc tagatattta ggatatccat tactttgagc atttatcatt 900

tctatgtatt gagaaaattt caaatcctca tttctgacca ttttgaaata tataataaat 960

agtaattaac tatagtcacc ctactcaaat atcaacatta taaactaact aatccttctt 1020

tccacttttt taccaaccaa catctcttaa atcccctgcc atacacatca cacatttttc 1080

agctctgata actatcattc tactctcata ccaccatgag accacttttt tagctccaca 1140

gatgaataaa aacatgtgat atttgacttt ctgtatctgg cttattttat tatctatctc 1200

tttggcatac caagagtttg tttttgttct gcttcagggc tttcaattaa cataatgacc 1260

tctggttcca tccatgttgc tacaaatgac aagatttcat tctttttcat ggcaaaatag 1320

tactgtgcaa aaaatacaat tttttaatcc gttcatctgt tgatagacac ttaggttgat 1380

cccaaacctt aactattgtg aataggtgct tcaataaaca tgagtgtaat gtgtccattg 1440

gatatactga tttcctttct tttggataaa taaccactag tgagattgct ggattgtatg 1500

atagttctgt ttttagttta ttgagaaatc ttcatactgt tttccataat ggttgtacta 1560

ttttacattc ccaccaacag tgtgtaagaa agagttccct tttctccata tcctcacaag 1620

gatctgttat tttttgtctt ttttgttaat agcattttaa ctagagtaag tagatatctc 1680

attgtagttt tgatttgcat ttccctgatc attagtgatg ttgagatttt ttcatatgtt 1740

tgttggtcat ttgtatatct ttttctgaga ttgtctgttc atgtccttat cctacttttta 1800

ttgggattgt tgttattttc ttgataatca ttgtgtcatt ttagagcctg gatattattc 1860

ttttgtcaga tgtatagatt gtgaagattt tctcctctgt gggttgtctg tttattctgc 1920

agactcttcc ttttgccatg caaaagctct ttagtttaat ttagtcccag atattttctt 1980

tgtttttatg tgtttgcatt tgtgttcttg tcatgaaatc ctttcctaag ccaatgtgta 2040

gaagggtttt tccgatgtta ttttctagaa ttgttacagt ttcaggctta gatttaagtc 2100

cttgatccat cttaagttga tttttgtata aggtgagaga tgaagatcca gtttcattct 2160

cctacatgta gcttgccagc tatcccgact catttgttga atagggtgcc ctttcccatt 2220

tatgtttttg tttgctttgt caaagatcag ttcggatgta agtatttgag tttatttctg 2280

ggttctctat tctgttccat tggtccgatg tgcctatttg tacaccagca tcatgctgtg 2340

tttttggtga ctatggcctt attgtatagt ttgaaatgag gtaatgtaat gccattcaga 2400

tttgttcttt tttttagact tgcttgttta ttgggctctt ttttggttcc ataagaattt 2460

taggattgtt ttttctagtt ctgtgaaggc taatggtggt atttatggga attgcaatgc 2520

aatttgtagg ttgcttctgg cattatggcc attttcacaa tattgattct acccatctat 2580

gagaatggca tgtgtttcca tttgtttgtg tcttatatga ttactatcag ccgtgttttg 2640

tagttttcct tgtagatgtc tttcacctcc ttggttaggt atatattcct aagtttttgt 2700

tttgttttgt tttgtttttt gcagctattg taaaaggggt tgagttattg attttattct 2760

catcttggtc attgctggta tgtaagaaag caactcattg gtgtacgtta attttgtatc 2820

cagaaacttt gctgaattat tttatcagtt ctaggggggtt ttggaggagt ctttagagtt 2880

ttctacatac acaatcatat catcagcaaa cagtgacagt ttgacttttct ctttaacaat 2940

ttggatgtgc tttacttgtt tctcttgtct gattgctctt gctaggactt ccagtaatat 3000

gttaaagaga agtggtgaga gtgggtatcc ttgtctcatt ccagttttca gacagaatgc 3060

ttttaacttt ttcccattca atataatgtt ggctgtgtgt ttaccatagc tggcttttat 3120

tacattgagg tatgtccttt gtaaaccgat tttgctgagt tttagtcata aagtgatgtt 3180

gaattttgtt gaatgcagtt tctgtggcta ttgagataat cacatgattt ttgtttccaa 3240

ttctctttat gttgtgtatc acacttattg acttgcgtat gttaaaccat ccgtgcatcc 3300

ctcgcatgaa accacttgat catgggtttt gatatgccgt gtgggatgct attagctata 3360

ttttgtcaag gatgttggca tctatgttca tcagggatat tgatctgtag tgtttttttt 3420

ttttggttat gttctttccc agttttggta ttaaggtgat actggcttca tagaatgatt 3480

tagggaggat tctctctttc tctatcttgt agaatactgt caataggatt ggtatcaatt 3540

cttctttgaa tgtctggtag aattcgaacg tctcctttag gttttctagt ttattcatgt 3600

aaaggtgttc atagtaacct tgaataatct t 3631

<210> 6
<211> 3386
<212> DNA
<213> Homo sapiens

<400> 6

tgctaatgct tcattacaaa cttatatcct ttaattccag atgggggcaa agtatgtcca 60
ggggtgagga acaattgaaa catttgggct ggagtagatt ttgaaagtca gctctgtgtg 120
tgtgtgtgtg tgtgtgtgtg tcagcgtgtg tttcttttaa cgtcttcagc ctacaacata 180
cagggttcat ggtgggaaga agatagcaag atttaaatta tggccagtga ctagtgcttg 240
aaggggaaca actacctgca tttaatggga aggcaaaatc tcaggctttg agggaagtta 300
acataggctt gattctgggt ggaagctggg tgtgtagtta tctggaggcc aggctggagc 360
tctcagctca ctatgggttc atctttattg tctcctttca tctcaacagc tcctgggaaa 420
tgtgctggtg accgttttgg caatccattt cggcaaagaa ttcacccctg aggtgcaggc 480
ttcctggcag aagatggtga ctgcagtggc cagtgccctg tcctccagat accactgagc 540
ctcttgccca tgattcagag ctttcaagga taggctttat tctgcaagca atacaaataa 600
taaatctatt ctgctgagag atcacacatg attttcttca gctctttttt ttacatcttt 660
ttaaatatat gagccacaaa gggtttatat tgagggaagt gtgtatgtgt atttctgcat 720
gcctgtttgt gtttgtggtg tgtgcatgct cctcatttat ttttatatga gatgtgcatt 780
ttgatgagca aataaaagca gtaaagacac ttgtacacgg gagttctgca agtgggagta 840
aatggtgttg gagaaatccg gtgggaagaa agacctctat aggacaggac ttctcagaaa 900
cagatgtttt ggaagagatg ggaaaaggtt cagtgaagac ctgggggctg gattgattgc 960
agctgagtag caaggatggt tcttaatgaa gggaaagtgt tccaagcttt aggaattcaa 1020
ggtttagtca ggtgtagcaa ttctatttta ttaggaggaa tactatttct aatggcactt 1080
agcttttcac agcccttgtg gatgcctaag aaagtgaaat taatcccatg ccctcaagtg 1140
tgcagattgg tcacagcatt tcaagggaga gacctcattg taagactctg ggggaggtgg 1200
ggacttaggt gtaagaaatg aatcagcaga ggctcacaag tcagcatgag catgttatgt 1260

ctgagaaaca gaccagcact gtgagatcaa aatgtagtgg gaagaatttg tacaacatta 1320

attggaaggt ttacttaatg gaatttttgt atagttggat gttagtgcat ctctataagt 1380

aagagtttaa tatgatggtg ttacggacct ggtgtttgtg tctcctcaaa attcacatgc 1440

tgaatcccca actcccaact gaccttatct gtgggggagg cttttgaaaa gtaattaggt 1500

ttagctgagc tcataagagc agatccccat cataaaatta ttttccttat cagaagcaga 1560

gagacaagcc atttctcttt cctcccggtg aggacacagt gagaagtccg ccatctgcaa 1620

tccaggaaga gaaccctgac cacgagtcag ccttcagaaa tgtgagaaaa aactctgttg 1680

ttgaagccac ccagtctttt gtattttgtt atagcacctt acactgagta aggcagatga 1740

agaaggagaa aaaaataagc ttgggttttg agtgaactac agaccatgtt atctcaggtt 1800

tgcaaagctc ccctcgtccc ctatgtttca gcataaaata cctactctac tactctcatc 1860

tataagaccc aaataataag cctgcgccct tctctctaac tttgatttct cctattttta 1920

cttcaacatg ctttactcta gccttgtaat gtctttacat acagtgaaat gtaaagttct 1980

ttattctttt tttctttctt tctttttct cctcagcctc agaatttggc acatgccctt 2040

ccttctttca ggaacttctc caacatctct gcctggctcc atcatatcat aaaggtccca 2100

cttcaaatgc agtcactacc gtttcaggat atgcactttc tttctttttt gttttttgtt 2160

tttttaagt caaagcaaat ttcttgagag agtaaagaaa taaacgaatg actactgcat 2220

aggcagagca gccccgaggg ccgctggttg ttccttttat ggttatttct tgatgatatg 2280

ttaaacaagt tttggattat ttatgccttc tctttttagg ccatataggg taactttctg 2340

acattgccat ggcatgtttc ttttaattta atttactgtt accttaaatt caggggtaca 2400

cgtacaggat atgcaggttt gttttatagg taaaagtgtg ccatggtttt aatgggtttt 2460

ttttttcttg taaagttgtt taagtttctt gtttactctg gatattggcc tttgtcagaa 2520

gaatagattg gaaaatcttt ttcccattct gtagattgtc tttcgctctg atggtagttt 2580

cttttgctga gcaggagctc tttagtttaa ttagattcca ttggtcaatt tttgcttttg 2640

ctgcaattgc ttttcacgct ttcatcatga aatctgtgcc cgtgtttata tcatgaatag 2700

tattgccttg atttttttct aggcttttta tagtttgggg tttttcattt aagtctctaa 2760

tccatccgga gttaattttg gataaggtat aaggaaggag tccagtttca tttttcagca 2820

tatggctagc cagttctccc ccatcattta ttaaattgaa aatcctttcc ccattgcttg 2880

cttttgtcag gtttctaaaa gacagatggt tgtaggtaca atatgcagtt tcttcaagtc 2940

atataatacc atctgaaatc tcttattaat tcatttcttt tagtatgtat gctggtctcc 3000

tctgctcact atagtgaggg caccattagc cagagaatct gtctgtctag ttcatgtaag 3060

attctcagaa ttaagaaaaa tggatggcat atgaatgaaa cttcatggat gacatatgga 3120

atctaatgtg tatttgttga attaatgcat aagatgcaac aagggaaagg ttgacaactg 3180

cagtgataac ctggtattga tgatataaga gtctatagat cacagtagaa gcaataatca 3240

tggaaaacaa ttggaaatgg ggaacagcca caaacaagaa agaatcaata ctaccaggaa 3300

agtgactgca ggtcactttt cctggagcgg gtgagagaaa agtggaagtt gcagtaactg 3360

ccgaattcct ggttggctga tggaaa 3386

<210> 7
<211> 2838
<212> DNA
<213> Homo sapiens

<400> 7

gtgtaagaag gttcctgagg ctctacagat agggagcact tgtttatttt acaaagagta 60

catgggaaaa gagaaaagca agggaaccgt acaaggcatt aatgggtgac acttctacct 120

ccaaagagca gaaattatca agaactcttg atacaaagat aatactggca ctgcagaggt 180

tctagggaag acctcaaccc taagacatag cctcaagggt aatagctacg attaaactcc 240

aacaattact gagaaaataa tgtgctcaat taaaggcata atgattactc aagacaatgt 300

tatgttgtct ttcttcctcc ttcctttgcc tgcacattgt agcccataat actatacccc 360

atcaagtgtt cctgctccaa gaaatagctt cctcctctta cttgccccag aacatctctg 420

taaagaattt cctcttatct tcccatattt cagtcaagat tcattgctca cgtattactt 480

gtgacctctc ttgaccccag ccacaataaa cttctctata ctacccaaaa aatctttcca 540

aaccctcccc gacaccatat ttttatattt ttcttattta tttcatgcac acacacacac 600

tccgtgcttt ataagcaatt ctgcctattc tctaccttct tacaatgcct actgtgcctc 660

atattaaatt catcaatggg cagaaagaaa atatttattc aagaaaacag tgaatgaatg 720

aacgaatgag taaatgagta aatgaaggaa tgattattcc ttgctttaga acttctggaa 780

ttagaggaca atattaataa taccatcgca cagtgtttct ttgttgttaa tgctacaaca 840

tacaaagagg aagcatgcag taaacaaccg aacagttatt tcctttctga tcataggagt 900

aatatttttt tccttgagca catttttgcc ataggtaaaa ttagaaggat ttttagaact 960

ttctcagttg tatacatttt taaaaatctg tattatatgc atgttgatta attttaaact 1020

tacttgaata cctaaacaga atctgttgtt tccttgtgtt tgaaagtgct ttcacagtaa 1080

ctctgtctgt actgccagaa tatactgaca atgtgttata gttaactgtt ttgatcacaa 1140

cattttgaat tgactggcag cagaagctct ttttatatcc atgtgttttc cttaagtcat 1200

tatacatagt aggcatgaga ctctttatac tgaataagat atttaggaac cactggttta 1260

catatcagaa gcagagctac tcagggcatt ttggggaaga tcactttcac attcctgagc 1320

atagggaagt tctcataaga gtaagatatt aaaaggagat acttgtgtgg tattcgaaag 1380

acagtaagag agattgtaga ccttatgatc ttgataggga aaacaaacta cattcctttc 1440

tccaaaagtc aaaaaaaaag agcaaatata gcttactata ccttctattc ctacaccatt 1500

agaagtagtc agtgagtcta ggcaagatgt tggccctaaa aatccaaata ccagagaatt 1560

catgagaaca tcacctggat gggacatgtg ccgagcaaca caattactat atgctaggca 1620

ttgctatctt catattgaag atgaggaggt caagagatga aaaaagactt ggcaccttgt 1680

tgttatatta aaattatttg ttagagtaga gcttttgtaa gagtctagga gtgtgggagc 1740

taaatgatga tacacatgga cacaaagaat agatcaacag acacccaggc ctacttgagg 1800

gttgagggtg ggaagaggga gacgatgaaa aagaacctat tgggtattaa gttcatcact 1860

gagtgatgaa ataatctgta catcaagacc cagtgatatg caatttacct atataacttg 1920

tacatgtacc cccaaattta aaataaagtt aaaacaaagt ataggaatgg aattaattcc 1980

tcaagatttg gctttaattt tatttgataa tttatcaaat ggttgttttt cttttctcac 2040

tatggcgttg ctttataaac tatgttcagt atgtctgaat gaaagggtgt gtgtgtgtgt 2100

gaaagagagg gagagaggaa gggaagagag gacgtaataa tgtgaatttg agttcatgaa 2160

aatttttcaa taaaataatt taatgtcagg agaattaagc ctaatagtct cctaaatcat 2220

ccatctcttg agcttcagag cagtcctctg aattaatgcc tacatgtttg taaagggtgt 2280

tcagactgaa gccaagattc tacctctaaa gagatgcaat ctcaaattta tctgaagact 2340

gtacctctgc tctccataaa ttgacaccat ggcccactta atgaggttaa aaaaaagcta 2400

attctgaatg aaaatctgag cccagtggag gaaatattaa tgaacaaggt gcagactgaa 2460

atataaattt tctgtaataa ttatgcatat actttagcaa agttctgtct atgttgactt 2520

tattgctttt ggtaagaaat acaacttttt aaagtgaact aaactatcct atttccaaac 2580

tattttgtgt gtgtgcggtt tgtttctatg ggttctggtt ttcttggagc atttttattt 2640

cattttaatt aattaattct gagagctgct gagttgtgtt tactgagaga ttgtgtatct 2700

gcgagagaag tctgtagcaa gtagctagac tgtgcttgac ctaggaacat atacagtaga 2760

ttgctaaaat gtctcacttg gggaatttta gactaaacag tagagcatgt ataaaaatac 2820

tctagtcaag tgctgctt 2838

<210> 8
<211> 6
<212> DNA
<213> Homo sapiens

<400> 8
ttaggg 6

<210> 9
<211> 1884
<212> DNA
<213> Homo sapiens

<400> 9

gtatacatac atacctgaat atggaatcaa atatttttct aagatgaaac agtcatgatt 60

tatttcaaat aggtacggat aagtagatat tgaggtaagc attaggtctt atattatgta 120

acactaatct attactgcgc tgaaactgtg gtctttatga aaattgtttt cactacacta 180

ttgagaaatt aagagataat ggcaaaagtc acaaagagta tattcaaaaa gaagtatagc 240

actttttcct tagaaaccac tgctaactga aagagactaa gatttgtccc gtcaaaaatc 300

ctggacctat gcctaaaaca catttcacaa tccctgaact tttcaaaaat tggtacatgc 360

tttagcttta aactacaggc ctcactggag ctacagacaa gaaggtaaaa aacggctgac 420

aaaagaagtc ctggtatcct ctatgatggg agaaggaaac tagctaaagg gaagaataaa 480

ttagagaaaa actggaatga ctgaatcgga acaaggcaaa ggctataaaa aaaattaagc 540

agcagtatcc tcttgggggc cccttcccca cactatctca atgcaaatat ctgtctgaaa 600

cggtccctgg ctaaactcca cccatgggtt ggccagcctt gccttgacca atagccttga 660

caaggcaaac ttgaccaata gtcttagagt atccagtgag gccaggggcc ggcggctggc 720

tagggatgaa gaataaaagg aagcaccctt cagcagttcc acacactcgc ttctggaacg 780

tctgagatta tcaataagct cctagtccag acgccatggg tcatttcaca gaggaggaca 840

aggctactat cacaagcctg tggggcaagg tgaatgtgga agatgctgga ggagaaaccc 900

tgggaaggta ggctctggtg accaggacaa gggagggaag gaaggaccct gtgcctggca 960

aaagtccagg tcgcttctca ggatttgtgg caccttctga ctgtcaaact gttcttgtca 1020

atctcacagg ctcctggttg tctacccatg gacccagagg ttctttgaca gctttggcaa 1080

cctgtcctct gcctctgcca tcatgggcaa ccccaaagtc aaggcacatg gcaagaaggt 1140

gctgacttcc ttgggagatg ccataaagca cctggatgat ctcaagggca cctttgccca 1200

gctgagtgaa ctgcactgtg acaagctgca tgtggatcct gagaacttca aggtgagtcc 1260

aggagatgtt tcagcactgt tgcctttagt ctcgaggcaa cttagacaac tgagtattga 1320

tctgagcaca gcagggtgtg agctgtttga agatactggg gttgggagtg aagaaactgc 1380

agaggactaa ctgggctgag acccagtggc aatgttttag ggcctaagga gtgcctctga 1440

aaatctagat ggacaacttt gactttgaga aaagagaggt ggaaatgagg aaaatgactt 1500

ttctttatta gatttcggta gaaagaactt tcacctttcc cctatttttg ttattcgttt 1560

taaaacatct atctggaggc aggacaagta tggtcgttaa aaagatgcag gcagaaggca 1620

tatattggct cagtcaaagt ggggaacttt ggtggccaaa catacattgc taaggctatt 1680

cctatatcag ctggacacat ataaaatgct gctaatgctt cattacaaac ttatatcctt 1740

taattccaga tgggggcaaa gtatgtccag gggtgaggaa caattgaaac atttgggctg 1800

gagtagattt tgaaagtcag ctctgtgtgt gtgtgtgtgt gtgtgtgtgt cagcgtgtgt 1860

ttcttttaac gtcttcagcc taca 1884

<210> 10
<211> 28
<212> DNA
<213> Artificial Sequence

<220>
<223> Description of Artificial Sequence:PCR primer

<400> 10
aagaccagat agtacagggc ctggctac 28

<210> 11
<211> 28
<212> DNA
<213> Artificial Sequence

<220>
<223> Description of Artificial Sequence:PCR primer

<400> 11
aagattattc aaggttacta tgaacacc 28

<210> 12
<211> 38
<212> DNA
<213> Artificial Sequence

<220>
<223> Description of Artificial Sequence:PCR primer

<400> 12
tgctaatgct tcatctagaa acttatatcc tttaattc 38

<210> 13
<211> 35
<212> DNA
<213> Artificial Sequence

<220>
<223> Description of Artificial Sequence:PCR primer

<400> 13

tttccactcg agccaaccag gaattcggca gttac 35

<210> 14
<211> 38
<212> DNA
<213> Artificial Sequence

<220>
<223> Description of Artificial Sequence:PCR primer

<400> 14
gtgtaagaag gttctctaga ggctctacag atagggag 38

<210> 15
<211> 39
<212> DNA
<213> Artificial Sequence

<220>
<223> Description of Artificial Sequence:PCR primer

<400> 15
aagcagcact tgactcgagt atttttatac atgctctac 39

<210> 16
<211> 23
<212> DNA
<213> Artificial Sequence

<220>
<223> Description of Artificial Sequence:PCR primer

<400> 16
gtatacatac atacctgaat atg 23

<210> 17
<211> 28
<212> DNA
<213> Artificial Sequence

<220>
<223> Description of Artificial Sequence:PCR primer

<400> 17
tgtaggctga agacgttaaa agaaacac 28

<210> 18
<211> 22
<212> DNA
<213> Artificial Sequence

<220>
<223> Description of Artificial Sequence:Primer for RT-PCR

<400> 18
gatgccataa agcacctgga tg 22

<210> 19

<211> 24
<212> DNA
<213> Artificial Sequence

<220>
<223> Description of Artificial Sequence:Primer for RT-PCR

<400> 19
ttgcagaata aagcctatcc ttga 24

<210> 20
<211> 25
<212> DNA
<213> Artificial Sequence

<220>
<223> Description of Artificial Sequence:Primer for RT-PCR

<400> 20
tcacccacac tgtgcccatc tacga 25

<210> 21
<211> 25
<212> DNA
<213> Artificial Sequence

<220>
<223> Description of Artificial Sequence:Primer for RT-PCR

<400> 21
cagcggaacc gctcattgcc aatgg 25

<210> 22
<211> 21
<212> DNA
<213> Artificial Sequence

<220>
<223> Description of Artificial Sequence:PCR primer

<400> 22
catcgtctct ctgaaaaatc g 21

<210> 23
<211> 21
<212> DNA
<213> Artificial Sequence

<220>
<223> Description of Artificial Sequence:PCR primer

<400> 23
aggaaacagc aaaactgtga c 21

## Description

### TECHNICAL FIELD

The present invention relates to a mammalian artificial chromosome. More particularly, the present invention relates to a production method of a mammalian artificial chromosome, a mammalian artificial chromosome and a use of a mammalian artificial chromosome. The mammalian artificial chromosome provided in the present invention can be used, for example, as a vector to carry a gene of interest to mammalian cells for gene therapy, transformation of cells, tissues or individual bodies of mammalian, and the like.

### BACKGROUND ART

Mitotically stable human artificial chromosomes (HACs), several mega-base pairs in size, are frequently generated *de novo* in the human fibroblast cell line, HT1080, upon introduction of precursor DNA constructs in either linear (YAC) or circular form (BAC or PAC) containing several tens of kilo-bases of human alpha-satellite (alphoid) DNA with frequent CENP-B boxes (Ikeno et al. 1998; Henning et al. 1999; Ebersole et al. 2000). Since essential kinetochore proteins are detected on such HACs, the input alpha-satellite arrays are capable of assembling a *de novo* active centromere/kinetochore structure similar to that of authentic human chromosomes (Ikeno et al. 1994; Ikeno et al. 1998; Henning et al. 1999; Ebersole et al. 2000; Ando et al. 2002). Since HACs duplicate once every cell cycle utilizing cellular protein factors, they also contain replication origin(s) in the alphoid sequence. Linear HACs made from the alphoid-YAC with telomere sequences acquired a functional telomere structure at the ends of the HACs, but circular HACs made from BAC or PAC had no telomere structure (Ikeno et al. 1998; Ebersole et al. 2000).

Treating human diseases by gene therapy is a challenging and promising field. Although we now have at hand tens of thousands of genes by which we might be able to cure defective human genes or to characterize in detail their function and regulation, the major obstacle still lies in the development of effective gene delivery technology. Presently available vectors for mammalian cells are mainly derived from small viruses (Mineta et al. 1995; Fisher et al. 1997; Pfeiter & Verna 2001). Although they have the advantage of highly efficient transduction of the genes of interest (transgenes), their cloning capacity is limited. They are too small to include large genome segments with tissue-specific regulatory regions. Moreover, transgenes are usually maintained stably only after random integration into host-cell chromosomes, the gene expression from which is usually unpredictable (mostly suppressed) and not under the control of the authentic regulatory region of the genes. Even worse, the step might induce unfavorable mutagenesis.

In contrast, HACs have the capacity to accommodate a large transgene with a controlling region in excess of 100 kb of DNA. HACs containing transgenes are generated *de novo* from a precursor construct with both the transgene and an alphoid array (Mejia et al. 2001) or from precursor constructs containing an alphoid array and the transgene in separate entities (Grimes et al. 2001). Thus, HACs may be used not only as vectors in therapeutic applications but also as model systems useful in the analysis of tissue or organ specific regulation of gene expression that is only possible with large genome segments.

### DISCLOSURE OF THE INVENTION

The present invention <u>relates to the embodiments characterized in the claims and</u> has been made under the above-mentioned circumstances. It is an object of the present invention to provide a technology for stably expressing a targeted functional sequence of a gene, etc. in a mammalian cell. Specifically, it is an object of the present invention to provide a mammalian artificial chromosome which is stably maintained in a mammalian cell and is capable of efficiently expressing a functional sequence contained therein, a production method of the same, and a method of transforming cells etc. by using the same, and the like. <u>The claimed invention does not concern the use of human embryonic cells.</u>

The present inventors have considered the objects mentioned above and have attempted to produce a mammalian artificial chromosome containing a target gene (GCH1 gene) in a state of being capable of expressing by employing a method of taking the target gene as a functional sequence during a process in which the mammalian artificial chromosome is formed from a precursor of an artificial chromosome. That is to say, the present inventors used BAC that is a circular vector as a artificial chromosome precursor, and co-transfected BAC (GCH1-BAC) containing about 180 kb of a genome region covering an entire GCH1 gene and its upstream regulatory region and BAC (alphoid BAC) including about 50 kb or about 100 kb of an alphoid array as a human centromere sequence with HT1080 cell, which is a human fibroblast cell. As a result, we successfully obtained a human artificial chromosome (HAC) having plural copies of GCH1 genes. It was shown that the HAC obtained was able to be maintained stably in both human cells and mouse cells even if selection operation is not carried out. When a further investigation was carried out, the increase in the GCH1 activity was observed in the transformed cell lines having the HAC and the activity showed the response with respect to the induction of interferon $\gamma$ as in the case that is present on the chromosome. That is to say, the natural expression of a GCH1 gene from the constructed HAC was confirmed.

Meanwhile, the present inventors have succeeded, by using a linear vector YAC as a precursor, in constructing a human artificial chromosome containing an entire region of human β globin gene cluster by the same method as in the case of BAC.

Furthermore, the present inventors have succeeded in transferring the constructed HAC into mouse embryonic stem cells (ES cells) and creating a chimeric mouse (HAC-containing mouse) by using the obtained ES cells. This is extremely significant that it was experimentally confirmed that an artificial chromosome could be used as a tool for gene introduction at the individual body level. Furthermore, the present inventors succeeded in transferring HAC into not only XY nuclear type ES cells but also XO nuclear type ES cells and further in creating a female chimeric mouse containing HAC by the use of the same. Note here that it is thought that the use of female chimeric mice makes it easy to transmit a mammalian artificial chromosome.

Furthermore, in the production of a mammalian artificial chromosome having a gene insertion site, when a mammalian artificial chromosome was constructed by inserting an insulator sequence for the purpose of promoting the expression of gene to be introduced later, surprisingly, the efficiency of gene transfer into the mammalian artificial chromosome was enhanced. In other words, it was found that the use of the insulator sequence makes it possible to produce efficiently mammalian artificial chromosome having a target gene.

The present invention was made based on the findings in the above-mentioned investigation and the present invention provides the following configurations.

[1] A production method of a mammalian artificial chromosome, comprising:

a first step of introducing a into a mammalian host cell first vector being circular in form and comprising a mammalian centromere sequence and a second vector being circular in form and comprising an insertion sequence for specifically inserting a sequence of interest and comprising an insulator sequence;
a second step of selecting transformed cells; and
a third step of selecting a cell containing a mammalian artificial chromosome from the selected transformed cells.

[2] A production method of a mammalian artificial chromosome, comprising:

a first step of introducing a first vector consisting of a yeast artificial chromosome having a mammalian centromere sequence and a mammalian telomere sequence and a second vector consisting of a yeast artificial chromosome having a functional sequence into a mammalian host cell;
a second step of selecting transformed cells; and
a third step of selecting a cell containing a mammalian artificial chromosome from the selected transformed cells.

[3] The production method according to 1 or 2, wherein the first vector has a selection marker gene and the selection of the transformed cells in the second step is carried out by using the selection marker gene.
[4] The production method according to any of 1 to 3, wherein the mammalian centromere sequence comprises a region in which a plurality of the following sequences are arranged at regular intervals:

5'-NTTCGNNNNANNCGGGN-3': SEQ ID NO. 1, wherein N is selected from the group consisting of A, T, C and G.

[5] The production method according to any one of 1 to 4, wherein the mammalian centromere sequence comprises a sequence derived from a human chromosome alpha satellite region.
[6] The production method according to 5, wherein the mammalian centromere sequence comprises a 11mer repeat unit derived from a human chromosome 21.
[7] The production method according to any of 1 to 6, wherein the size of the mammalian centromere sequence is about 50 kb or less.
[8] The production method according to any of 1 to 7, wherein the functional sequence consists of a sequence encoding a target gene and a regulatory region thereof.
[9] The production method according to 8, wherein the target gene is a gene other than housekeeping genes.
[10] The production method according to 8, wherein the target gene is a structural gene of human guanosine triphosphate cyclohydrolase I.
[11] The production method according to 8, wherein the functional sequence is a sequence encoding an entire region of a human β globin gene cluster.
[12] The production method according to any of 1 to 7, wherein the functional sequence consists of an insertion sequence for specifically inserting a sequence of interest.
[13] The production method according to 12, wherein the insertion sequence is a loxP site, a FRT site, or a sequence obtained by partial modification of a loxP site or a FRT site and has a function for inserting the sequence of interest.
[14] The production method according to any of 1 to 13, wherein the quantity ratio of the first vector to the second vector, which are inserted in the first step, is in the range from about 10 : 1 molecular ratio to about 1 : 10 molecular ratio.
[15] The production method according to any of 1 to 14, wherein a plurality of vectors comprising different functional sequences are used as the second vector.
[16] The production method according to any of 1 to 15, wherein the second vector comprises an insulator sequence.
[17] A mammalian artificial chromosome obtainable by the production method described in any of 1 to 16,
which comprises a mammalian replication origin, a mammalian centromere sequence and an insertion sequence for specifically inserting a sequence of interest and comprising an insulator sequence; and
which is circular in form and is replicated in a mammalian cell, maintained extrachromosomally in a host cell, and transmitted to daughter cells during cell division.
[18] A mammalian artificial chromosome obtainable by the production method described in any of 1 to 16,
which comprises a mammalian replication origin, a mammalian centromere sequence, a mammalian telomere sequence, and an insertion sequence for specifically inserting a sequence of interest and comprising an insulator sequence encoding a target gene and a regulatory region thereof; and
which is linear in form and is replicated in a mammalian cell, maintained extrachromosomally in a host cell, and transmitted to daughter cells during cell division.
[19] A mammalian artificial chromosome,
which comprises a mammalian replication origin, a mammalian centromere sequence, and a functional sequence encoding a target gene (excluding a housekeeping gene) and a regulatory region thereof, and
which is circular in form and is replicated in a mammalian cell, maintained extrachromosomally in a host cell, and transmitted to daughter cells during cell division.
[20] The mammalian artificial chromosome according to 19, wherein the target gene is a structural gene of a human guanosine triphosphate cyclohydrolase I.
[21] A mammalian artificial chromosome,
which comprises a mammalian replication origin, a mammalian centromere sequence, a mammalian telomere sequence, and a functional sequence encoding a target gene (excluding a housekeeping gene) and a regulatory region thereof, and
which is linear in form and is replicated in a mammalian cell, maintained extrachromosomally in a host cell, and transmitted to daughter cells during cell division.
[22] The mammalian artificial chromosome according to 21, wherein the functional sequence consists of an entire region of a human P globin gene cluster.
[23] A mammalian artificial chromosome,
which comprises a mammalian replication origin, a mammalian centromere sequence, and an insertion sequence for specifically inserting a sequence of interest, and
which is circular in form and is replicated in a mammalian cell, maintained extrachromosomally in a host cell, and transmitted to daughter cells during cell division.
[24] A mammalian artificial chromosome,
which comprises a mammalian replication origin, a mammalian centromere sequence, a mammalian telomere sequence, and an insertion sequence for specifically inserting a sequence of interest,
which is linear in form and is replicated in a mammalian cell, maintained extrachromosomally in a host cell, and transmitted to daughter cells during cell division.
[25] The mammalian artificial chromosome according to 23 or 24, wherein the insertion sequence is a loxP site, a FRT site, or a sequence obtained by partial modification of a loxP site or a FRT site and has a function for inserting the sequence of interest.
[26] The mammalian artificial chromosome according to any of 17 to 25, wherein the mammalian centromere sequence comprises a region in which a plurality of the following sequences are arranged at regular intervals:

5'-NTTCGNNNNANNCGGGN-3': SEQ ID NO. 1, wherein N is selected from the group consisting of A, T, C and G.

[27] The mammalian artificial chromosome according to any of 17 to 25, wherein the mammalian centromere sequence comprises a sequence derived from a human chromosome alpha satellite region.
[28] The mammalian artificial chromosome according to 27, wherein the mammalian centromere sequence comprises an 11mer repeat unit derived from a human chromosome 21.
[29] The mammalian artificial chromosome according to any of 17 to 28, comprising a plurality of the functional sequences or the insertion sequences.
[30] The mammalian artificial chromosome according to any of 17 to 29, comprising an insulator sequence.

[31] A mammalian cell containing the mammalian artificial chromosome described in any of 17 to 30 outside the autonomous chromosome.
[32] A human cell containing the mammalian artificial chromosome described in any of 17 to 30 outside the autonomous chromosome.
[33] An non-human embryonic stem cell containing the mammalian artificial chromosome described in any of 17 to 30 outside the autonomous chromosome.
[34] A production method of a mammalian cell in which the functional sequence or the insertion sequence is introduced in a state in which they can be maintained stably for a long term, the method comprising:

introducing the mammalian artificial chromosome obtained by the production method described in any of 1 to 16 or the mammalian artificial chromosome described in any of 17 to 30 into mammalian cells as target cells.

[35] A production method of a mammalian cell containing a mammalian artificial chromosome, the method comprising:

a first step of introducing a first vector being circular in form and comprising a mammalian centromere sequence and a second vector being circular in form and comprising an insertion sequence for specifically inserting a sequence of interest and comprising an insulator sequence into mammalian host cells;
a second step of selecting transformed cells;
a third step of selecting a cell containing a mammalian artificial chromosome from the selected transformed cells;
a fourth step of isolating the mammalian artificial chromosome from the selected cells; and
a fifth step of introducing the isolated mammalian artificial chromosome into a mammalian cell as a target cell.

[36] A production method of a mammalian cell containing a mammalian artificial chromosome, the method comprising:

a first step of introducing a first vector consisting of a yeast artificial chromosome having a mammalian centromere sequence and a mammalian telomere sequence and a second vector consisting of a yeast artificial chromosome having an insertion sequence for specifically inserting a sequence of interest and comprising an insulator sequence into mammalian host cells;
a second step of selecting transformed cells;
a third step of selecting a cell containing a mammalian artificial chromosome from the selected transformed cells;
a fourth step of isolating the mammalian artificial chromosome from the selected cell; and
a fifth step of introducing the isolated mammalian artificial chromosome into a mammalian cell as a target cell.

[37] A production method of a micro-cell containing a mammalian artificial chromosome, the method comprising:

a first step of introducing a first vector being circular in form and comprising a mammalian centromere sequence and a second vector being circular in form and comprising a functional sequence into mammalian host cells;
a second step of selecting transformed cells;
a third step of selecting a cell containing a mammalian artificial chromosome from the selected transformed cells;
a fourth step of fusing the selected cell with a mammalian cell having an ability of forming micro-cells;
a fifth step of selecting a hybrid cell capable of forming micro-cells and containing the mammalian artificial chromosome; and
a sixth step of forming micro-cells from the selected hybrid cell.

[38] A production method of a micro-cell containing a mammalian artificial chromosome, the method comprising:

a first step of introducing a first vector consisting of a yeast artificial chromosome including a mammalian centromere sequence and a mammalian telomere sequence and a second vector consisting of a yeast artificial chromosome including a functional sequence into mammalian host cells;
a second step of selecting transformed cells;
a third step of selecting a cell containing a mammalian artificial chromosome from the selected transformed cells;
a fourth step of fusing the selected cell with a mammalian cell having an ability of forming micro-cells;
a fifth step of selecting a hybrid cell having an ability of forming micro-cells and containing a mammalian artificial chromosome; and
a sixth step of forming micro-cells from the selected hybrid cell.

[39] A production method of mammalian cells containing a mammalian artificial chromosome, comprising:

fusing the micro-cell obtainable by the production method described in 37 or 38 with a mammalian cell as a target cell.

[40] A production method of a mammalian cell containing a mammalian artificial chromosome, comprising:

isolating the mammalian artificial chromosome from the host cell containing the mammalian artificial chromosome described in any of 17 to 30; and
introducing the isolated mammalian artificial chromosome into a mammalian cell as a target cell.

[41] A production method of a micro-cell containing a mammalian artificial chromosome, the method comprising:

fusing a host cell containing the mammalian artificial chromosome described in any of 17 to 30 and a mammalian cell having an ability of forming micro-cells;
selecting a hybrid cell having an ability of forming micro-cellsi and containing the mammalian artificial chromosome; and
forming micro-cells from the selected hybrid cells.

[42] A production method of a mammalian cell containing a mammalian artificial chromosome, the method comprising:

fusing the micro-cell obtainable by the production method described in 41 with a mammalian cell as a target.

[43] The production method of a mammalian cell according to any of 34, 35, 36, 39, 40 and 42, wherein the mammalian cell as a target cell is a non-human embryonic stem cell, non-human embryonic germ cell, or tissue stem cell.
[44] The production method of a mammalian cell according to any of 34, 35, 36, 39, 40 and 42, wherein the mammalian cell as a target cell is formed by inducing an non-human embryonic stem cell, non-human embryonic germ cell, or tissue stem cell so as to be differentiated to a cell of specific tissue.
[45] The production method of a mammalian cell according to any of 34, 35, 36, 39, 40 and 42, wherein the mammalian cell as a target cell is a non-human fertilized egg.
[46] Use of vector for producing a mammalian artificial chromosome, comprising a mammalian centromere sequence having the size of about 50 kb or less and a selection marker gene.
[47] Use of the vector according to 46, wherein the mammalian centromere sequence comprises a region in which a plurality of the following sequences are arranged at regular intervals:

5'-NTTCGNNNNANNCGGGN-3': SEQ ID NO. 1, wherein N is selected from the group consisting of A, T, C and G.

[48] The vector according to 46 or 47, wherein the mammalian centromere sequence comprises a sequence derived from a human chromosome alpha satellite region.
[49] The vector according to 48, wherein the mammalian centromere sequence comprises an 11mer repeat unit derived from a human chromosome 21.
[50] Use of A vector for producing a mammalian artificial chromosome, comprising: a sequence of a loxP site or FRT site, or a sequence obtainable by partial modification of a loxP site or FRT site, the sequence having a function for inserting the sequence of interest, and an insulator sequence.
[51] A non-human transformed animal into which a mammalian artificial chromosome is introduced.
[52] The non-human transformed animal according to 51, wherein the mammalian artificial chromosome is a mammalian artificial chromosome described in any of 17 to 19.
[53] An XO type mouse embryonic stem cell into which a mammalian artificial chromosome is introduced.
[54] The XO type mouse embryonic stem cell according to 53, wherein the mammalian artificial chromosome is a mammalian artificial chromosome described in any of 17 to 19.
[55] A female chimeric mouse into which a mammalian artificial chromosome is introduced.
[56] The female chimeric mouse according to 55, wherein the mammalian artificial chromosome is a mammalian artificial chromosome described in any of 17 to 19.

## BRIEF DESCRIPTION OF THE DRAWINGS

Fig.1 is a table summarizing the fates of co-transfected BACs in the transformed cell lines. BS-resistant cell lines obtained by co-transfection of GCH1-BAC plus CMV/a100 BAC or SV/a50 BAC were analyzed by FISH. "HAC" indicates cell lines with an artificial chromosome detected both with a 21-I alphoid DNA and BAC vector probes. One copy of HAC was detected in more than 95% of the inspected metaphase spread of these cell lines. In the remaining cell lines, introduced BACs were either integrated into chromosomes of HT1080 (Chromosome) or signals were undetectable (Non) by FISH analysis. "HAC with GCH" indicates the cell lines carrying a HAC with signals for the GCH1 gene.

Fig.2 is a table summarizing GCH1 activity in HAC-containing cell lines. GCH1 activity of HT/GCH2-10, HT/GCH5-18 and HT1080 cells was measured in the presence or absence of IFN-$\gamma$ induction. Data are mean +/- SD values from three independent experiments.

Fig.3 shows constructs of alphoid-BACs and GCH1-BAC. CMV/a100 BAC contains 100 kb of the a21-I alphoid array from human chromosome 21 and a CMV-Bsd (Blasticidin S deaminase gene from Aspergillus terreus) selection marker for mammalian cells in the BAC vector. SV/a50 BAC contains 50kb of the a21-I alphoid array and an SV2-Bsr (Blasticidin S deaminase gene from Bacillus cereus) selection marker. GCH1-BAC contains a 180 kb genomic DNA fragment containing the GCH1 gene. The regions used as probes for FISH analysis, Southern analysis and exons (1 to 6) of the GCH1 gene are indicated as shadowed boxes, black boxes and open boxes, respectively. BAC vectors contain chloramphenicol-resistance gene (Cm) for selection in E. coli.

Fig.4 shows the result of FISH analysis for GCH1 signals on HAC. The cell lines HT/GCH2-10, generated by co-transfection of CMV/a100 BAC and GCH1-BAC, and the cell line HT/GCH5-18 generated by co-transfection of SV/a50 BAC and GCH1-BAC were hybridized with probes for GCH1 exon 1 (green) and BAC vector (red) (Left) or with probes for GCH1 exon 4-6 (green) and GCH1 exon 1 (red) (Right). Arrowheads indicate HACs.

Fig.5 shows the result of structural analysis of GCH1-HAC. The result of restriction analysis of GCH1 genes in HACs is idicated. Genomic DNAs from HT/GCH2-10, HT/GCH5-18 and non-transfected HT1080 were digested with BamHI (A) or StuI (B) and fractionated by conventional gel electrophoresis. The expected size of the BamHI and StuI fragments detected by the US probe (A) and the exon 6 probe (B), respectively, using the endogenous GCH1 locus and GCH1-HAC, are shown on the top.

Fig.6 is a graph used for estimation of the copy number of GCH1-BAC and alphoid BAC in the HACs by dot hybridization. Left: The intensity value obtained with the GCH1 exon 6 probe. Input DNA of GCH1-BAC (0.4, 0.2, 0.1 ng) and genomic DNA (1.0, 0.5 $\mu$g) from HT1080, HT/GCH2-10 and HT/GCH5-18 were hybridized with the GCH1 exon 6 probe. The value obtained with 0.1 ng GCH1-BAC DNA was used as a standard. Right: The intensity value obtained with BAC vector probe. GCH1-BAC (0.5, 0.1, 0.05 ng) and genomic DNA (0.5, 0.25 $\mu$g) from HT1080, HT/GCH2-10 and HT/GCH5-18 were hybridized with the BAC vector probe. The signal intensity obtained with each probe was determined using a Fuji image-analyzer BAS1000.

Fig.7 shows the result of FISH analysis of hybrid cells which have been obtained by cell fusion of HAC-containing cell line with mouse A9 cells. HT/GCH5-18 cell lines were fused with A9 cells mediated by PEG. BS- and Ouabain-resistant cell lines were analyzed by FISH. Metaphase spreads were hybridized with the BAC vector probe (red) and an Alu repeat probe (green) (A) or hybridized with the BAC vector probe (green) and a mouse minor satellite probe (red) (B). Arrows indicate HACs.

Fig. 8 shows the result of FISH analysis of ES cells in which HAC was transfered. A shows the result of detection using alphoid DNA and a BAC vector as probes; B shows the result of detection using an exon 1 region of GCH1 and a BAC vector as probes; and C shows the result of detection using mouse minor satellite DNA and a BAC vector as probes.

Fig. 9 is a graph showing the result of an analysis of the stability of HAC in ES cells. Black box shows the rate of HAC-containing cells in the case where culturing is carried out in the presence of blasticidin S (bs+); and void box shows the rate of HAC-containing cells in the case where culturing is carried out in the absence of blasticidin S (bs-).

Fig. 10A shows the results of PFGE analysis of A201F4.3 (lane 1 and lane 2) and 7c5hTEL (lane 3 and lane 4). In addition to a chromosome of the host cell, the presence of globin or alphoid YAC is observed at 150kb or 100kb (lane 1 and lane 3). Purified and condensed YACs (lane 2 and lane 4) and mixed YAC (5 lane) were introduced into HT1080 cells. M in the view indicates a molecular weight marker. Fig. 10B shows the results of FISH analysis of transformed cells obtained by the introduction of YAC. An arrow shows a mini chromosome observed in the transformed cell (upper view). Furthermore, signals of arm portions of YAC (green: arrow heads) and alphoid (red: arrow) are shown (lower view). Staining was carried out by using DAPI (blue).

Fig. 11 shows the results of FISH analysis of transformed cells containing mini chromosomes. The result in the case of using arm portions of YAC (green: arrow heads) and alphoid (red: arrow) as probes (left upper view), the result in the case of using A (green, arrow head) of $\beta$ globin shown in the lower part of Fig. 11 and alphoid (red, arrow) (upper right view), the result in the case of using B (green, arrow head) of $\beta$ globin and alphoid (red, arrow) (lower left view), and the result in the case of using C (green, arrow head) and alphoid (red, arrow) (lower right view) are shown, respectively.

Fig. 12 shows the results of FISH analysis of two clones (C11 and C29) that are transformed cells containing a mini chromosome by using a human $\beta$ globin (SEQ ID NO. 5, SEQ ID NO. 6, and SEQ ID NO. 9) or telomere repeat sequence (about 500 bp of sequence consisting of sequences of SEQ ID NO. 8) as probes. Blue, green and red indicate signals of DAPI, human β globin and telomere, respectively.

Fig. 13 shows the results of FISH analysis of transformed cells obtained by fusing A9 cells and cells containing a mini chromosome. The upper left view shows the result of staining with DAPI (blue), the upper right view shows the result of detection of signal (green) by β globin probe (SEQ ID NO. 5, SEQ ID NO. 6 and SEQ ID NO. 9), the lower left view shows the result of detection of signal (red) by an alphoid probe (SEQ ID NO. 3) and the lower right view was obtained by superposing the above-mentioned views. An alphoid signal can be observed only in the mini chromosome.

Fig. 14 shows the results of fiber FISH analysis of a mini chromosome. The upper view shows the result when a β globin probe (SEQ ID NO. 5, SEQ ID NO. 6, and SEQ ID NO. 9) was used, the middle view shows the result when an alphoid probe (SEQ ID NO. 3) was used, and the lower view was obtained by superposing the above-mentioned two results. Signals of alphoid and β globin are represented by red and green, respectively.

Fig. 15 shows the result of analysis of transcription amount of globin gene in HAC-containing cells. The upper part shows the results of analysis by a RT-PCR, and the lower part shows the results of analysis by a real-time PCR.

Fig. 16(a) shows a chimeric mouse created by using HAC-containing ES cell lines. Fig. 16(b) shows the results of PCR analysis of DNA derived from various organs of a child mouse (24 hours after its birth) obtained by natural childbirth from a mouse (provisional parent) transplanted with an embryo into which HAC-containing ES cells are introduced. TT2 indicates an ES cell, TT2/GCH2-10 indicates HAC-containing ES cell, brain indicates the brain, heart indicates the heart, thymus indicates the thymus, liver indicates the liver, spleen indicates the spleen, and kidney indicates the kidney, respectively. Furthermore, c1 to c15 indicate individual bodies, respectively. Fig. 16(c) shows the results of FISH analysis of a mouse individual body created by using ES cells. Signals of the alphoid array and signals of BAC vector are observed (arrow head).

Fig. 17 shows a chimeric mouse created by using XO nuclear type ES cell lines containing HAC.

Fig. 18 shows a characterized portion of an acceptor precursor BAC-LCR-lox71 used for construction of a mammalian artificial chromosome.

Fig. 19 shows the results of measurement of EGFP intensity in an artificial chromosome constructed by using a precursor including human B globin LCR and a lox site. HAC: artificial chromosome constructed by using a precursor including human B globin LCR and a lox site, INT1 and INT2: two cell lines with highest two fluorescence intensities selected from stable cell lines into which pEGFP-C1 is introduced on random places of the chromosome. The lower right graph summarizes measurement results.

## BEST MODE FOR CARRYING OUT THE INVENTION

The first aspect of the present invention relates to a production method of a mammalian artificial chromosome and includes a method using a circular vector as a precursor and a method using a linear vector as a precursor. Note here that a mammalian artificial chromosome is also referred to as MAC and this includes a human artificial chromosome (hereinafter, which is also referred to as "HAC").

(Vector as mammalian artificial chromosome precursor)

In the present invention, as a mammalian artificial chromosome (MAC) precursor, a first vector (circular vector or yeast artificial chromosome) and a second vector (circular vector or yeast artificial chromosome) are used. The first vector includes a mammalian centromere sequence and supplies centromere necessary for replication and maintaining of MAC. On the other hand, the second vector includes a functional sequence and becomes a source of a functional sequence incorporated into the MAC. It is possible to use plural kinds of second vectors including different functional sequences therein. That is to say, for example, MAC of the present invention can be produced by using, for example, a first vector and two kinds of vectors including different functional sequences therein. In this way, when plural kinds of second vectors are used, it is possible to construct a MAC that holds a plurality of functional sequences in a state of being capable of expressing. This signifies that the MAC of the present invention can be used as, for example, a tool for introducing a plurality of genes which are acting cooperatively.

As the first vector and second vector, circular vector or linear vector can be used. As the circular vector, a BAC (bacterial artificial chromosome) or a PAC (P1 artificial chromosome) capable of autonomously replicating in bacteria (for example, E. coli) can be used. It is advantageous to use BAC or PAC in that introducing operation, amplification and maintaining, etc. are easy and various kinds thereof are available.

The circular vector used in the present invention can be constructed by providing necessary modification for a known BAC or PAC. For example, Belo-BAC (New England Biolabs inc., Beverly, MA 01915-5599) is used as the starting material, and an insertion site for a mammalian centromere sequence is produced therein by restriction enzyme treatment, etc., followed by inserting a mammalian centromere sequence, which has separately been prepared, into this insertion site. Thereby, the circular vector (first vector) including a mammalian centromere sequence can be constructed. On the other hand, the vector (second vector) including a functional sequence can be prepared from a library if a library including the clone thereof is provided. Needless to say, similar to the first vector, the second vector also may be produced from a known vector by genetic engineering technique.

As the linear vector, a DNA construct (yeast artificial chromosome, hereinafter, which is also referred to as "YAC") that functions as a chromosome in yeast is used. The first vector in this case includes at least a mammalian centromere sequence and a telomere sequence. Herein, "mammalian telomere" denotes a repeat sequence existing in the telomere region of a chromosome in mammalians. Human telomere is composed of repeated 5'-TTAGGG-3'. It is preferable to use a centromere sequence including the repetition of this sequence when a human artificial chromosome (HAC) is produced.

It is preferable that the first vector and/or the second vector include a selection marker gene. It is advantageous because when the transformation (transfection) is carried out by using these vectors, transformed cells can be selected easily by using the selection maker gene. It is preferable that only one of the vectors includes a selection marker gene. It is advantageous because by reducing the number of selecting makers to be used, selection operations necessary for the process of the production of a MAC or the use thereof can be simplified.

Furthermore, it is preferable that only the first vector includes a selection maker gene. According to such a configuration, by using the selection marker gene, it is possible to select transformed cells into which mammalian centromere sequences are appropriately introduced. In other words, it is possible to effectively select transformed cells with high possibility of containing DNA contracts that function as a chromosome. On the other hand, since it is not necessary to insert a selection marker into a vector (second vector) including a functional sequence, advantageously, intact vectors prepared from a commercially available library consisting of clones without including selection marker genes are used (i.e., without carrying out the insertion of the selection marker gene) as the second vector. In addition, since the second vector need not include a selection marker gene, the insert DNA to be inserted into the second vector has room by the size of the selection marker gene. As a result, it is possible to construct a MAC containing a larger sized functional sequence.

(Mammalian centromere sequence)

In the present invention, "mammalian centromere sequence" denotes a sequence that functions as a centromere in mammalian cells. As the mammalian centromere sequence, for example, a sequence derived from an alpha satellite region of a human chromosome can be used. Herein, "a sequence derived from an alpha satellite region" denotes a part or entire of the alpha satellite region or a sequence obtained by partially modifying any of the sequences. Herein, "partially modifying" denotes substitution, deletion, insertion and/or addition of one or plurality of bases in the sequence of interest. Such modification may be given to a plurality of regions.

In the alpha satellite region of a human chromosome, in general, a plurality of sequences referred to as a CENP-B box consisting of 5'-NTTCGNNNNANNCGGGN-3' (SEQ ID NO: 1) are disposed at regular intervals (Masumoto et al. NATO ASI Series. vol. H72, Springer-Verlag. pp31-43, 1993; Yoda et al. Mol. Cell. Biol., 16, 5169-5177, 1996). The mammalian centromere sequence of the present invention preferably includes a region having this CENP-B box with high frequency.

It is preferably to use a sequence derived from an alpha satellite region of a human chromosome 21. The alpha satellite region of the human chromosome 21 has been investigated in detail and has a region called α21-I. The α21-I region includes a sequence called an alphoid 11mer repeat unit. This repeat unit includes a plurality of CENP-B boxes consisting of 5'-NTTCGTTGGAAACGGGA-3' (SEQ ID NO: 2) at regular intervals (Ikeno et al. Human Mol. Genet., 3, 1245-1247, 1994).

Preferably, the mammalian centromere sequence of the present invention includes a plurality of such alphoid 11mer repeat units. A sequence isolated from the alphoid region of the human chromosome 21 so as to be identified is shown by SEQ ID NO: 3 (about 25 kb alphoid fragment).

The centromere sequence has a sufficient length to form a centromere having an appropriate function in the constructed mammalian artificial chromosome. For example, a centromere sequence having a size of about 25 kb to about 150 kb (for example, about 50 kb, about 80 kb and about 100 kb) is used. A centromere sequence having size of preferably about 80 kb or less and further preferably about 50 kb or less is used. The use of a small-sized centromere sequence facilitates operations such as separation, purification of the first vector including the centromere sequence, and furthermore reduces the probability of exfoliation and modification, which possibly occur at the time of cloning and/or proliferation. Herein, as shown in Examples mentioned later, in an example in which a circular vector (BAC) was used, even in the case where about 50 kb alphoid DNA was used as a centromere sequence, it was confirmed that an artificial chromosome capable of appropriately forming a centromere/kinetochore structure was constructed. Similarly, in an example in which a linear vector (yeast artificial chromosome) was used, even in the case where about 80 kb alphoid DNA was used as a centromere sequence, it was confirmed that an artificial chromosome capable of appropriately forming a centromere/kinetochore structure was constructed.

The mammalian centromere sequence can be prepared from an appropriate human cell, fusion cell containing human chromosome such as WAV17, or non-human mammalian cells. For example, one of these cells is fixed as an agarose plug, followed by purifying and condensing DNA fragments including the target centromere sequence by way of restriction enzyme treatment, pulsed-field gel electrophoresis (hereinafter, referred to as "PEGE") and the like. Then, the DNA fragments are cloned to an appropriate vector and stored before use.

On the other hand, when the library including a clone containing a mammalian centromere sequence is available, it is possible to obtain a mammalian centromere sequence appropriately from the library by way of restriction treatment. For example, $\alpha$21-I alphoid fragment is obtained by using the LL21NC02 library (Lawrence Livermore Laboratory) and this fragment can be used as a mammalian centromere sequence. A mammalian centromere sequence may be constructed by using a plurality of the obtained $\alpha$21-I alphoid fragments. Furthermore, a plurality of $\alpha$21-I alphoid fragments which differ in size from each other are obtained and by combining these fragments, a mammalian centromere sequence may be constructed.

(Mammalian replication origin)

In general, a mammalian centromere sequence has one or more replication origins. Therefore, usually, the first vector including a mammalian centromere sequence includes a mammalian replication origin. In the case where the mammalian centromere sequence does not include mammalian replication origin, the first vector or the second vector is allowed to include a mammalian replication origin additionally. However, this is not required when the functional sequence contained by the second vector has already include a mammalian replication origin.

(Functional sequence)

The functional sequence is a sequence capable of exhibiting specific effects by the expression thereof and typically consists of a sequence encoding the target gene and the regulatory region thereof. As the functional sequence of the present invention, a sequence having a function of suppressing the expression of a certain gene and suppressing the activity of a certain RNA upon expression thereof, and the like, for example, a sequence encoding a so-called antisense RNA or ribozyme RNA, etc., can be used.

As the target gene, various genes can be employed and examples thereof may include a human guanosine triphosphate cyclohydrolase I (GCH1) gene, human $\beta$ globin gene cluster, a tumor suppressor gene such as RB and p53, an apoptosis induction gene such as c-myc and p53, genes encoding cytokine, various growth factors, antibody, tumor antigen, etc. and the like. A sequence encoding the target gene may be genome DNA or cDNA.

As the functional sequence, it is possible to use a sequence encoding a plurality of target genes. As such a sequence, a sequence including a base sequence corresponding to a plurality of proteins in a case where the plurality of proteins are interacting with each other so as to obtain a specific effect, and a sequence including a base sequence corresponding to a plurality of enzymes necessary for a series of reaction system. In such cases, it is possible to use a sequence for controlling the expression for each sequence corresponding to each expression product. However, a sequence capable of controlling the expression of all or a part (two or more) expression product as a whole may be used. For example, a construct configured by disposing sequences corresponding to a plurality of expression products under the control of one promoter sequence may be used.

Sequence of the target gene can be prepared by, for example, a known library. In a case where a library consisting of vector clones including a sequence of the target gene (and the regulatory region thereof) is available, a vector containing a sequence of the target gene (and regulatory region thereof) prepared from the library can be used as the second vector (or production material thereof). For example, BAC libraries such as CITB (California Institute of Technology) Human BAC Libraries, RPCI-11 (Roswell Park Cancer Institute) Human BAC Library (Keio University), CITB Mouse BAC Library, RPCI-22 Mouse BAC Library, etc., PAC libraries such as RPCI Human PAC Libraries, RPCI-21 Mouse PAC Library, etc., or YAC libraries such as CEPH Human YAC Library, Washington University Human YAC library, WI/MIT 820 YAC Library, Whitehead I Mouse YAC Library, etc. (which are provided by Reseach Genetics, 2130 Memorial Parkway SW, Huntsville, AL 35801, US) can be used.

In the present invention, since a vector with large cloning capacity is used, a large-sized DNA fragment including a regulatory region in addition to the structural gene can be used as a functional sequence. In principle, the regulatory region herein means the regulatory sequence of a the target gene (a sequence of the region directly involved in the regulation of the target gene in the chromosome), however, it may include a sequence in which partial modification is provided to this as long as the function is maintained. "Partial modification" herein denotes substitution, deletion, insertion and/or addition of one or plurality of bases in the sequence of interest. Such modifications may be done to the plurality of regions.

In accordance with the present invention a second vector including a sequence for specifically inserting a sequence of interest (in the present invention, which is referred to as "inserting sequence") as a functional sequence is used. By using such a second vector, it is possible to construct a general-purpose mammalian artificial chromosome (MAC) to which a predetermined sequence can be inserted later. The sequence of interest herein denotes typically a sequence encoding genes of interest (preferably, a sequence including a sequence encoding the regulatory region together). However, the sequence is not particularly limited thereto and may be a sequence having a function of suppressing a predetermined gene or a function of suppressing a predetermined RNA, and the like. For example, the sequence may be a sequence encoding a so-called antisense RNA or a ribozyme RNA, etc.

The kinds of the inserting sequences are not particularly limited, but loxP site or FRT (Flp Recombination Target) site can be preferably used. For example, when the loxP site is used, firstly, a MAC having the loxP site is produced and Cre recombinase is allowed to act on this, whereby a sequence of interest can be introduced site-specifically and finally a MAC including the sequence of interest can be constructed. Similarly, when a MAC having an FRT site is produced, Flp ricombinase is used so as to finally construct a MAC including a sequence of interest. Note here that even a sequence obtained by modifying a part of the lsxP site or the FRT site, etc. can be used as an inserting sequence as long as it has a function for inserting a sequence of interest. Examples of modification include deletion, addition or substitution of a part thereof, thereby increasing the introduction efficiency or enabling only introduction reaction to be carried out specifically.

By adjusting the ratio of the first vector including a mammalian centromere sequence and the second vector including the inserting sequence as a functional sequence, it is possible to change the number of inserting sequences incorporated into a mammalian artificial chromosome to be produced. Furthermore, when the mammalian artificial chromosome is produced by the co-introduction of such first vector and second vector, it is possible to incorporate the inserting sequence at a distance from a centromere (i.e. location which is not between centromere) in a mammalian artificial chromosome to be produced, so that a mammalian artificial chromosome that holds an insertion sequence functioning appropriately can be constructed.

The second vector to be used in the present invention has an insulator sequence. Herein, the insulator sequence is a base sequence characterized by exhibiting an enhancer blocking effect (expressions of neighboring genes are not affected by each other) or a chromosome boundary effect (a region assuring the gene expression and a region suppressing the gene expression are separated with each other). It is expected that the use of the insulator sequence promotes the expression of a target gene contained by a mammalian artificial chromosome. On the other hand, as shown in Examples mentioned below, when the above-mentioned inserting sequence such as loxP, etc. is used, if the insulator sequence is used together, it was found that the introduction rate of the target gene into the mammalian artificial chromosome was increased. Thus, when the insulator sequence is used, the effect of increasing the rate of introducing genes into the mammalian artificial chromosome can be exhibited. Therefore, it is possible to construct effectively and more certainly the mammalian artificial chromosome that holds the target gene. Usable insulator sequences are not particularly limited. It is possible to use not only an insulator, which has been identified as an insulator, but also a sequence obtained by providing modification for the sequence as long as the expected effect (the increase in promoting the expression of target gene or the increase in the gene introduction efficiency) is not reduced. A plurality of insulator sequences may be used together. When a plurality of insulator sequences are used one kind of insulator sequence may be used or plural kinds of insulator sequences in combination may be used. Note here that human β globin HS1 to 5, chicken β-globin HS4, Drosophila gypsy retrotransposon, sea urchin 5' flanking region of arylsulfatase, blocking element α/d of human T-cell receptor α/d, repeat organizer of Xenopus 40S ribosomal RNA gene, and the like, have been known as insulator sequence.

Concrete examples of the mammalian artificial chromosome precursor (second vector) used in the case where the insulator sequence is used include one having an inserting sequence of loxP etc. as a functional sequence and having an insulator sequence at the 5' side of the inserting sequence can be used.

In the mammalian artificial chromosome precursor (second vector), an insulator sequence may be disposed at 3' side instead of 5' site of the inserting sequence. Alternatively, a mammalian artificial chromosome precursor (second vector) in which insulator sequences are disposed at both sides so that they sandwich the inserting sequence. Furthermore, when an insulator sequence is disposed at any positions, a plurality of insulator sequences may be continuously disposed or may be disposed with other sequence interposed therebetween.

(Host cell)

As a host cell into which the first vector and the second vector are introduced, a host cell in which the recombination of the both vectors is carried out can be used. For example, human fibroblast cell line such as HT1080 cells, HeLa cells, CHO cells, K-562 cells, and the like may be used as a host cell.

(Production method for mammalian chromosome)

The production method of the mammalian artificial chromosome (MAC) of the present invention as characterised in the claims includes (1) a first step of introducing a first vector including a mammalian centromere sequence and a second vector including an insertion sequence for specifically inserting a sequence of interest and comprising an insulator sequence functional sequence into a mammalian host cell; (2) a second step of selecting transformed cells; and (3) a third step of selecting a cell containing a MAC from the selected transformed cells.

The method of introducing the first vector and the second vector in the first step is not particularly limited. However, it is preferable that these two vectors are introduced into the mammalian host cell at the same time. It is advantageous because recombination between the vectors in the mammalian host cell is carried out efficiently. It is also advantageous because introduction operation can be simplified. For introducing two vectors at the same time, for example, firstly both vectors, which were mixed with each other prior to the introduction operation, may be introduced into the host cell.

The amount ratio of the first vector and the second vector to be introduced is, for example, first vector : second vector =about 10 : 1 to about 1 : 10 in a molecular ratio so that a MAC containing a functional sequence in a state capable of expressing is appropriately formed. Preferably, the ratio is first vector: second vector =about 1 : 1. Herein, when the amount of the first vector is too small, a MAC including active centromere may not be formed. Meanwhile, when the amount of the second vector is too small, a functional sequence may not be taken into a MAC. On the other hand, the increase in the amount of the second vector enables efficient taking of the functional sequences. As a result, the construction of the MAC including plural copies of the functional sequences can be expected. As shown in the following example, according to the production method of the present invention, the construction of mammalian artificial chromosomes containing plural copies of a target gene has been achieved. In the MAC including plural copies of a target gene, the total amount of expression of the target genes is necessarily increased. Therefore, in the case where the MAC of the present invention is used as a vector for introduction the target genes, high expression efficiency in the cell, in which the MAC has been introduced, can be obtained. This is particularly useful in the case where the MAC of the present invention is used as a vector for gene therapy. This is also beneficial in the case where the MAC of the present invention is used as a material for evaluating the operation/effect of drugs or candidate compounds of drugs.

The method of introducing each vector into the host cell is not particularly limited. Methods such as lipofection (Felgner, P.L. et al., Proc. Natl. Acad. Sci. U.S.A. 84,7413-7417(1984)), transfection using calcium phosphate, microinjection (Graessmann,M. & Graessmann,A., Proc. Natl. Acad. Sci. U.S.A. 73,366-370(1976)), electroporation (Potter,H. et al., Proc. Natl. Acad. Sci. U.S.A. 81, 7161-7165(1984)), and the like can be employed.

In the host cell, the recombination between the first vector and the second vector occurs. As a result, a MAC including a centromere sequence derived from the first vector and a functional sequence derived from the second vector can be formed.

After the first vector and the second vector are introduced, transformed cells (transformants) are selected (second step). The selection of the transformed cells can be carried out by selectively culturing the cells after introduction of the vectors by using the selection marker gene which was inserted in the first vector or second vector in advance. Note here that as a result of isolating cells arbitrarily from the cell group to which both vectors were introduced, the isolation operation in the case where the isolated cells are transformed cells is encompassed in the "selection of transformed cells" according to the present invention.

After the transformed cells are selected, a cell containing a MAC is selected (third step). Such a selection operation can be carried out by a detection method using a probe or antibody specific to MAC. Concretely, for example, it can be carried out by in situ hybridization method using a probe that hybridizes specifically with respect to at least a part of the mammalian centromere sequence included in the first vector. In this step, in order to confirm that a MAC is formed in which the second vector is appropriately incorporated, it is preferable to carry out the similar hybridization analysis using a probe that specifically hybridizes at least a part sequence (for example, functional sequence) specific to the second vector. For detection of each probe used in the above mention, fluorescent substance, radioactive substance, etc. can be used. A method of using a fluorescent substance as a label of the probe is referred to as FISH (Fluorescence in situ hybridization) method and enables safe and simple detection of MAC (Lawrence, J. B. et al. Cell 52:51-61, 1998; Takahashi, E. et al. Jpn. J. Hum. Genet. 34:307-311, 1989).

It is preferable to carry out a step of confirming that MAC in which a functional sequence is appropriately incorporated is formed in addition to the third step. Such a confirming step can be carried out, for example, by detecting the expressed product of the gene in a case where the functional sequence includes the target gene.

The mammalian artificial chromosome (MAC) obtained by the above-mentioned production method can be maintained extremely stably even under the non-selective conditions. Note here that "non-selective condition" means a condition that dose not include the selective operation enabling the existence of only cells in which a MAC is present.

Although it may be different depending upon the kinds of precursor vectors and host cell to be used, etc., according to the production method of the present invention, it is possible to allow about 95% or more of cells (group) to hold a MAC after about 30 days (after about 30 passages) under non-selective conditions after DNA construct (first vector and second vector) is introduced into the host cell. Furthermore, it is possible to maintain the state that one copy of MAC is present in the cell (see Example mentioned below).

It is preferable that the number of MACs contained by the finally obtained transformed cells (mammalian cells) is fewer, and it is particularly preferable that one MAC per nucleus is contained. According to the production method of the present invention, it is possible to efficiently obtain transformed cells containing one mammalian artificial chromosome per nucleus.

Another aspect of the present invention is to provide a transformed cell (transformant) containing a mammalian artificial chromosome (MAC) produced by the above-mentioned method. Such a transformed cell can be used as a supply source for transferring MACs to the other cells. Furthermore, such a transformed cell can be used as a carrier for introducing a mammalian artificial chromosome into the living body by, for example, introducing the transformed cell per se into the living body.

(Properties of mammalian artificial chromosome)

The mammalian artificial chromosome (MAC) constructed in the present invention is characterized by (1) having a mammalian replication origin, a mammalian centromere sequence, and an insertion sequence for specifically inserting a sequence of interest and comprising an insulator sequence (a sequence encoding a target gene and the regulatory region thereof or an inserting sequence for inserting a sequence of interest); (2) being replicated in mammalian cells; (3) being maintained extrachromosomally in a host cell; (4) being transmitted to daughter cells at the time of cell division; and (5) being circular or linear in form. In a case where the MAC is produced by using a circular vector (BAC or PAC) as a precursor, its form becomes circular because a telomere sequence is not included. On the other hand, in a case where the MAC is produced by using a linear vector (yeast artificial chromosome) as a precursor, it is thought that when telomere sequences that function sufficiently are provided at the both ends, the MAC has a linear form and that if not so, the MAC has a circular form. Note here that the mammalian replication origin may exist in a mammalian centromere sequence.

According to the above-mentioned characteristics, the MAC of the present invention functions as a chromosome in a mammalian cell into which a MAC is introduced and is appropriately segregated to daughter cells so as to be maintained without accompanying substantial change of the structure at the time of cell division.

Furthermore, in the MAC of the present invention, the target gene of interest can be maintained together with its regulatory region and allowed to express the target gene sufficiently in the cell into which the MAC is introduced. Note here that as shown in Examples mentioned below, in an example in which GCH1 gene was used as the target gene, wee realized regulation of expression that is same as in the case existing on the chromosome.

The mammalian artificial chromosome of the present invention may include a DNA sequence which enables the mammalian artificial chromosome to autonomously replicate and being segregated in cells other than mammalian cells (for example, yeast cells, bacteria such as E. coli). Since such a DNA sequence is included, the MAC of the present invention can function as a chromosome also in cells other than mammalian cells. Therefore, the MAC of the present invention can be used as a shuttle vector.

It is preferable that a mammalian centromere sequence include a CENP-B box sequence. It is particularly preferable that a region expressing CENP-B boxes with high frequency is included. Furthermore, it is preferable that the mammalian centromere sequence includes a sequence derived from alpha satellite region of the human chromosome 21, and particularly a sequence of $\alpha$21-I alphoid region.

As shown in Examples mentioned later, the present inventors succeeded in production of a human artificial chromosome (HAC) containing about 180 kb gene encoding human GCH1 (EC 3.5.4.16; GCH1) in a state capable of expressing in a system using a BAC as a precursor. One human GCH1 gene is located in the chromosome 14q22.1-q22.2 and the gene is composed of six exons spanning more than 60 kb (Fig. 1) (Ichinose et al. 1995; Hubbard et al. 2001). GCH1 is the first enzyme for the biosynthetic pathway of tetrahydrobiopterin, the essential cofactor for enzymatic reactions as described below and is present in higher organisms (Nichol et al. 1985; Tanaka et al. 1989; Werner et al. 1990). Tetrahydrobiopterin is synthesized from GTP in a three-step reaction by GCH1, 6-pyruvoyl-tetrahydropterin synthase (EC 4.6.1.10; PTPS) and sepiapterin reductase (EC 1.1.1.153; SR). Among these enzymes, the major controlling point is GCH1, the expression of which is under the control of cytokine induction (Werner et al. 1993) and the feedback regulatory protein, GFRP, at the transcriptional and post-translational levels, respectively. Tetrahydrobiopterin functions as a natural cofactor of the aromatic amino acid hydroxylases; phenylalanine hydroxylase (EC 1.14.16.2; PAH), tyrosine hydroxylase (EC 1.14.16.3; TH), the first and rate-limiting enzyme of dopamine synthesis, tryptophan 5-hydroxylase (EC 1.14.16.4; TPH), serotonin biosynthesis. Tetrahydrobiopterin is also essential for all three forms of nitric oxide synthase (NOS) (Kaufman 1993). Decreases in GCH1 activity, tetrahydrobiopterin level and/or TH activity causes dopamine deficiency in the nigrostriatum dopamine neurons and provokes several well-known clinical symptoms, such as hereditary dopa-responsive dystonia (DRD/Segawa's syndrome) (Ichinose et al. 1994) or parkinsonism. Thus, HACs carrying the GCH1 gene with the authentic regulatory region would almost certainly prove useful for compensating for the defects in the GCH1 gene as well as facilitating a close study of the complex regulatory mechanism of GCH1 gene expression *in vivo.*

(Transfer of mammalian artificial chromosome)

The introduction of a mammalian artificial chromosome (MAC) into a mammalian cell can be carried out by, for example, the following method.

First of all, from a host cell containing a MAC, the MAC is isolated. The isolated MAC is introduced into a mammalian cell (target cell). The isolation of MAC can be carried out by, for example, the following method. First of all, suspension of the host cells containing the MAC is prepared and a nucleic acid component is extracted. Thereafter, fractions containing a chromosome is obtained by density-gradient centrifugation using Ficoll, etc. Then, artificial chromosomes with small molecular weight are separated by using a filter, etc.

An example of the method of introducing the separated MAC into mammalian cells includes lipofection, transfection using calcium phosphate, microinjection, electroporation, and the like.

A MAC can be introduced into mammalian cells by the following method using cell infusion. First of all, host cells containing a MAC and mammalian cells capable of forming micronuclei are fused to each other, followed by selecting hybrid cells which are capable of forming micronuclei and hold MAC from the fused cells. Herein, as the mammalian cells capable of forming micronuclei, for example, A9 cells (American Type Culture Collection, Manassas, VA 20110-2209), mouse ES cells, CHO cells, and the like can be used. The cell infusion can be carried out by using PEG (Polyethlene Glycol). The selection of the target hybrid cells can be carried out by a selection culture using a selection marker specific to the host cell used in the cell infusion and ouabain in the case where, for example, mouse A9 is used.

Then, micronuclei are formed from the selected hybrid cells. In general, micronucleate multinuclear-cells are formed by colcemid treatment, followed by carrying out cytochalasin B treatment and centrifugation so as to micro-cells.

The micro-cells are fused to mammalian cells (target cells) by fusion using PEG, etc. From the above-mentioned step, MACs are transferred (introduced) to mammalian cells, so that mammalian cells containing the MAC can be obtained.

Herein, example of the target cells include cells forming a certain tissue of human or non-human mammalian (mouse, rat, etc.) (fibroblast cells, endothelial cells, cardiac muscle cells), non-human germ cells (including a fertilized egg), non-human embryonic stem cells (ES cells), non-human embryonic germ cells (EG cells), tissue stem cells (hematopoietic stem cells, mesenchymal cells, nervous system stem cells, osseous system stem cells, cartilage stem cells, epithelial stem cells, hepatic stem cell, etc.), and the like. Cells obtained by providing such stem cells with induction treatment for allowing them to differentiate into cells of specific tissue can be used as the target cells. Examples of such target cells include cells obtained by differentiated-inducing nervous system stem cells to neuron, astrocyte and oligodendrocyte by using a platelet-derived growth factor (PDGF), a ciliary derived neurotrophic factor (DNTF) and triiodothyronine (T3), respectively; cells obtained by differentiated-inducing mesenchymal cells to osteoblast by using dexamethasone and ascorbic acid, and the like; and cells obtained by differentiated-induciong mesenchymal cells to cartridge cells by culturing in the presence of TGF-$\beta$, etc.

As the cells into which the MAC of the present invention is introduced, cells of vertebrate animal other than mammalian, for example, Pisces (Aplocheilus latipes, zebrafish, etc.), Amphibia (Xenopus laevis, etc.), Aves (chicken, quail, etc.), and the like may be used.

The transferring of the MAC into the target cells is carried out in vitro, ex vivo or in non-human cells in vivo. For example, by directly transferring a mammalian artificial chromosome (MAC) into the non-human cells in vivo, or by introducing cells into which a MAC is transferred ex vivo into a living body, the MAC can be introduced into the site of interest (for example, specific tissue such as heart, lungs, etc.). As a result, expression is carried out from a functional sequence contained in the MAC in the introduction site. In this way, a MAC can be used as a vector for introducing a foreign gene into the living body. Since a MAC has a large cloning capacity, in particular, it can be preferably used as a vector for introducing a large foreign gene including a regulatory region.

More concretely, the mammalian artificial chromosome (MAC) of the present invention can be used as a vector for, for example, gene therapy. That is to say, the MAC of the present invention can be used for the introduction of foreign genes for the purpose of compensating the function of defective genes, suppression of expression of abnormal genes, or suppression of the effect of the expressed products. Since the MAC of the present invention can be maintained stably in the cell into which the MAC is introduced, the transgene is expressed stably and for a long term. Thus, excellent therapy effect can be expected. Furthermore, since a large-sized foreign gene including regulatory region can be introduced when the MAC of the present invention is used, gene expression under the control of original regulatory region can be carried out. Also from this viewpoint, excellent therapy effect can be expected.

Furthermore, the MAC of the present invention also provides a means for clarifying the function or the action mechanism of the gene of interest. In particular, it is useful to provide a means for clarifying the function or action mechanism of a gene, which was not able to be introduced by a conventional vector due to it large size. That is to say, it provides a means for studying of a gene whose function or action mechanism is unknown. In particular, since the MAC of the present invention can hold foreign genes so that they can express under the control of the original regulatory region, analysis of tissue specific expression mechanism or analysis of expression of a human gene which has been introduced into a mouse, and the development of inhibitors and promoters.

As shown in Examples mentioned later, the present inventors succeeded in creating a mouse (chimeric mouse) into which the mammalian artificial chromosome (MAC) of the present invention is introduced by using ES cell. Note here that the present inventors succeeded in not only the creation of chimeric mouse (male) using XY nuclear type ES cells but also the creation of chimeric mouse (female) using XO nuclear type ES cells. Thus, it was confirmed that the mammalian artificial chromosome of the present invention could be used for creating transformed animals, in particular mice. Based on such results, another aspect of the present invention relates to transformed mice comprise a mammalian artificial chromosome of the present invention .

The transformed mice can be created by introducing the MAC at its development stage. As the creating method, a method using ES cells, a microinjection method in which introduction of nucleus construct (MAC) is directly infused to the pronucleus of fertilized egg, and the like, can be employed. Hereinafter, as a concrete example of the method of creating the transformed mice of the present invention, a method using mouse ES cells will be described. In this method, first of all, ES cells containing a MAC are prepared. Such ES cells can be prepared by using the above-mentioned micronucleus fusion method. That is to say, first of all, cells containing a MAC having a desired configuration (for example, HT1080) are prepared and fused to cells having the ability of fusing micronuclei (for example, mouse A9 cells) so as to transfer the MAC. Thereafter, micronucleus is formed by, for example, colcemid treatment from cells into which the MAC is appropriately transferred. The obtained micronucleus is fused to ES cells by, for example, use of PEG, and the like. Then, from the fused cells, one containing the MAC is selected. The thus prepared ES cells containing the MAC are introduced into the blastocyst of mouse. That is to say, first of all, after the entire uterus including the ovary is extracted from the mated female mouse, the blastocyst is collected from the uterus, and ES cells containing a HAC is introduced into the blastocyst cavity of the blastocyst by microinjection. Then, the blastocyst which the injection was completed is transplanted into the uterus pseudopregnancy mouse (provisional parent) so as to obtain a child mouse (fetus) by natural childbirth or Cesarean section.

Note here that it can be confirmed that the MAC is introduced into the obtained child mouse by observation of hair color of the child mouse or DNA analysis using a probe having a sequence specific to the used MAC.

<EXAMPLE 1> Construction of alphoid-BAC

pBAC-TAN was created by insertion of a MluI-SfiI-SacII linker into the XhoI site of Belo-BAC. pBAC-CMV and pBAC-SV were created by insertion of a 1.3 kb NotI-HindIII fragment from pCMV/Bsd (Invitrogen) or a 2.6 kb PvuII-EcoRI fragment from pSV2bsr (Kakenseiyaku), both contain a Blasticidin S resistance gene, into the NotI-HindIII sites of pBAC-TAN. The 25 kb alpha 21-I alphoid fragment ($\alpha$25: SEQ ID No: 3) was isolated from the cosmid clone, Q25F12, obtained from the LL21NC02 library (Lawrence Livermore Laboratory) by SfiI digestion and cloned into the SfiI site of pBAC-TAN. The resulting alphoid-BACs which contain either 50 kb or 100 kb of tandem alphoid insert were digested with MluI and SacII, and the alphoid fragments were inserted into the MluI-SacII sites of pBAC-CMV or pBAC-SV, respectively. As a result, SV/$\alpha$50 and CMV/$\alpha$100, which are alphoid-BACs containing 50 kb (SV/$\alpha$50) and 100 kb (CMV/$\alpha$100) alphoid fragments, were obtained (Fig. 3).

<EXAMPLE 2> Generation of HAC containing the GCH1 genomic locus

Alpha 21-I alphoid, consisting of an 11mer higher order repeat unit derived from human chromosome 21 (Ikeno et al. 1994), is able to generate a HAC efficiently when introduced into HT1080 cells (Ikeno et al. 1998). We generated HACs containing a GCH1 genomic locus with naturally regulated gene expression, utilizing alphoid-BACs and GCH1-BAC. BACs used in this study are shown in Figure 3. CMV/a100 contains 100 kb of an $\alpha$21-I alphoid array and a CMV-Bsd as a selectable marker, and SV/a50 contains 50 kb of an $\alpha$21-I alphoid array and a SV2-Bsr selection marker. The GCH1-BAC was obtained from a BAC library (Genome systems) and has a 180 kb genomic DNA fragment containing the GCH1 gene. BAC-DNAs were purified by CsCl banding using a gradient.

We co-transfected either one of the alphoid-BACs and the GCH1-BAC in a 1: 1 molecular ratio into HT1080 cells by lipofection and isolated Blasticidin S (BS) resistant cell lines after 10 days. Specifically, for generation of HAC, 0.5 $\mu$g of alphoid-BACs and 1.0 $\mu$g of GCH1-BAC (186L09, Genomesystems) were co-transfected into HT1080 cells (5 x $10^5$) using lipofectamine (Gibco BRL) according to the manufacturer's instructions. The cells were selected with 4$\mu$g/ml Blasticidin S (BS, Kakenseiyaku) and colonies were picked after 10 days.

To detect the presence of HAC as an extrachromosomal element, the BS-resistant cell lines were analyzed by FISH using both $\alpha$21-I alphoid DNA and BAC vector as probes. Namely, metaphase chromosome spreads were prepared on glass slides after methanol/acetate (3: 1) fixation and FISH was carried out according to conventional procedures. For detection of HAC, biotin-labeled alpha 21-I alphoid DNA (11-4) (Ikeno et al. 1994) and digoxigenin-labeled Belo-BAC were used as probes. In dual FISH, biotin-labeled DNA was visualized with FITC conjugated avidin (Vector) and digoxigenin-labeled DNA was visualized with TRITC conjugated anti-digoxigenin (Boehringer Mannheim). Photographs were taken using a CCD camera (Princeton instruments) mounted on a Zeiss microscope. Images were processed using IPLab and Adobe Photoshop 6.0.

One out of 16 transformed cell lines obtained by co-transfection of CMV/$\alpha$100 and GCH1-BAC (HT/GCH2-10), and three out of 17 cell lines obtained by co-transfection of SV/$\alpha$50 and GCH1-BAC (one of them is HT/GCH5-18), contained one copy of HAC per nuclei in more than 95% of the inspected cells. In the remaining cell lines, introduced BACs were either integrated into the chromosomes of HT1080 or the signals were undetectable by FISH analyses.

To examine whether the established HACs contained the genomic fragment of the GCH1 gene, four cell lines containing a HAC were further hybridized with probes for GCH1 exon 1 and exons 4-6 (Fig.3). As a probe for exon 1, 13 kb of biotin-labeled fragment including exon 1 was used, and for probes for exons 4 to 6, 8 kb of digoxigenin-labeled fragments including exons 4, 5 and 6 were used.

The signals for both probes were detected on a HAC in the HT/GCH2-10 cell line which was generated by co-transfection of CMV/$\alpha$100 and GCH1-BAC, and on a HAC in the HT/GCH5-18 cell line which was generated by co-transfection of SV/$\alpha$50 and GCH1-BAC (Fig. 4). The GCH1 signals detected on the HACs were stronger than that of the endogenous gene on the HT1080 chromosomes. As the minority of cells (less than 5%) both in HT/GCH2-10 and HT/GCH5-18 were integrated with the transfected DNA into a chromosome of HT1080, the cell lines were single cell cloned to produce sub-clones containing only one copy of HAC per nuclei and the subsequent re-cloned cell lines were investigated further.

<EXAMPLE 3> Centromere/kinetochore structure and mitotic stability of the HACs

To investigate the centromere/kinetochore structure on the HAC, the presence of essential centromere/kinetochore proteins, CENP-A and CENP-E (Palmer et al. 1991; Yen et al. 1991; Howman et al. 2000) was investigated on metaphase chromosomes of HT/GCH2-10 and HT/GCH5-18 by indirect immunofluorescence as follows. Swollen and 1% paraformaldehyde fixed cells were incubated with anti-CENP-A (Ando et al. 2002) or anti-CENP-E (Santa Cruz) antibodies. Antibody localization was visualized with FITC-conjugated anti-mouse IgG. For subsequent FISH analysis, the cells were fixed again with 1% paraformaldehyde and then with methanol/acetate (3: 1).

CENP-A and CENP-E signals were detected on HACs in doublets corresponding to the paired sister chromatids, and were similarly detected at the centromeres of all endogenous chromosomes (data not shown).

We examined the mitotic stability of the HACs in the cell line HT/GCH2-10 and HT/GCH5-18 under the non-selective conditions. Maintenance of the HAC in each cell line was measured by FISH analysis on metaphase spreads, which were prepared after 10, 20 and 30 days of culturing. On each sampling day, 50 spreads from each cell line were examined and the percentage of cells that carried the HAC was determined. Namely, the following formula: Nn=N0x(1-R)n, where No is the number of the metaphase spreads containing a HAC under selective conditions, and Nn is the number of metaphase spreads containing a HAC after n days of culture under non-selective conditions. Fish analysis was performed in the same way as the method mentioned above.

After 30 days without selection, 95% of metaphase cells in HT/GCH5-18 and 80% of metaphase cells in HT/GCH2-10 retained the HAC and the number of HAC copies per cell was kept at one under non-selective conditions. The integration into host chromosomes was not observed in either cell line. The rate of chromosome loss per day was calculated from the percentages of cells retaining HAC after 30 days under non-selective conditions. The values were 0.2% and 0.5% for HT/GCH5-18 and HT/GCH2-10, respectively. These results indicated that an active centromere/kinetochore structure was formed on the HACs and that the HACs were stably maintained through mitosis.

<EXAMPLE 4> DNA structure of HACs

To determine whether the HACs in HT/GCH2-10 and HT/GCH5-18 were circular or linear, FISH was performed using a telomere sequence and BAC vector as probes. No telomere signal was detected on the HACs when HACs were stained using a BAC vector probe. In contrast, the ends of the chromosomes from the host cell, HT1080, were hybridized as clear speckles. As expected, BAC-derived HACs are likely to be circular in form.

The DNA organization of the HACs was analyzed by restriction digestion of DNA isolated from HT/GCH2-10, HT/GCH5-18 and non-transfected HT1080 cells. The DNA samples (5 $\mu$g) were digested with BamHI or StuI for 4 hours followed by conventional gel electrophoresis. The DNA in the gel was transferred to a nylon membrane and hybridized with $^{32}$P labeled DNA probe prepared from GCH1 exon 6 (2.1 kb) and the upstream region of GCH1 (1.4 kb, position 595-1959 in GCH1-BAC).

The size of BamHI fragments detected by the US probe were 5.0 kb from the endogenous GCH1 gene and 3.5 kb from GCH1-BAC. The 5.0 and 3.5 kb fragments were detected with DNA from HT/GCH2-10 and HT/GCH5-18 at almost the same signal intensity (Fig. 5(A)). The size of StuI fragments detected by the exon 6 probe were 24.5 kb from the endogenous GCH1 gene and 14.4 kb from GCH1-BAC. The 24.5 and 14.4 kb fragments were detected with DNA from HT/GCH5-18 at almost the same signal intensity, while three fragments heterogeneous in size were detected in addition to the endogenous fragment with the DNA from HT/GCH2-10 (Fig. 5(B)). The results indicated that GCH1-containing HACs in HT/GCH5-18 were established by the assembly of about three copies of transfected GCH1-BAC DNA since the karyo-type of HT1080 cells used in this study is 3n, while HT/GCH2-10 was accompanied by some rearrangements of the terminal region of GCH1 exon 6, but it may also contain three copies of GCH1-BAC as judged by the density of the US band. The internal rearrangements of GCH1 genes were confirmed by RT-PCR analyses of GCH1 transcripts in HT/GCH2-10, which revealed the synthesis of abnormal transcripts (data not shown).

The copy numbers of the GCH1-BAC and the alphoid-BAC in GCH2-10 and GCH5-18 HACs were determined by dot hybridization using GCH1 exon 6 and BAC vector, respectively, as probes. Relative copy numbers of each BAC in the HACs were estimated from the hybridization signal-intensity values, which were determined using each DNA probe and standardized using the values obtained with 0.1 ng GCH1-BAC DNA (Fig. 6). In the case where GCH1 exon 6 was used as the probe, the same hybridization intensity values to that obtained with 0.1 ng GCH1-BAC DNA were obtained with 0.5 μg DNA from both HT/GCH2-10 and HT/GCH5-18, and with 1 μg DNA from HT1080 (Fig. 6, Left). Since HT1080 karyo-type used in this study is 3n, three copies of GCH1 genes occur on its chromosomes, and given that HT/GCH2-10 and HT/GCH5-18 resulted in the same signal values with half the amount DNA as that from HT1080, they must contain six copies of GCH1 genes; three on the chromosomes and three on the HAC. The total copy number of BACs was estimated from the intensity values obtained with the BAC vector probe. The same hybridization intensity values to that obtained with 0.1 ng GCH1-BAC were obtained with 0.33 μg DNA from HT/GCH2-10 and HT/GCH5-18, while HT1080 showed no signal as expected (Fig. 6, Right). Therefore, both HACs have roughly 3-fold more copies of the BAC vector than copies of the GCH1 gene. Thus, copy numbers of the total BAC vectors must be approximately nine per cell; three copies of GCH1 genes are in the form of GCH1-BAC and the remaining six copies of BACs must exist in the form of alphoid-BAC in both HACs.

<EXAMPLE 5> Transfer of HAC-containing cell lines to mouse A9 cells

*De novo* HAC formation using cloned alphoid DNA has been successful in the human fibrocarcinoma cell line, HT1080. To determine the natural expression of the GCH1 gene in the neural cell line, the HAC that has been established in the HT1080 cell line needs to be transferred into a neural cell line.

Fusion of the HAC-containing cell lines and mouse A9 cell line was performed using PEG which allows micro-cell mediated chromosome transfer (MMCT) (Fournier et al. 1977). Cell lines containing a HAC (5 x 105) and mouse A9 cells (5 x 105) were co-cultivated and fused in PEG/DMSO solution (SIGMA). BS- and Ouabain- resistant cells were selected with 2.5 μg/ml BS and 3 μM Ouabain. BS- and Ouabain-resistant cell lines were analyzed by FISH. Metaphase spreads were hybridized with a BAC vector probe and Alu repeat probe to identify the HACs and human chromosomes, respectively (Fig. 7(A)). One of the fusion cell lines, F/GCH5-18, contained one or two copies of HAC together with eight to ten human chromosomes.

The HACs in the fusion cells were maintained stably during mitotic growth under non-selective conditions with a loss of approximately 1% of the mitotic chromosomes per day (data not shown). The mitotic stability of human chromosomes in mouse cell lines was sometimes caused by the acquisition of minor satellite DNA from the mouse which was localized at the centromere of the mouse chromosomes and may serve as functional centromere sequences (Shen et al. 1997). Therefore the presence of mouse minor satellite DNA on HAC was examined by FISH. Signals of minor satellite DNA were not detected on HAC, while strong signals were detected at the centromeres of mouse chromosomes (Fig. 7(B)). The fusion cell lines were able to form micro-cells under colcemid treatment conditions (data not shown). Therefore, the HACs could be easily transferred to neural cell lines.

<EXAMPLE 6> GCH1 expression from HAC

Naturally regulated gene expression was expected from the transgenes in the large genome segments carried by HACs. The GCH1-BAC used in the generation of HACs contained over 100 kb of genomic sequence from the 5' upstream region of the GCH1 exon 1. Therefore, we have measured GTP cyclohydrolase I (GCH1) activities in HT1080 and the HAC-containing derivatives that were developed from it. It would have been expected from the previous report that the activity of GCH1 would have been hardly detectable in fibroblast cell lines but up-regulated by induction of IFN-γ (Werner et al. 1990). GCH1 activity in HT1080, HT/GCH2-10 and HT/GCH5-18 were analyzed in the presence and absence of IFN-γ induction (Fig.2). GCH1 activity was measured as follows. Cells were grown in the absence or presence of human IFN-γ at 250 U/ml in culture medium for 48 h. Trypsinized cells were washed in phosphate-buffered saline (PBS), then lysed in 0.1 M Tris-HCl (pH 8.0), 0.3 M KCl, 2.5 mM EDTA, 10% glycerol. GCH1 activity was measured as described (Hibiya et al. 2000).

HT1080 without GCH1-HAC exhibit barely detectable levels of GCH1 activity in the absence of IFN-λ induction, while the activity was increased fifteen times upon the addition of IFN-λ. In HT/GCH2-10 cell line in the absence of IFN-λ induction, the GCH1 level was three times the values of HT1080 without a HAC. After the IFN-λ induction, nearly 30-fold up-regulation was observed. In contrast, GCH1 activity in HT/GCH5-18 was elevated 70-fold in the absence of IFN-λ and addition of IFN-λ further up-regulated the activity 5-fold. In both HT/GCH-HAC cell lines, the GCH1 activities were elevated but differ in degree, possibly reflecting the difference in chromatin structure and/or DNA rearrangements in HACs. They are still susceptible to IFN-λ induction, just like the response of the expression of the GCH1 gene from the authentic chromosome.

As showed above, we obtained HACs containing large DNA fragments with the GCH1 gene (GCH1-HAC) by simple co-transfection methods using alphoid-BAC and GCH1-BAC at a DNA ratio of 1:1. The GCH1-HAC was maintained at one copy after 30 or more rounds of generation under non-selective conditions in spite of being circular in form without telomeres, indicating that HAC replicates once in each cell cycle and is segregated precisely into daughter cells. Therefore, the circular HACs in this study did not cause topological problem, which may result in the abnormal segregation of the circular chromosomes, since the catenated form arose from DNA replication. The HACs were cytologically megabases in size and approximately 10-fold larger than the transfected BAC DNA.

The DNA structure of the HAC was examined to understand the properties and mechanism of *de novo* generation of HAC. The restriction analysis of the whole area of the GCH1 gene was difficult because almost all rare-cutting enzyme sites in the BAC constructs were subjected to methylation and the cell lines contain the endogenous GCH1 locus. Therefore, we applied restriction analysis to the region corresponding to the junction of the BAC vector and the GCH1 locus. The result showed that GCH1-HACs in the two cell lines contained three copies of the GCH1-BAC and six copies of the alphoid-BAC as components (Fig. 5, 6). Although the exact mechanism of the formation of the HAC remains unknown, GCH1-HAC was composed of multimer of the input DNA, which was similar to the HAC generated by alphoid-YAC (Ikeno et al. 1998) or alphoid-BAC alone (data not shown) as an input element. The fact that the formation of HAC was accompanied by assembly of the distinct BACs indicated that the multimerization of BAC molecules might be mediated by non-specific recombination of input BAC in addition to the amplification of BAC DNA itself.

The generation of the HAC containing large human genomic DNA was previously reported using a 140 kb or 162 kb HPRT locus (Grimas et al. 2001; Mejia et al. 2001). They obtained the HAC containing HPRT gene in the HPRT-deficient HT1080 cell lines in HAT medium depending on complementation. The feasibility of such an approach for genes with tissue and stage specific expression (i.e. not house-keeping gene) will be low in HT1080 cells. In this study, we found that as short as 50 kb of a21-I alphoid DNA in BAC was able to generate HACs (centromere/kinetochore) and that the BACs containing large transgene without a selection markers could be incorporated efficiently into the HAC, since 50% of the HACs included the transgene. Thus, HACs containing any large genomic region of interest could be generated using alphoid-BAC containing 50 kb of alphoid DNA and a readily available BAC library without any modification. Intactness of the incorporated transgenes may be checked after HAC generation.

The selection of transformants with CMV promoter-driven Bsd gene increase the number of BS-resistant cells, but the FISH signals for HACs or integrated loci on the host chromosomes were not found in the majority of transformants (Fig.1). Southern hybridization analyses using alphoid and BAC vector sequences as probes indicated that these cell lines have Bsd genes only integrated in the chromosomes. Thus, the selection marker, driven by a high expression promoter, was not suitable for the screening of HAC-containing cell lines.

Gene expression was affected by chromosome structure. The insertion of a transgene into a chromosome often results in stably inherited gene silencing in a clonal sub-population of the cells, a phenomenon commonly known as position effect variegation (PEV) (Karpen 1994). Recent molecular analysis showed that methylation of histone H3 on lysine 9 contributes to the targeting of HP1 to the chromatin and results in heterochromatinization and the silencing of gene expression (Platero et al. 1995; Bannister et al. 2001; Lachner et al. 2001). Gene silencing at or near the centromere/kinetochore in yeast and fly was also reported (Karpen & Allshire 1997) and was expected to occur in mammalian cells. We have recently demonstrated that in the alphoid array of a HAC, once centomere/kinetochore structure was formed, the expression of the short marker genes inserted into the HACs were repressed strongly even if they were driven by strong promoters (Abe et al. submitted). Thus, to get expression of transgenes in HACs, we will need to solve the topological problem of the genes in relation to the centromere/kinetochore structure.

The GCH1 gene expression from HAC might be correlated with the chromatin structure at or near the GCH1 locus. The present inventors addressed whether the centromere/kinetochore structure was formed on only the alphoid array or whether it spread into the GCH1 locus. Since CENP-A is an essential protein for a functional centromere/kinetochore and constitutes the histone component for centromere specific nucleosomes (Palmer et al. 1991; Howman et al. 2000), we analyzed the chromatin structure on HAC by ChIP using anti-CENP-A antibody (Ando et al. 2002). ChIP method was performed as follows. The nuclei of HT/GCH5-18 cells (5 x 107) were isolated and dissolved in WB (20 mM HEPES (pH 8.0), 20 mM KCl, 0.5 mM EDTA, 0.5 mM dithiothreitol, 0.05 mM phenylmethylsulfonyl fluoride). After digestion with MNase, solubilized chromatin was immunoprecipitated using anti-CENP-A antibody as described previously (Ando et al. 2002).

In such analyses using HeLa and HT1080 cells, the alphoid array was enriched to 60-80% in total immunoprecipitated DNA. The alphoid array in the GCH1-HAC was also enriched by anti-CENP-A antibody, while the GCH1 region was not. In contrast, the BAC vector sequence about 3 kb away from the alphoid sequence was also immunoprecipitated, indicating that the centromere/kinetochore structure was formed on the alphoid array and spread to flanking non-alphoid region (data not shown). The invasion of the GCH1 locus in the HAC by centromere/kinetochore structure, was prevented by an as yet unknown protection mechanism that probably resides in the upstream regulatory sequence.

GCH1 encodes the first and rate-limiting enzyme for the biosynthetic pathway of tetrahydrobiopterin (Nichol et al 1985), the co-factor of aromatic amino acid hydroxylase (PAH, TH, TPH) as well as nitric oxide synthase (NOS) and is present in higher organisms (Kaufman 1993). The GCH1 gene is a causative gene for dopamine deficiency in dopa responsive dystonia (DRD/Segawa's disease) (Ichinose et al. 1994). Deficiency of GCH1 in conjunction with a mutation in the TH gene results in severe early-onset dystonia/parkinsonism (Ichinose et al. 1999).

Although only limited analyses have been performed on the upstream regulatory sequence of the human and mouse GCH1 gene, it has been reported that the CCAAT and TATA boxes are conserved (Ichinose et al. 1995; Hubbard et al. 2002). It was established that GCH1 gene expression could be induced by IFN-$\gamma$ in various rodent and human cells (Werner et al. 1990). However, the exact mechanism involved in IFN-$\gamma$ signal transduction is yet unknown.

Some gene expression, such as that for human beta-globin, was regulated by locus control regions (LCRs) responsible for initiating and maintaining a stable tissue-specific open chromatin structure (Festentein et al. 1996; Milot et al. 1996). The GCH1-HACs used in this study carry a 180 kb genomic fragment containing the GCH1 gene, and therefore may contain the regulatory sequences required for tissue specific expression and for prevention of the silencing effect of the flanking centromere. The expression of the GCH1 gene from HAC was measured by GTP cyclohydrolase I activity in the presence and absence of IFN-$\gamma$ (Fig.2). Activity in the HAC-containing cell line, HT/GCH2-10, was slightly higher than the activity obtained with HT1080 in the absence of IFN-$\gamma$. Addition of IFN-$\gamma$ increased the GCH1 activity approximately 30-fold. In another cell line, HT/GCH5-18, which also carries twice the number of GCH1 genes as HT1080, showed 70-fold higher enzyme activity than TH1080 in the absence of IFN-$\gamma$ and the activity was further increased 5-fold by IFN-$\gamma$ induction. The small difference in values between HT/GCH2-10 and HT1080 may correspond to the small copy numbers of intact GCH1 genes, since it seems that some copies of GCH1 genes on GCH2-10 HAC have the structural abnormality described in the Results section. These results indicated that although the gene expression of GCH1 may be affected by the difference of chromatin structure assembled on the GCH1 locus in the HAC, the genes still responded to IFN-$\gamma$. The final levels of GCH1 activity after IFN-$\gamma$ induction in the cell lines were still repressed and kept to a similar order of magnitude. This might indicate the presence of complex cellular regulation systems to maintain the GCH1 activity in the proper range. The GCH1-HAC should prove to be a suitable system to understand the complex regulatory mechanisms of GCH1 expression *in vivo.*

The adeno-associated virus (AAV) vector was often used for gene therapy in the helper virus-dependent manner for productive infection. The AAV vector has limited cloning capacity that usually carry cDNA without original regulatory sequence for gene expression (Dong et al. 1996). GCH1 is necessary for efficient dopamine production together with tyrosine hydroxylase (TH) and aromatic-L-amino-acid decarboxylase (AADC). Expression of these three enzymes from the AAV vector in the striatum resulted in relatively long-term behavioral recovery in a primate model of Parkinson's disease (Muramatsu et al. 2002).

Recently, an Epstein-Barr virus (EBV)-based episomal vector was reported that was capable of transferring a HPRT gene (115 kb) to some mammalian cell lines, in which the expression was not silenced (Wade-Martins et al. 2000). However, EBV-based vectors are lost more rapidly than HAC in the absence of selection and their replication is reliant on the presence of the viral trans-activator, EBNA1. Safety in the clinical gene therapy with EBV vectors requires further investigation. HACs may overcome the above problems as gene transfer vectors and have further advantages in term of safety. HACs carried a long genomic locus in this study were maintained extrachromosomally, and expressed regulated level of genes for long periods. Therefore, the HAC containing TH, AADC and GCH1 may offer a potential therapeutic strategy for Parkinson's disease.

However, the low efficiency of *de novo* generation of HACs from BACs, requirement of the limited cell line for generation of HACs and the large size of HACs presents a difficulty in the delivery of HACs to cells or tissues at required sites. To utilize the HAC as a gene transfer vector, the HAC that has been established in HT1080 needs to be transferred into suitable cell lines. The HAC could be transferred by MMCT using the mouse A9 cells, which enable the formation of micro-cells (Fournier et al. 1977). The present inventors have established mouse A9 cell lines, which maintained HACs stably in mitotic growth under non-selective conditions without detectable structural changes in the HAC. The HACs would be easily transferrable from A9 to other cell lines.

We have demonstrated in this study the generation of a HAC containing the GCH1 gene, together with its original regulatory region, from a GCH1-BAC by co-transfection with the alphoid-BAC. The GCH1-HACs expressed GCH1 genes in regulated manner and thus proved to be a good system to study regulatory mechanism of GCH1 gene *in vivo.* Further study on the GCH1-HACs will reveal the minimum number of alphoid arrays used to assemble centromere/kinetochore, the structure of the upstream region required for regulated expression of the GCH1 gene and sites of action of transcription factors on the regulatory region. Results we have obtained also indicated that the GCH1-HAC may also serve as a gene delivery tool in animal models or therapeutic trials in the future.

<EXAMPLE 7> Transfer of HACs into ES cells by micro-cell mediated chromosome transfer

HT1080 cells containing HACs retaining GCH1 gene were transferred to mouse A9 cells by a cell fusion method. First of all, by the same procedure as in Example 5, HAC-containing cell lines (HT/GCH2-10) were fused to mouse A9 cells, and BS- and Ouabain-resistant cell lines were selected. To the selected sell lines F(A9/2-10)4, colcemid was added so that the final concentration became 0.05 μg/ml, followed by culturing at 37°C under conditions of 5% $CO_2$ for 72 hours. Cells were collected by trypsinization and suspended in a D-MEM medium without serum. Cytochalasin B was added so that the concentration became 20 μg/ml and left at 37°C for 5 minutes, followed by adding an equal amount of Percol which had been kept at 37°C in advance. Then, micro-nuclei were collected by centrifugation (15,000 rpm for 90 mins). The collected micro-nuclei were suspended in a D-MEM medium without serum, followed by centrifugation (2,000 rpm for 5 mins) again. The obtained precipitates (micro-nuclei) were suspended in a D-MEM medium without serum again. After repeating this operation twice, to the precipitates including micro-nuclei, ES cells TT2 (C57BL/6 x CBA), which were collected by trypsinization, were added, followed by centrifugation (1,500 rpm for 5 minutes). Thus, cells and micro-nuclei were precipitated. After removing supernatant, 1 ml D-MEM medium without serum was added so as to suspend the precipitates, which was kept in this state for 10 minutes (at room temperature). Then, cells and micro-nuclei were precipitated by centrifugation (1,500 rpm for 5 minutes) and the supernatant was removed, followed by adding 1 ml of PEG1500 (Roche) so as to suspend the precipitates. After leaving it for 90 seconds at room temperature, 5 ml D-MEM medium without serum was added and cells were collected by centrifugation (1,000 rpm and 5 min).

To the collected cells, 10 ml of D-MEM medium without serum was added, followed by washing by centrifugation at 1,000 rpm for 5 minutes twice. Precipitates after washing were suspended in an ESM medium (D-MEM + non-essential amino acid (Invitrogen) + 0.1 mM β-mercaptoethanol + $10^3$ U/ml ESGRO (Chemicon) + nucleoside). The obtained cell suspension was plated on feeder cell SLB (provided by Dr. Yuzo Kadokawa at Fujita Health University School of Medicine) which were treated with mitomycin C so as to stop the proliferation. After 24 hours the culturing was started, a medium was replaced with an ESM medium containing blastcidin S so that the final concentration was 4 μg/ml and the culturing was continued. After five days the selection operation was started, the medium was replaced with an ESM medium (4 μg/ml blastcidin S, 1 x HAT (Sigma)), and then the culturing was continued further for five days.

The resultant colony was isolated and plated on feeder cells SLB (24 well culture dish) which were treated with mitomycin C to stop the proliferation. From proliferated cells, cell lines containing BAC DNA were selected by PCR, and subjected to FISH analysis using an alphoid DNA, a BAC vector, a GCH1 gene and a mouse minor satellite DNA as probes (see Examples 2 and 5).

Fig. 8A shows the result of the FISH analysis using an alphoid DNA and a BAC vector as probes. Green indicates a signal of the alphoid DNA (arrow) and red indicates a signal of the BAC vector (arrow head). It is shown that the isolated ES cells contain one copy of HAC and maintain a normal nucleus type.

Fig. 8B shows the result of FISH analysis using an exon 1 region of a human GCH1 gene and a BAC vector as probes. A green signal (arrow) of GCH1 gene and a red signal (arrow head) of the BAC vector were simultaneously detected on the HAC.

Fig. 8C shows the result of FISH analysis using a mouse minor satellite DNA and a BAC vector as probes. On the HAC, a signal (a part of which is shown by an arrow) of the mouse minor satellite DNA were not detected. Note here that an arrow head show a signal of the BAC vector.

<EXAMPLE 8> Stability of HAC in ES cells

The stability of HAC in ES cells was analyzed by culturing in the absence of selective agents for a long time. The HAC-containing ES cells obtained in Example 7 were cultured (20 days) both in the presence and absence of blastcidin S, followed by calculating the rate of HAC-containing cells by FISH analysis. Fig. 9 shows the result of the analysis. Even after a long term culturing in the absence of agent, 80% or more of cells retain HAC in a state of one copy. When the loss rate of chromosomes per cell division was calculated, it was 0.2 %, which showed substantially the same level of stability as in the case of HAC in HT/GCH2-10 cells. Note here that the loss rate R of chromosomes was calculated from the following equation:

$$N_n = N_0 \times (1 - R)^n$$

<Example 9> Construction of human artificial chromosome (HAC) using yeast artificial chromosome (YAC)

Human artificial chromosome containing an entire region of β-globin gene group (cluster) of the human chromosome 11 by using YAC as a precursor was constructed by the following procedure. The precursors used follow.

(9-1) Precursor YAC

A201F4.3: 150 kb of YAC containing human β globin gene locus in which the right arm portion of A201F4 was modified and PGKneo was inserted (provided from Keiji Tanimono, Douglas Engel, Nucleic Acid Research, 27; 3130-3137).

7c5hTEL: an artificial chromosome precursor YAC including about 80 kb of alpha-satellite array (α21-I) derived from the human chromosome 21 alphoid region and a marker gene SVbsr, and having yeast telomere sequences at both ends and human telomere sequence inside thereof. Yeast containing 7c5hTEL (Saccaromyces serevisiae EPY 305-5b α7C5hTEL) was disposed with Agency of Industrial Science and Tehnology, National Institute of Bioscience and Human Technology in Ministry of International Trade and Industry (at present, National Institute of Advanced Industrial Science and Technology, International Patent Organism Depositary, of which address is Chuo No. 6, 1-3, Higashi 1-chome, Tsukuba-shi, Ibaraki-ken, 305-8566, Japan) on August 14, 1996 (deposition No: FERM BP-5625), and 7c5Htel is prepared from this yeast cell line. As to the production method of the yeast cell line, see, for example, Published Japanese translation of a PCT application No. 2000-517182.

F61: a tetracycline-induced expression system cell established by introducing pTet-OFF (CLONTECH) into HT1080 and by the selection of G418.

(9-2) Purification of yeast artificial chromosome

Pulsed Field Gel Electrophoresis (PFGE) was carried out by the following procedure so as to isolate two kinds of yeast artificial chromosomes (A201F4.3 and 7c5hTEL), respectively. PFGE was carried out on 0.7 % agarose gel under the conditions of 0.5 x TBE, 180 Volt and 15 second pulse for 15 hours by using Gene Navigator (Amersham Pharmacia Biotech). YAC DNA isolated from the PFGE gel was transferred to agarose gel with 1 % low melting point by electrophoresis, and then this gel was immersed in a buffer solution of 10 mM Tris (pH 8.0), 1 mM EDTA and 100 mM NaCl for 16 hours. 100 μg E. coli tRNA was added to YAC DNA (0.3 μg/0.3 ml), which was heated at 70°C for 10 minutes so as to melt the gel. 30U β agarase (Sigma) was added and reacted at 42°C for 2 hours to digest the agarose. These were subjected to PFGE so as to confirm bands of 7c5hTEL (90 kb) and A201F4.3 (150 kb) (see Fig. 10A).

(9-3) Introduction of YAC

0.3 μg each of the purified 7c5hTEL and A201F4.3 were mixed and 60 μl of Superfect (Qiagen) was added, followed by gently mixing thereof so as to cause a reaction at room temperature for 10 minutes. The reacted solution was added to F61 cells. 90 minutes later, the culture solution (10% FBS (Trace Scientific Ltd., Noble Park, Australia) in D-MEM: Dulbecco's Modified Eagle Medium (Invitrogen Corp., Carlsbad, CA, USA)) was replaced with a new one. 72 hours later, resistance cell lines were selectively cultured on 8 μg/ml Blasticidin S added medium so as to isolate the transformed cell lines. As a result, 19 transformed cell lines were obtained.

<Example 10> Cytogenetic analysis of transformed cell line

The obtained transformed cell lines were subjected to FISH analysis by using an α21-I probe (alphoid probe obtained by labeling a DNA fragment of SEQ ID NO: 3 with digoxigenin) and a probe of the arm portion of YAC (obtained by labeling about 8 kb of DNA fragment (SEQ ID NO: 4) obtained by XhoI-cutting a pYAC5 vector (Dr. Maynard V. Olson (Washington University)) with biotin). As a result, it was observed that mini chromosome was formed in one transformed cell line and signals was included in both α21-I and the arm portion of YAC in this mini chromosome (see Fig. 10B). In the rest of clones, signals were observed on the host chromosome or no signals were detected.

On the other hand, when the transformed cell lines including mini chromosome were subjected to FISH analysis by using three kinds of probes (SEQ ID NO: 5, SEQ ID NO: 6 and SEQ ID NO: 7) each recognizing different sites of a human P globin cluster non-coding region, signal from each probe was observed on the mini chromosome (see FIG. 11). Note here that each probe was obtained by subjecting it to PCR (25 cycles each cycle including 96°C for 30 seconds, 58°C for 40 seconds and 72°C for 10 minutes) by using A201F4.3 as a template and by using the following primers and labeling the resultant amplified DNA with biotin.
Primer for probe shown in SEQ ID NO: 5
Sense: aagaccagatagtacagggcctggctac (SEQ ID NO: 10)

Antisense: aagattattcaaggttactatgaacacc (SEQ ID NO: 11)
Primer for probe shown in SEQ ID NO: 6
Sense :tgctaatgcttcatctagaaacttatatcctttaattc (SEQ ID NO: 12)
Antisense: tttccactcgagccaaccaggaattcggcagttac (SEQ ID NO:13)
Primer for probe shown in SEQ ID NO: 7
Sense: gtgtaagaaggttctctagaggctctacagatagggag (SEQ ID NO: 14)
Antisense: aagcagcacttgactcgagtatttttatacatgctctac (SEQ ID NO: 15)

Furthermore, in the FISH analysis using a digoxigenin-labeled telomere repeat sequence (about 500 bp of sequence consisting of repeat sequences of SEQ ID NO: 8) as a probe, two points or four points of signals of telomere were observed on the mini chromosome (see FIG. 12).

From the results mentioned above, YAC including an alpha-satellite array and YAC including human β globin cluster entire region were introduced into HT1080 cells, whereby it was confirmed that mini chromosome (human artificial chromosome) retaining an entire region of human β-globin cluster could be constructed.

<Example 11> Analysis of macro structure of mini chromosome using fiber FISH

Mouse A9 cells and cells with mini chromosomes ($1\times10^6$ each) were plated on a culture dish and 3 ml of 50% PEG (SIGMA) was added thereto and cultured for one minute. Then, they were cultured in a selection medium containing 10 μM Oubain and 5 μg/ml of Blasticidin S so as to obtain resistant transformed cells to Oubain and Blasticidin S. When FISH analysis was carried out as mentioned above, it was confirmed that there were transformed cell lines in which mini chromosomes were contained and the remaining chromosomes were derived from the mouse. These transformed cell lines were subjected to FISH analysis using an alphoid probe (SEQ ID NO: 3) and a β globin probe (mixture of SEQ ID NO: 5, SEQ ID NO: 6 and SEQ ID NO: 9). Note here that the probe of SEQ ID NO: 9 was a biotin-labeled DNA fragment which was amplified by PCR (25 cycles each cycle including 96°C for 30 seconds, 58°C for 40 seconds and 72°C for 10 minutes) using A201F4.3 as a template and the following primers.
Sense: gtatacatacatacctgaatatg (SEQ ID NO: 16)
Antisense: tgtaggctgaagacgttaaaagaaacac (SEQ ID NO: 17)

As a result of the FISH analysis, since signals of the alpha-satellite array are not observed on the chromosome other than the mini chromosome (see FIG. 13), fiber FISH analysis of alpha-satellite array of the mini chromosome was possible. When the fiber FISH analysis was carried out, it was confirmed that a plurality of signals of globin and alphoid array were arranged irregularly in the mini chromosome (FIG. 14).

<Example 12> Analysis of transcription amount of target gene from HAC

Then, the transcription amount of globin genes in HAC-containing cells retaining β-globin gene was analyzed.

By the same procedures as in Example 9, 7c5hTEL and A201F4.3 were introduced into leukocyte K562 cells (ATCC CCL-243) so as to obtain HAC-containing cells retaining β-globin gene (HAC-containing K562 cells). The expression states of HAC-containing K562 cells and globin gene in HAC-containing HT1080 cells were analyzed by using the transcription amount of Gγ globin as an index as follows. Note here that HT1080 cells and K562 cells before the introduction operation of 7c5hTEL and A201F4.3, that is, HT1080 cells and K562 cells which do not contain HACs, were used as a control for comparison.

First of all, RNA was extracted by a conventional method from each cell, and cDNA was synthesized by using reverse transcriptase of MMLV and an Oligo (dT) 15 primer. The thus obtained cDNA was, as a template, subjected to RT-PCR using the following primer set (exon 2 and exon 3 of Gγ globin).
Sense primer: gatgccataaagcacctggatg (SEQ ID NO: 18)
Antisense primer: ttgcagaataaagcctatccttga (SEQ ID NO: 19)

The results of RT-PCR were shown in the upper part of Fig. 15. Note here that the results of RT-PCR which were similarly carried out by using the following primers specific to β-actin gene are also shown.
Sense primer: tcacccacactgtgcccatctacga (SEQ ID NO: 20)
Antisense primer: cagcggaaccgctcattgccaatgg (SEQ ID NO: 21)

On the other hand, the transcription amount of each sample of Gγ globin genes was quantified by a real-time PCR. The real-time PCR was carried out by using ABI PRISM 7700 (ABI, Applied Bio systems Inc.) and Qiagen QuantiTect SYBR Green PCR kit (Cat 204143). Furthermore, as the primer used for amplification reaction, the above-mentioned primers were used. Note here that the transcription amount of β actin gene in each sample was calculated and the difference of the numbers of cells between the samples was corrected based on the calculated transcription amount.

The lower part of Fig. 15 shows the analysis results by the real-time PCR. Note here that the transcription amount of Gγ globin in each sample was expressed as a relative value when the trnscription amount of HT1080 without containing HAC was 1. By the introduction of HAC, the amount of expression of Gγ globin became 1.5 times when the target cell was HT1080. Meanshile, when the target cell was K-562, the expression amount became 5 time or more. Thus, regardless of target cell to be used, the expression of Gγ globin from the introduced HAC, that is, the expression of foreign gene contained in HAC was confirmed. In particular, it was shown that in a case where K-562 was used, foreign genes could be expressed with extremely high activity.

<Example 13> Creation of HAC-containing mouse (chimeric mouse)

Cell lines established by culturing ES cells containing HAC (HAC-containing ES cell lines TT2/GCH2-10) obtained in Example 7 were transfused into 8 cell-stage embryo or blastocyst stage embryo collected from ICR mouse (CLEA Japan Inc.) by an injection method, and ES cell-introduced embryo was transplanted into a provisional parent. Thereafter, a child mouse was born by natural childbirth. From the mouse 24 hours after its birth, organs (brain, heart, thymus, liver, spleen and kidney) were isolated and genomic DNAs were prepared with respect to each organ. The obtained DNA was subjected to PCR by using FastStart Taq DNA polymerase (Roche) so as to detect DNA derived from BAC. The sequence of primer to be used and cycle (reaction conditions) are as follows.
BAC3a primer: catcgtctctctgaaaaatcg (SEQ ID NO: 22)
CHIPBAC3b primer: aggaaacagcaaaactgtgac (SEQ ID NO: 23)
Cycle: 95°C, 4 minutes × 1; 95°C, 15 seconds; 55°C, 10 seconds; 72°C, 30 seconds × 35; and 72°C, 9 minutes × 1
The results of analysis by PCR are shown in Fig. 16 (b). As a result of the analysis of 15 mice, as shown in this figure, in 7 mice, BAC DNA were detected in all organs.
Then, to confirm the presence of GCH-HAC in a chimeric mouse individual body created by using HAC-containing ES cells, chromosome sample of cell division stage was made and subjected to FISH analysis. First of all, a chimeric mouse excluding head portion and visceral organs was washed with PBS and stripped, and then kept at 37°C for 1 hour in the presence of 0.05% trypsin/1 mM EDTA. Cells trypsinized from the strip were collected by centrifugation and washed with DMEM medium including 10% FCS twice. The cells were floated in DMEM containing 10% FCS again and cultured in the presence of 5% $CO^2$ at 37°C. To the culture, which was increased approximately to confluent, TN16 was added and synchronized to the division stage, followed by making chromosome sample of cell division stage.
As a result of FISH analysis using the alphoid array and the BAC vector sequence as probes, artificial chromosome (GCH-HAC) was confirmed in cells derived from chimeric mouse (derived from ES cells) (see Fig. 16 (c)). Note here that Fig. 16 (a) shows the obtained chimeric mouse. It could be confirmed that it was a chimeric mouse from a hair color.

<Example 14> Transfer of HAC to XO nuclear type ES cell lines and creation of chimeric mouse

By the same procedures as shown in Example 7, HAC was transferred into the mouse ES cells by MMCT. In Example 7, XY nuclear type ES cells were used in Example 7, but in this Example, XO nuclear type ES cells TT2-F (provided by Dr. Aizawa) was used. When cells obtained after MMCT treatment were subjected to FISH analysis, some cells contained HACs as expected (data are not shown). The thus obtained HAC-containing ES cells were cultured so as to establish cell lines. Thereafter, by using these cell lines, a chimeric mouse was attempted to produce by the same procedure as in Example 13. As a result, as shown in Fig. 17, a chimeric mouse (female) with mosaic hair color was obtained.

<Example 15> Construction of mammalian artificial chromosome having gene insertion site

Artificial chromosome including a gene insertion site and human β globin LCR as a candidate of an insulator sequence was constructed, and the effect of the insulator sequence in the artificial chromosome was verified.

<u>15-1. DNA Construct</u>

(1) Human β globin LCR

20836 kb (GenBank data base NG000007: 4818 to 25654) from YAC clone (A201F4.3, provided by Dr. Douglas Engel, Northwestern Univ.) covering the human β globin gene region was cloned to a multi-cloning site of pTWV229 vector (TAKARA BIO INC.) (TWV-LCR). (2) Acceptor precursor
1.7 kb of fragment of EcoR1-XhoI of pAc-lox71-bsr-pA (provided by Dr. Yamamura, Kumamoto Univ., Kimi Araki, Masatake Araki and Ken-ichi Yamamura (1997)) was inserted into the EcoRI site of pSV2-bsr so as to obtain SV-bsr-lox71. 6 kb of ApaLI fragment of the SV-bsr-lox71 was inserted into the SalI site of pBeloBAC so as to construct BAC-bsr-lox71.
In order to construct a precursor in which β globin LCR (Locus control region, including HS 1 to 5) was added, 20 kb of FspI fragment of TWV-LCR was inserted into the Eco065I site of BAC-bsr-lox71 (BAC-LCR-lox71, see Fig. 18). Note here that this precursor BAC-LCR-lox71 has a feature that CAG promoter (stable gene expression was expected in various mammalian culture cells and a mouse individual body) was disposed at 5' side of the lox71 site and CAG selection marker gene was constructed and the expression of gene occurs only when recombinant with respect to a selection marker gene without containing a promoter (promoterless) can be performed as expected at the time of recombination. (3) Alphoid precursor

A precursor (Δα50) was constructed by removing SalI-SalI (Cos, loxP sequence) of CMV-a50 (including about 50 kb of alphoid insert (see Example 1) in which alphoid arrays are arranged in tandem).

(4) Donor plasmid

A 1.2 kb of HindIII-SalI fragment (coding region of puro gene) from pGK-puro (E. coli vector including a PGK promoter, a puro gene, a poly A sequence of a PGK gene, Ampicillin-resistant gene, and replication origin (ori)) and a 3.0 kb of HindIII-XhoI (including lox66) from lox66-Nlaczeo (provided by Dr. Yamamura, Kumamoto Univ., Kimi Araki, Masatake Araki and Ken-ichi Yamamura (1997)) were ligated to each other so as to obtain plox66-puro. 1.2 kb of SpeI-KpnI fragment (lox66-puro cassette) was blunted from plox66-puro and inserted into the HindIII site of pTWV229 (TWV-lox-puro). 1.6 kb of AseI-MluI fragment of pEGFP-C1 (clontech) was blunted and inserted into the SalI site of TWV-lox-puro (Dn-EGFP).

<u>15-2. Construction of artificial chromosome having the Lox site</u>

The alphoid precursor (Δα50) and the acceptor precursor (BAC-bsr-lox7 or BAC-LCR-lox71) were co-introduced into HT1080 cells, and cell lines containing artificial chromosomes were selected from drug tolerance (bs) cells by FISH.

<u>15-3. Insertion of GFP gene to mammalian artificial chromosome</u>

To lox15-13 cell lines (containing artificial chromosome having β globin LCR· lox71, $2x10^5$), 1 μg of pCAG-Cre (Cre recombinase gene) and 1 μg of Dn-EGFP (lox66 sequence and EGFP gene) were transfected by using lipofectamine plus reagent (Invitrogen). After selection by puromycin, it was confirmed that EGFP inserted by FISH was present on the artificial chromosome.

<u>15-4. Quantification of expression amount of EGFP from artificial chromosome</u>

In the case where an accepter precursor that does not contain human β globin LCR (BAC-bsr-lox71ox) was used, since the insertion of Dn-EGFP did not succeed, comparison with stable cell lines, in which pEGFP-C1 (EGFP gene used for production of Dn-EGFP) was incorporated at random on the chromosome, was carried out. The fluorescence intensity of EGFP of individual cells by trypsinization was quantified by the use of IPLab software (NIPPON ROPER Co., Ltd.). As a result, it was shown that the fluorescence of EGFP inserted into the artificial chromosome emitted several times to ten times more than EGFP fluorescence on the chromosome (see Fig. 19).

The present invention is not limited to the description of the above embodiments. A variety of modifications, which are within the scopes of the following claims and which are achieved easily by a person skilled in the art, are included in the present invention.

Document cited in the present description will be listed below.

Ando, S., Yang, H., Nozaki, N., Okazaki, T. & Yoda, K. (2002) CENP-A, -B, and -C chromatin complex that contains the 1-type alpha-satellite array constitutes the prekinetochore in HeLa cells. Mol. Cell. Biol. 22, 2229-2241.

Bannister, A.J., Zegerman, P., Partridge, J.F., et al. (2001) Selective recognition of methylated lysine 9 on histone H3 by the HP1 chromo domain. Nature 410, 120-124.

Ebersole, T.A., Ross, A., Clark, E., et al. (2000) Mammalian artificial chromosome formation from circular alphoid input DNA does not require telomere repeats. Hum. Mo. Genet. 9, 1623-1631

Dong, J.Y, Fan, P.D. & Frizzell, R.A. (1996) Quantitative analysis of the packaging capacity of recombinant adeno-associated virus. Hum. Gene Ther. 7, 2101-2112.

Festentein, R., Tolaini, M., Corbella, P., et al. (1996) Locus control region function and heterochromatin-induced position effect variegation. Science 271, 1123-1125.

Fisher, K.J., Jooss, K., Alston, J., Yang, Y., Haeker, S.E., High, K., Pathak, R., Raper, S.E. & Wilson, J.M. (1997) Recombinant adeno-associated virus for muscle directed gene therapy. Nature Med. 3, 306-312.

Fournier, R.E.K. & Ruddle, F.H. (1977) Micro-cell-mediated transfer of murine chromosomes into mouse, Chinese hamster, and human somatic cells. Proc. Natl. Acad. Sci. USA 74, 319-323.

Grimes, B.R., Schindelhauer, D., McGill, N.I., et al. (2001) Stable gene expression from a mammalian artificial chromosome. EMBO Rep. 2, 910-914.
Henning, K.A., Novotny, E.A., Compton, S.T., et al. (1999) Human artificial chromosomes generated by modification of a yeast artificial chromosome containing both human alpha-satellite and single-copy DNA sequences. Proc. Natl. Acad. Sci. USA 96, 592-597.
Hibiya, M., Ichinose, H., Ozaki, N., et al. (2000) Normal values and age-dependent changes in GTP cyclohydrolase I activity in stimulated mononuclear blood cells measured by high-performance liquid chromatography. J. Chromatogr B. Biomed. Sci. Appl. 740, 35-42.
Howman, E.V., Fowler, K.J., Newson, A.J., et al. (2000) Early disruption of centromeric chromatin organization in centromere protein A (Cenpa) null mice. Proc. Natl. Acad. Sci. U S A 97, 1148-1153.
Hubbard, T., Barker, D., Birney, E., et al. (2002) The Ensembl genome database project. Nucl. Acids Res. 30, 38-41.
Ichinose, H., Ohye, T., Takahashi, E., et al. (1994) Hereditary progressive dystonia with marked diurnal fluctuation caused by mutations in the GTP cyclohydrolase I gene. Nature. Genet. 8, 236-242.
Ichinose, H., Ohye, T., Matsuda, Y., et al. (1995) Characterization of mouse and human GTP cyclohydrolase I genes. Mutations in patients with GTP cyclohydrolase I deficiency. J. Biol. Chem. 270, 10062-10071.
Ichinose, H., Suzuki, T., Inagaki, H., Ohye, T. & Nagatsu, T. (1999) Molecular genetics of dopa-responsive dystonia. Biol. Chem. 380, 1355-1364.
Ikeno, M., Masumoto, H. & Okazaki, T. (1994) Distribution of CENP-B boxes reflected in CREST centromere antigenic sites on long-range alpha-satellite DNA arrays of human chromosome 21. Hum. Mol. Genet. 3, 1245-1257.
Ikeno, M., Grimes, B., Okazaki, T., et al. (1998) Construction of YAC-based mammalian artificial chromosomes. Nature Biotechnol. 16, 431-439.
Karpen, G.H. (1994) Position-effect variegation and the new biology ofheterochromatin. Curr. Opin .Genet. Dev. 4, 281-291.
Karpen, G.H. & Allshire, R.C. (1997) The case for epigenetic effects on centromere identity and function. Trends Genet. 13, 489-496.
Kaufman, S. (1993) New tetrahydrobiopterin-dependent systems. Annu. Rev. Nutr. 13, 261-286.
Lachner, M., O'Carroll, D., Rea, S., Mechtler, K. & Jenuwein, T. (2001) Methylation of histone H3 lysine 9 creates a binding site for HP1 proteins. Nature 410, 116-120.
Mejia, J.E., Willmott, A., Levy, E., Earnshaw, W.C. & Larin, Z. (2001) Functional complementation of a genetic deficiency with human artificial chromosomes. Am. J. Hum. Genet. 69, 315-326.
Milot, E., Strouboulis, J., Trimborn, T., et al. (1996) Heterochromatin effects on the frequency and duration of LCR-mediated gene transcription. Cell 87, 105-114.
Mineta, T., Rabkin, S.D., Yazaki, T., Hunter, W.D., & Martuza, R.L. (1995) Attenuated multi-mutated herpes simplex virus-1 for the treatment of malignant gliomas. Nature Med. 1, 938-943.
Muramatsu, S., Fujimoto, K., Ikeguchi, K., et al. (2002) Behavioral recovery in a primate model of Parkinson's disease by triple transduction of striatal cells with adeno-associated viral vectors expressing dopamine-synthesizing enzymes. Hum. Gene Ther. 13, 345-354.
Nichol, C.A., Smith, G.K. & Duch, D.S. (1985) Biosynthesis and metabolism of tetrahydrobiopterin and molybdopterin. Annu. Rev. Biochem. 54, 729-764.
Ota, A., Yoshida, S., Nomura, T., Matsui, S., Hagino, Y., Umezawa, K., Katoh, S. & Nagatsu, T. (1996) Tetrahydrobiopterin biosynthesis enhanced by lipopolysaccharide stimulation in murine neuroblastoma cell line N1E-115. J. Neurochem. 67, 2540-2548.
Palmer, D.K., O'Day, K., Trong, H.L., Charbonneau, H. & Margolis, R.L. (1991) Purification of the centromere-specific protein CENP-A and demonstration that it is a distinctive histone. Proc. Natl. Acad. Sci. U S A 88, 3734-3738.
Pfeifer, A. & Verma, I.M. (2001) Gene therapy: promises and problems. Annu. Rev. Genomics Hum. Genet. 2, 177-211
Platero, J. S., Hartnett, T. & Eissenberg, J. C. (1995) Functional analysis of the chromo domain of HP1. EMBO J. 14, 3977-3986.
Shen, M.H., Yang, J., Loupart, M.L., Smith, A., Brown, W. (1997) Human mini-chromosomes in mouse embryonal stem cells. Hum. Mol. Genet. 6, 1375-82.
Tanaka, K., Kaufman, S. & Milstein, S. (1989) Tetrahydrobiopterin, the cofactor for aromatic amino acid hydroxylases, is synthesized by and regulates proliferation of erythroid cells. Proc. Natl. Acad. Sci. USA 86, 5864-5867.
Wade-Martins, R., White, R.E., Kimura, H., Cook, P.R. & James M.R. (2000) Stable correction of a genetic deficiency in human cells by an episome carrying a 115 kb of genomic transgene. Nature Biotechnol. 18, 1311-1314.
Werner, E.R., Werner-Felmayer ,G., Fuchs ,D., et al. (1990) Tetrahydrobiopterin biosynthetic activities in human macrophages, fibroblasts, THP-1, and T 24 cells. GTP-cyclohydrolase I is stimulated by interferon-gamma, and 6-pyruvoyl tetrahydropterin synthase and sepiapterin reductase are constitutively present. J. Biol. Chem. 265, 3189-3192.

Werner, E.R., Werner-Felmayer, G. & Wachter, H. (1993) Tetrahydrobiopterin and cytokines. Proc. Soc. Exp. Biol. Med. 203, 1-12.
Yen, T.J., Compton, D.A., Wise, D., et al. (1991) CENP-E, a novel human centromere-associated protein required for progression from metaphase to anaphase. EMBO J. 10, 1245-1254.
Kimi Araki, Masatake Araki and Ken-ichi Yamamura. Target integration of DNA using munatn lox sites in embryonic stem cells. Nucleic Acids Research, 1997, vol.25, No4, 868-872

**INDUSTRIAL APPLICABILITY**

The present invention provides a mammalian artificial chromosome containing a huge DNA region including an original regulatory region in addition to a gene of interest. Therefore, gene expression from the gene contained in the mammalian artificial chromosome can be carried out in an original regulation system.

The mammalian artificial chromosome of the present invention can be used also for transferring itself to the other cells, or also can be used for study at the individual body level by way of non-human embryonic stem cells, etc. Therefore, it is an extremely useful tool for study of tissue specific gene expression and gene expression over time, study of human-type genes using a model animal, development of drugs (inhibitors, promoters, etc.), and the like.

For example, by using the non-human embryonic stem cell containing an artificial chromosome with a gene of interest obtained by the method of the present invention, transformed animals (including chimeric animals) containing an artificial chromosome expressing a gene of interest can be produced, thus enabling the analysis of expression system of the single gene at the individual level. Furthermore, it is though that a clone animal carrying HAC of the present invention can be produced. The transformed animal containing the above-mentioned human artificial chromosome can be used as a model for gene therapy. Furthermore, it can be also used for analyzing the effect of drug on the target gene under physiological conditions.

The mammalian artificial chromosome of the present invention is useful as a vector for gene therapy. Thus, the mammalian artificial chromosome of the present invention provides a simple and general method of transporting a huge DNA region including the original regulatory region in addition to a gene of interest.

## Claims

**1.** A production method of a mammalian artificial chromosome, comprising:

a first step of introducing into a mammalian host cell a first vector being circular in form and comprising a mammalian centromere sequence and a second vector being circular in form and comprising an insertion sequence for specifically inserting a sequence of interest and comprising an insulator sequence;
a second step of selecting transformed cells; and
a third step of selecting a cell containing a mammalian artificial chromosome from the selected transformed cells, wherein the mammalian cell is not a human embryonic cell.

**2.** A production method of a mammalian artificial chromosome, comprising:

a first step of introducing into a mammalian host cell a first vector consisting of a yeast artificial chromosome having a mammalian centromere sequence and a mammalian telomere sequence and a second vector consisting of a yeast artificial chromosome having an insertion sequence for specifically inserting a sequence of interest and comprising an insulator sequence;
a second step of selecting transformed cells; and
a third step of selecting a cell containing a mammalian artificial chromosome from the selected transformed cells, wherein the mammalian cell is not a human embryonic cell.

**3.** The production method according to claim 1 or 2, wherein the first vector has a selection marker gene and the selection of the transformed cells in the second step is carried out by using the selection marker gene.

**4.** The production method according to any of claims 1 to 3, wherein the mammalian centromere sequence comprises a region in which a plurality of the following sequences are arranged at regular intervals:

5'-NTTCGNNNNANNCGGGN-3': SEQ ID NO. 1, wherein N is selected from the group consisting of A, T, C and G.

**5.** The production method according to any of claims 1 to 4, wherein the mammalian centromere sequence comprises a sequence derived from a human chromosome alpha satellite region.

**6.** The production method according to claim 5, wherein the mammalian centromere sequence comprises a 11mer repeat unit derived from a human chromosome 21.

**7.** The production method according to any of claims 1 to 6, wherein the size of the mammalian centromere sequence is about 50 kb or less.

**8.** The production method according to any of claims 1 to 7, wherein the insertion sequence is a loxP site, a FRT site, or a sequence obtained by partial modification of a loxP site or a FRT site and has a function for inserting the sequence of interest.

**9.** The production method according to any of claims 1 to 8, wherein the quantity ratio of the first vector to the second vector, which are inserted in the first step, is in the range from about 10 : 1 molecular ratio to about 1 : 10 molecular ratio.

**10.** A mammalian artificial chromosome obtainable by the production method described in any of claims 1 to 9, which comprises

(a) a mammalian replication origin, a mammalian centromere sequence, an insertion sequence for specifically inserting a sequence of interest and an insulator sequence, and
which is circular in form and is replicated in a mammalian cell, maintained extrachromosomally in a host cell, and transmitted to daughter cells during cell division; or
(b) a mammalian replication origin, a mammalian centromere sequence, a mammalian telomere sequence, an insertion sequence for specifically inserting a sequence of interest and an insulator sequence, and
which is linear in form and is replicated in a mammalian cell, maintained extrachromosomally in a host cell, and transmitted to daughter cells during cell division.

**11.** The mammalian artificial chromosome according to claim 10, wherein the insertion sequence is a loxP site, a FRT site, or a sequence obtained by partial modification of a loxP site or a FRT site and has a function for inserting the sequence of interest.

**12.** The mammalian artificial chromosome according to claim 10 or 11, wherein the mammalian centromere sequence comprises a region in which a plurality of the following sequences are arranged at regular intervals:

5'-NTTCGNNNNANNCGGGN-3': SEQ ID NO. 1, wherein N is selected from the group consisting of A, T, C and G.

**13.** The mammalian artificial chromosome according to any of claims 10 to 12, wherein the mammalian centromere sequence comprises a sequence derived from a human chromosome alpha satellite region.

**14.** The mammalian artificial chromosome according to claim 13, wherein the mammalian centromere sequence comprises an 11mer repeat unit derived from a human chromosome 21.

**15.** The mammalian artificial chromosome according to any of claims 10 to 14, comprising a plurality of the sequences of interest or the insertion sequences.

**16.** A mammalian cell containing the mammalian artificial chromosome described in any of claims 10 to 15 outside the autonomous chromosome, wherein the mammalian cell is not a human embryonic cell.

**17.** The mammalian cell of claim 16, which is a human cell.

**18.** A non-human embryonic stem cell containing the mammalian artificial chromosome described in any of claims 10 to 15 outside the autonomous chromosome.

**19.** An in vitro method of stably maintaining the sequence of interest or the insertion sequence for a long term in a mammalian cell, the method comprising:

introducing the mammalian artificial chromosome of any one of claims 10 to 15 into mammalian cells as target, wherein the mammalian cell is not a human embryonic cell.

**20.** A production method of a mammalian cell containing a mammalian artificial chromosome, the method comprising the steps of the method of any of claims 1 to 9, and
a fourth step of isolating the mammalian artificial chromosome of any one of claims 10 to 15 from the selected cells; and
a fifth step of introducing the isolated mammalian artificial chromosome into a mammalian cell as a target cell, wherein the mammalian cell is not a human embryonic cell.

**21.** A production method of a micro-cell containing a mammalian artificial chromosome, the method comprising the steps of the method of any of claims 1 to 9, and
a fourth step of fusing the selected cell with a mammalian cell having an ability of forming micro-cells;
a fifth step of selecting a hybrid cell capable of forming micro-cells and containing the mammalian artificial chromosome of any one of claims 10 to 15; and
a sixth step of forming micro-cells from the selected hybrid cell, wherein said micro-cells contain said mammalian artificial chromosome.

**22.** A production method of a mammalian cell containing a mammalian artificial chromosome, comprising:

isolating the mammalian artificial chromosome from a host cell containing the mammalian artificial chromosome of any of claims 10 to 15; and
introducing the isolated mammalian artificial chromosome into a mammalian cell as a target cell, wherein the mammalian cell is not a human embryonic cell.

**23.** A production method of a micro-cell containing a mammalian artificial chromosome, the method comprising:

fusing a host cell containing the mammalian artificial chromosome of any of claims 10 to 15 and a mammalian cell having an ability of forming micro-cells;
selecting a hybrid cell having an ability of forming micro-cells and containing the mammalian artificial chromosome; and
forming micro-cells from the selected hybrid cells, wherein said micro-cells contain said mammalian artificial chromosome.

**24.** A production method of a mammalian cell containing a mammalian artificial chromosome, the method comprising:

fusing the micro-cell obtainable by the production method of claim 21 or 23 with a mammalian cell as a target, wherein the mammalian cell is not a human embryonic cell.

**25.** The production method of a mammalian cell according to any of claims 19, 20, 22 and 24, wherein the mammalian cell as a target cell is a non-human embryonic stem cell, non-human embryonic germ cell, tissue stem cell, or a non-human fertilized egg.

**26.** The production method of a mammalian cell according to any of claims 19, 20, 22 and 24, wherein the mammalian cell as a target cell is formed by inducing a non-human embryonic stem cell, non-human embryonic germ cell, or tissue stem cell so as to be differentiated to a cell of specific tissue.

**27.** Use of a vector for producing a mammalian artificial chromosome in a method according to any of claims 1 to 9, said vector comprising:

a sequence of a loxP site or FRT site, or a sequence obtainable by partial modification of a loxP site or FRT site, the sequence having a function for inserting the sequence of interest, and
an insulator sequence.

**28.** A transformed mouse comprising the mammalian artificial chromosome of claim 10.

**Patentansprüche**

**1.** Herstellungsverfahren von einem künstlichen Säugetierchromosom, umfassend:

einen ersten Schritt der Einführung eines ersten Vektors in eine Säugetierwirtszelle, der kreisförmig in seiner Form ist und eine Säugetier-Zentromersequenz umfasst und einen zweiten Vektor, der kreisförmig in seiner Form ist und eine Insertionssequenz umfasst, um eine Sequenz von Interesse spezifisch einzufügen und umfassend eine Isolatorsequenz;
einen zweiten Schritt der Auswahl von transformierten Zellen; und
einen dritten Schritt der Auswahl einer Zelle aus den ausgewählten transformierten Zellen, die ein künstliches Säugetierchromosom umfasst, wobei die Säugetierzelle keine humane embryonale Zelle ist.

**2.** Herstellungsverfahren von einem künstlichen Säugetierchromosom, umfassend:

einen ersten Schritt der Einführung eines ersten Vektors in eine Säugetierwirtszelle, bestehend aus einem künstlichen Hefechromosom, das eine Säugetier-Zentromersequenz und eine Säugetier-Telomersequenz aufweist, und einen zweiten Vektor bestehend aus einem künstlichen Hefechromosom, das eine Insertionssequenz aufweist, um eine Sequenz von Interesse spezifisch einzufügen und umfassend eine Isolatorsequenz;
einen zweiten Schritt der Auswahl von transformierten Zellen; und
einen dritten Schritt der Auswahl einer Zelle aus den ausgewählten transformierten Zellen, die ein artifizielles Säugetierchromosom umfasst, wobei die Säugetierzelle keine humane embryonale Zelle ist.

**3.** Herstellungsverfahren nach Anspruch 1 oder 2, wobei der erste Vektor ein Selektionsmarkergen aufweist und die Selektion von den transformierten Zellen in dem zweiten Schritt unter Verwendung des Selektionsmarkergens durchgeführt wird.

**4.** Herstellungsverfahren nach einem der Ansprüche 1 bis 3, wobei die Säugetier-Zentromersequenz eine Region umfasst, in der eine Vielzahl von den folgenden Sequenzen in regelmäßigen Abständen angeordnet sind:

5'-NTTCGNNNNANNCGGGN-3 ': SEQ ID NR. 1, wobei N ausgewählt ist aus der Gruppe bestehend aus A, T, C und G.

**5.** Herstellungsverfahren nach einem der Ansprüche 1 bis 4, wobei die Säugetier-Zentromersequenz eine Sequenz umfasst, die von einem humanen Chromosom-alpha Satelliten-Region abgeleitet ist.

**6.** Herstellungsverfahren nach Anspruch 5, wobei die Säugetier-Zentromersequenz eine 11-mer Wiederholungseinheit umfasst, die von einem humanen Chromosom 21 stammt.

**7.** Herstellungsverfahren nach einem der Ansprüche 1 bis 6, wobei die Größe von der Säugetier-Zentromersequenz etwa 50 kb oder weniger beträgt.

**8.** Herstellungsverfahren nach einem der Ansprüche 1 bis 7, wobei die Insertionssequenz eine loxP-Stelle, eine FRT-Stelle oder eine Sequenz erhalten durch teilweise Modifikation einer loxP-Stelle oder einer FRT-Stelle ist und eine Funktion zur Insertion der Sequenz von Interesse aufweist.

**9.** Herstellungsverfahren nach einem der Ansprüche 1 bis 8, wobei das Mengenverhältnis von dem ersten Vektor zu dem zweiten Vektor, welche in dem ersten Schritt eingeführt werden, in dem Bereich von ungefähr 10 : 1 molekularem Verhältnis bis ungefähr 1 : 10 molekularem Verhältnis liegt.

**10.** Künstliches Säugetierchromosom, das durch das Herstellungsverfahren beschrieben in einem der Ansprüche 1 bis 9 erhältlich ist, das umfasst

(a) einen Säugetier-Replikationsursprung, eine Säugetier-Zentromersequenz, eine Insertionssequenz, um eine Sequenz von Interesse spezifisch einzufügen und eine Isolatorsequenz, und
das kreisförmig in der Form ist und in einer Säugetierzelle repliziert wird, extrachromosomal in einer Wirtszelle beibehalten und an die Tochterzellen während der Zellteilung übertragen wird; oder
(b) einen Säugetier-Replikationsursprung, eine Säugetier-Zentromersequenz, eine Säugetier-Telomersequenz, eine Insertionssequenz, um eine Sequenz von Interesse spezifisch einzufügen und eine Isolatorsequenz, und das linear in der Form ist und in einer Säugetierzelle repliziert wird, extrachromosomal in einer Wirtszelle beibehalten und an die Tochterzellen während der Zellteilung übertragen wird.

**11.** Künstliches Säugetierchromosom nach Anspruch 10, wobei die Insertionssequenz eine loxP-Stelle, eine FRT-Stelle oder eine Sequenz erhalten durch teilweise Modifikation von einer loxP-Stelle oder einer FRT-Stelle ist und eine Funktion zur Insertion der Sequenz von Interesse aufweist.

**12.** Künstliches Säugetierchromosom nach Anspruch 10 oder 11, wobei die Säugetier-Zentromersequenz eine Region umfasst, in der eine Vielzahl von den folgenden Sequenzen in regelmäßigen Abständen angeordnet sind:

5'-NTTCGNNNNANNCGGGN-3': SEQ ID NR. 1, wobei N ausgewählt ist aus der Gruppe, bestehend aus A, T, C und G.

**13.** Künstliches Säugetierchromosom nach einem der Ansprüche 10 bis 12, wobei die Säugetier-Zentromersequenz eine Sequenz umfasst, die von einem humanen Chromosom-alpha Satelliten-Region abgeleitet ist.

**14.** Künstliches Säugetierchromosom nach Anspruch 13, wobei die Säugetier-Zentromersequenz eine 11-mer Wiederholungseinheit umfasst, die von einem humanen Chromosom 21 abgeleitet ist.

**15.** Künstliches Säugetierchromosom nach einem der Ansprüche 10 bis 14, umfassend eine Vielzahl von Sequenzen von Interesse oder von den Insertionssequenzen.

**16.** Säugetierzelle, beinhaltend das künstliche Säugetierchromosom beschrieben in einem der Ansprüche 10 bis 15 außerhalb des autonomen Chromosoms, wobei die Säugetierzelle keine humane embryonale Zelle ist.

**17.** Säugetierzelle nach Anspruch 16, die eine humane Zelle ist.

**18.** Nicht-humane embryonale Stammzelle, beinhaltend das künstliche Säugetierchromosom beschrieben in einem der Ansprüche 10 bis 15 außerhalb des autonomen Chromosoms.

**19.** In-vitro Verfahren zur stabilen Aufrechterhaltung der Sequenz von Interesse oder der Insertionssequenz für eine langen Zeitraum in einer Säugetierzelle, das Verfahren umfasst:

Einführen des künstlichen Säugetierchromosoms nach einem der Ansprüche 10 bis 15 in Säugetierzellen als Ziel, wobei die Säugetierzelle keine humane embryonale Zelle ist.

**20.** Herstellungsverfahren von einer Säugetierzelle, die ein künstliches Säugetierchromosom enthält, wobei das Verfahren die Schritte des Verfahrens nach einem der Ansprüche 1 bis 9 umfasst, und
einen vierten Schritt des Isolierens des künstlichen Säugetierchromosoms nach einem der Ansprüche 10 bis 15 aus den ausgewählten Zellen; und
einen fünften Schritt des Einführens des isolierten künstlichen Säugetierchromosoms in eine Säugetierzelle als Zielzelle, wobei die Säugetierzelle keine humane embryonale Zelle ist.

**21.** Herstellungsverfahren von einer Mikrozelle, die ein künstliches Säugetierchromosom enthält, das Verfahren die Schritte des Verfahren nach einem der Ansprüche 1 bis 9 umfasst und
einen vierten Schritt zum Fusionieren der ausgewählten Zelle mit einer Säugetierzelle, die die Fähigkeit zur Bildung von Mikrozellen besitzt;
einen fünften Schritt der Auswahl einer Hybridzelle, die in der Lage ist Mikrozellen zu bilden und die ein künstliches Säugetierchromosom nach einem der Ansprüche 10 bis 15 enthält; und
einen sechsten Schritt der Bildung von Mikrozellen der ausgewählten Hybridzelle, wobei die Mikrozellen das künstliches Säugetierchromosom enthalten.

**22.** Herstellungsverfahren von einer Säugetierzelle, die ein künstliches Säugetierchromosom enthält, umfassend:

Isolieren des künstlichen Säugetierchromosoms aus einer Wirtszelle, die das künstliche Säugetierchromosom nach einem der Ansprüche 10 bis 15 enthält; und Einführen des isolierten künstlichen Säugetierchromosoms in eine Säugetierzelle, als eine Zielzelle, wobei die Säugetierzelle keine humane embryonale Zelle ist.

**23.** Herstellungsverfahren einer Mikrozelle, die ein künstliches Säugetierchromosom enthält, das Verfahren umfassend:

Fusionieren einer Wirtszelle, beinhaltend das künstliche Säugetierchromosom nach einem der Ansprüche 10 bis 15 und eine Säugetierzelle, die die Fähigkeit zur Bildung von Mikrozellen aufweist;
Auswählen einer Hybridzelle, die die Fähigkeit aufweist Mikrozellen zu bilden und die das künstliche Säugetierchromosom enthält; und
Bilden von Mikrozellen aus den ausgewählten Hybridzellen, wobei die Mikrozellen das künstliche Säugetierchromosom enthalten.

**24.** Herstellungsverfahren einer Säugetierzelle, die ein künstliches Säugetierchromosom enthält, das Verfahren umfassend:

Fusionieren der Mikrozelle, erhältlich durch das Herstellungsverfahren nach Anspruch 21 oder 23 mit einer Säugetierzelle als ein Ziel, wobei die Säugetierzelle keine humane embryonale Zelle ist.

**25.** Herstellungsverfahren einer Säugetierzelle nach einem der Ansprüche 19, 20, 22 und 24, wobei die Säugetierzelle als Zielzelle eine nicht-humane embryonale Stammzelle, eine nicht-humane embryonale Keimzelle, eine Gewebestammzelle oder eine nicht-humane befruchtete Eizelle ist.

**26.** Herstellungsverfahren einer Säugetierzelle nach einem der Ansprüche 19, 20, 22 und 24, wobei die Säugetierzelle als Zielzelle durch Induzieren einer nicht-humanen embryonalen Stammzelle, einer nicht-humanen embryonalen Keimzelle oder einer Gewebestammzelle gebildet wird, sodass in eine Zelle des spezifischen Gewebes differenziert wird.

**27.** Verwendung eines Vektors zur Herstellung eines künstlichen Säugetierchromosoms in einem Verfahren nach einem der Ansprüche 1 bis 9, der Vektor umfasst:

eine Sequenz einer loxP-Stelle oder FRT-Stelle, oder eine Sequenz erhalten durch teilweise Modifikation einer loxP-Stelle oder FRT-Stelle, wobei die Sequenz eine Funktion zum Einführen der Sequenz von Interesse aufweist und eine Isolatorsequenz.

**28.** Transformierte Maus, die das künstliche Säugetierchromosom nach Anspruch 10 umfasst.

## Revendications

1. Procédé de production d'un chromosome artificiel de mammifère, comprenant:

    une première étape d'introduction dans une cellule hôte de mammifère d'un premier vecteur de forme circulaire et comprenant une séquence de centromère de mammifère et d'un second vecteur de forme circulaire et comprenant une séquence d'insertion permettant d'insérer spécifiquement une séquence d'intérêt et comprenant une séquence d'isolateur;
    une deuxième étape de sélection des cellules transformées; et
    une troisième étape de sélection d'une cellule contenant un chromosome artificiel de mammifère parmi les cellules transformées sélectionnées, dans lequel la cellule de mammifère n'est pas une cellule embryonnaire humaine.

2. Procédé de production d'un chromosome artificiel de mammifère, comprenant:

    une première étape d'introduction dans une cellule hôte de mammifère d'un premier vecteur constitué d'un chromosome artificiel de levure comportant une séquence de centromère de mammifère et une séquence de télomère de mammifère et d'un second vecteur constitué d'un chromosome artificiel de levure comportant une séquence d'insertion permettant d'insérer spécifiquement une séquence d'intérêt et comprenant une séquence d'isolateur;
    une deuxième étape de sélection des cellules transformées; et
    une troisième étape de sélection d'une cellule contenant un chromosome artificiel de mammifère parmi les cellules transformées sélectionnées, dans lequel la cellule de mammifère n'est pas une cellule embryonnaire humaine.

3. Procédé de production selon la revendication 1 ou 2, dans lequel le premier vecteur contient un gène de marqueur de sélection et la sélection des cellules transformées dans la deuxième étape est réalisée en utilisant le gène de marqueur de sélection.

4. Procédé de production selon l'une quelconque des revendications 1 à 3, dans lequel la séquence de centromère de mammifère comprend une région dans laquelle plusieurs des séquences suivantes sont agencées à intervalles réguliers:

    5'NTTCGNNNNANNCGGGN-3': SEQ ID NO. 1, dans laquelle N est choisi dans le groupe constitué de A, T, C et G.

5. Procédé de production selon l'une quelconque des revendications 1 à 4, dans lequel la séquence de centromère de mammifère comprend une séquence dérivée d'une région alpha-satellite de chromosome humain.

6. Procédé de production selon la revendication 5, dans lequel la séquence de centromère de mammifère comprend un motif répétitif 11 mères dérivé d'un chromosome 21 humain.

7. Procédé de production selon l'une quelconque des revendications 1 à 6, dans lequel la taille de la séquence de centromère de mammifère est d'environ 50 kb ou moins.

8. Procédé de production selon l'une quelconque des revendications 1 à 7, dans lequel la séquence d'insertion est un site loxP, un site FRT, ou une séquence obtenue par la modification partielle d'un site loxP ou d'un site FRT et a comme fonction d'insérer la séquence d'intérêt.

9. Procédé de production selon l'une quelconque des revendications 1 à 8, dans lequel le rapport quantité du premier vecteur sur quantité du deuxième vecteur, qui sont insérés dans la première étape, est situé dans la plage de rapport moléculaire allant d'environ 10/1 à environ 1/10.

10. Chromosome artificiel de mammifère susceptible d'être obtenu par le procédé de production décrit dans l'une quelconque des revendications 1 à 9, qui comprend

    (a) une origine réplication de mammifère, une séquence de centromère de mammifère, une séquence d'insertion permettant d'insérer spécifiquement une séquence d'intérêt et une séquence d'isolateur, et

qui est de forme circulaire et qui est répliqué dans une cellule de mammifère, conservé de façon extrachromosomique dans une cellule hôte et transmis aux cellules filles pendant la division cellulaire; ou
(b) une origine réplication de mammifère, une séquence de centromère de mammifère, une séquence de télomère de mammifère, une séquence d'insertion permettant d'insérer spécifiquement une séquence d'intérêt et une séquence d'isolateur, et
qui est de forme linéaire et qui est répliqué dans une cellule de mammifère, conservé de façon extrachromosomique dans une cellule hôte et transmis aux cellules filles pendant la division cellulaire.

**11.** Chromosome artificiel de mammifère selon la revendication 10, dans lequel la séquence d'insertion est un site loxP, un site FRT, ou une séquence obtenue par la modification partielle d'un site loxP ou d'un site FRT et a comme fonction d'insérer la séquence d'intérêt.

**12.** Chromosome artificiel de mammifère selon la revendication 10 ou 11, dans lequel la séquence de centromère de mammifère comprend une région dans laquelle plusieurs des séquences suivantes sont agencées à intervalles réguliers: 5'NTTCGNNNNANNCGGGN-3': SEQ ID NO. 1, dans laquelle N est choisi dans le groupe constitué de A, T, C et G.

**13.** Chromosome artificiel de mammifère selon l'une quelconque des revendications 10 à 12, dans lequel la séquence de centromère de mammifère comprend une séquence dérivée d'une région alpha-satellite de chromosome humain.

**14.** Chromosome artificiel de mammifère selon la revendication 13, dans lequel la séquence de centromère de mammifère comprend un motif répétitif 11 mères dérivé d'un chromosome 21 humain.

**15.** Chromosome artificiel de mammifère selon l'une quelconque des revendications 10 à 14, comprenant une pluralité des séquences d'intérêt ou des séquences d'insertion.

**16.** Cellule de mammifère contenant le chromosome artificiel de mammifère décrit dans l'une quelconque des revendications 10 à 15 hors du chromosome autonome, dans laquelle la cellule de mammifère n'est pas une cellule embryonnaire humaine.

**17.** Cellule de mammifère selon la revendication 16, qui est une cellule humaine.

**18.** Cellule souche embryonnaire non humaine contenant le chromosome artificiel de mammifère décrit dans l'une quelconque des revendications 10 à 15 hors du chromosome autonome.

**19.** *Procédé in vitro* permettant de conserver de manière stable la séquence d'intérêt ou la séquence d'insertion pendant une longue durée dans une cellule de mammifère, le procédé comprenant:

l'introduction du chromosome artificiel de mammifère selon l'une quelconque des revendications 10 à 15 dans des cellules de mammifère jouant le rôle de cible, dans lequel la cellule de mammifère n'est pas une cellule embryonnaire humaine.

**20.** Procédé de production d'une cellule de mammifère contenant un chromosome artificiel de mammifère, le procédé comprenant les étapes du procédé selon l'une quelconque des revendications 1 à 9, et
une quatrième étape d'isolement du chromosome artificiel de mammifère selon l'une quelconque des revendications 10 à 15 à partir des cellules sélectionnées; et
une cinquième étape d'introduction du chromosome artificiel de mammifère isolé dans une cellule de mammifère jouant le rôle de cellule cible, dans lequel la cellule de mammifère n'est pas une cellule embryonnaire humaine.

**21.** Procédé de production d'une micro-cellule contenant un chromosome artificiel de mammifère, le procédé comprenant les étapes du procédé selon l'une quelconque des revendications 1 à 9, et
une quatrième étape de fusion de la cellule sélectionnée à une cellule de mammifère ayant la capacité de former des micro-cellules;
une cinquième étape de sélection d'une cellule hybride capable de former des micro-cellules et contenant le chromosome artificiel de mammifère selon l'une quelconque des revendications 10 à 15; et
une sixième étape de formation de micro-cellules à partir de la cellule hybride sélectionnée, dans lequel lesdites micro-cellules contiennent ledit chromosome artificiel de mammifère.

**22.** Procédé de production d'une cellule de mammifère contenant un chromosome artificiel de mammifère, comprenant:

l'isolement du chromosome artificiel de mammifère à partir d'une cellule hôte contenant le chromosome artificiel de mammifère selon l'une quelconque des revendications 10 à 15; et
l'introduction du chromosome artificiel de mammifère isolé dans une cellule de mammifère jouant le rôle de cellule cible, dans lequel la cellule de mammifère n'est pas une cellule embryonnaire humaine.

**23.** Procédé de production d'une micro-cellule contenant un chromosome artificiel de mammifère, le procédé comprenant:

la fusion d'une cellule hôte contenant le chromosome artificiel de mammifère selon l'une quelconque des revendications 10 à 15 et d'une cellule de mammifère ayant la capacité de former des micro-cellules;
la sélection d'une cellule hybride ayant la capacité de former des micro-cellules et contenant le chromosome artificiel de mammifère; et
la formation de micro-cellules à partir des cellules hybride sélectionnées, dans lequel lesdites micro-cellules contiennent ledit chromosome artificiel de mammifère.

**24.** Procédé de production d'une cellule de mammifère contenant un chromosome artificiel de mammifère, le procédé comprenant:

la fusion de la micro-cellule susceptible d'être obtenue par le procédé de production de la revendication 21 ou 23 à une cellule de mammifère jouant le rôle de cible, dans lequel la cellule de mammifère n'est pas une cellule embryonnaire humaine.

**25.** Procédé de production d'une cellule de mammifère selon l'une quelconque des revendications 19, 20, 22 et 24, dans lequel la cellule de mammifère jouant le rôle de cellule cible est une cellule souche embryonnaire non humaine, une cellule germinale embryonnaire non humaine, une cellule souche de tissu ou un oeuf fertilisé non humain.

**26.** Procédé de production d'une cellule de mammifère selon l'une quelconque des revendications 19, 20, 22 et 24, dans lequel la cellule de mammifère jouant le rôle de cellule cible est formée par l'induction d'une cellule souche embryonnaire non humaine, d'une cellule germinale embryonnaire non humaine ou d'une cellule souche de tissu afin qu'elle se différencie en une cellule d'un tissu spécifique.

**27.** Utilisation d'un vecteur pour produire un chromosome artificiel de mammifère dans un procédé selon l'une quelconque des revendications 1 à 9, ledit vecteur comprenant:

une séquence d'un site loxP ou d'un site FRT, ou une séquence susceptible d'être obtenue par la modification partielle d'un site loxP ou d'un site FRT, la séquence ayant comme fonction d'insérer la séquence d'intérêt, et une séquence d'isolateur.

**28.** Souris transformée, comprenant le chromosome artificiel de mammifère selon la revendication 10.

Fig.1

| Input BACs | $BS^r$ cell lines inspected | Alphoid and BAC signals on | | | HAC with GCH1 |
|---|---|---|---|---|---|
| | | HAC | Chromosome | Non | |
| CMV/α100 BAC +GCH1-BAC | 16 | 1 | 4 | 11 | 1 (HT/GCH2-10) |
| SV/α50 BAC +GCH1-BAC | 17 | 3 | 13 | 1 | 1 (HT/GCH5-18) |

Fig.2

| Cell lines | GCH1 activity (pmol/h/mg protein) | | Ratio of increase |
|---|---|---|---|
| | - IFN | + IFN | |
| HT1080 | 0.05 ± 0.02 | 0.76 ± 0.03 | 15 |
| HT/GCH2-10 | 0.16 ± 0.02 | 5.0 ± 0.2 | 31 |
| HT/GCH5-18 | 3.4 ± 0.2 | 16.5 ± 0.6 | 4.8 |

Fig.3

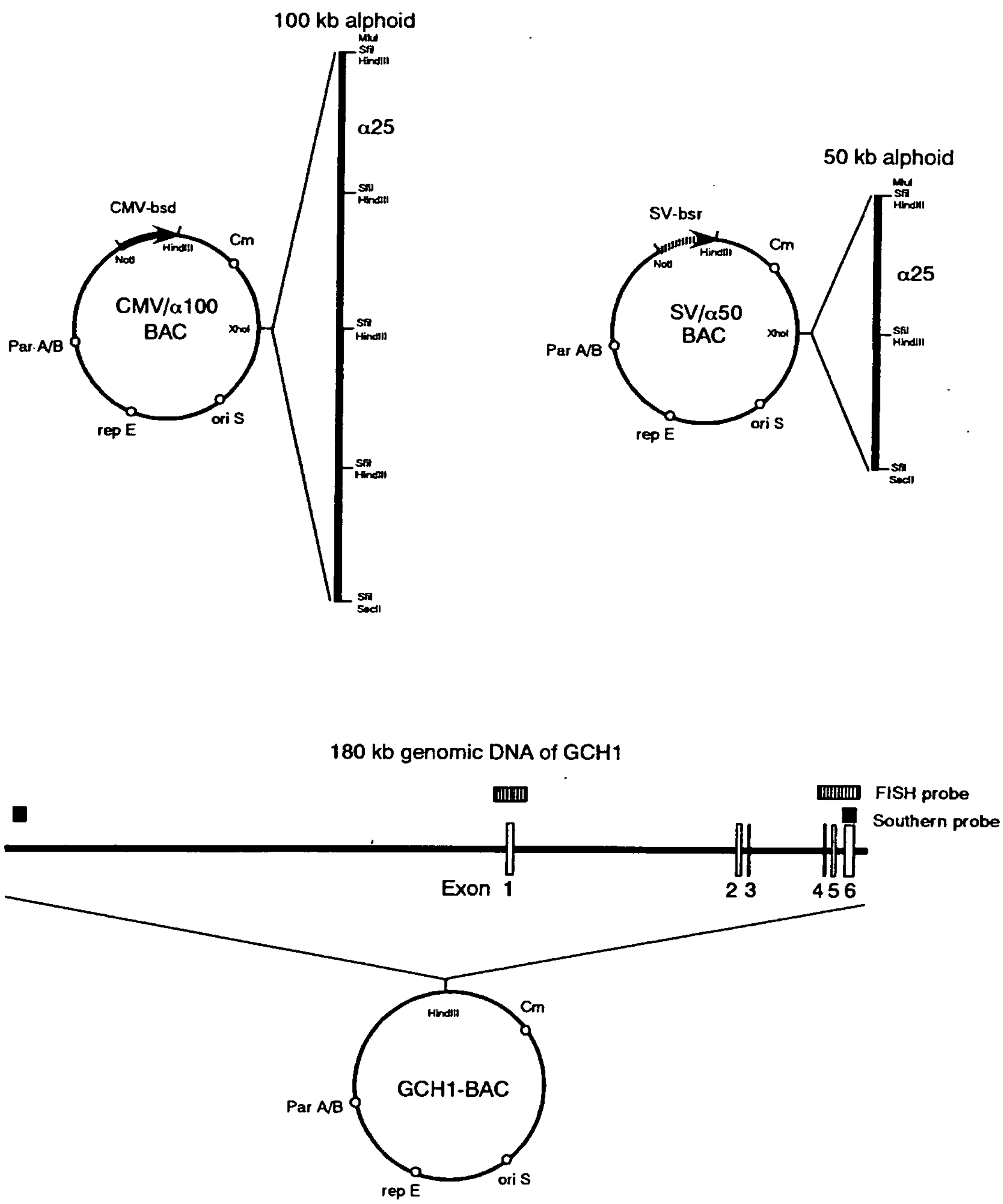
100 kb alphoid
α25
CMV-bsd
Cm
CMV/α100
BAC
Par A/B
rep E
ori S
50 kb alphoid
α25
SV-bsr
Cm
SV/α50
BAC
Par A/B
rep E
ori S
180 kb genomic DNA of GCH1
FISH probe
Southern probe
Exon 1
2 3
4 5 6
Cm
GCH1-BAC
Par A/B
rep E
ori S

Fig.4

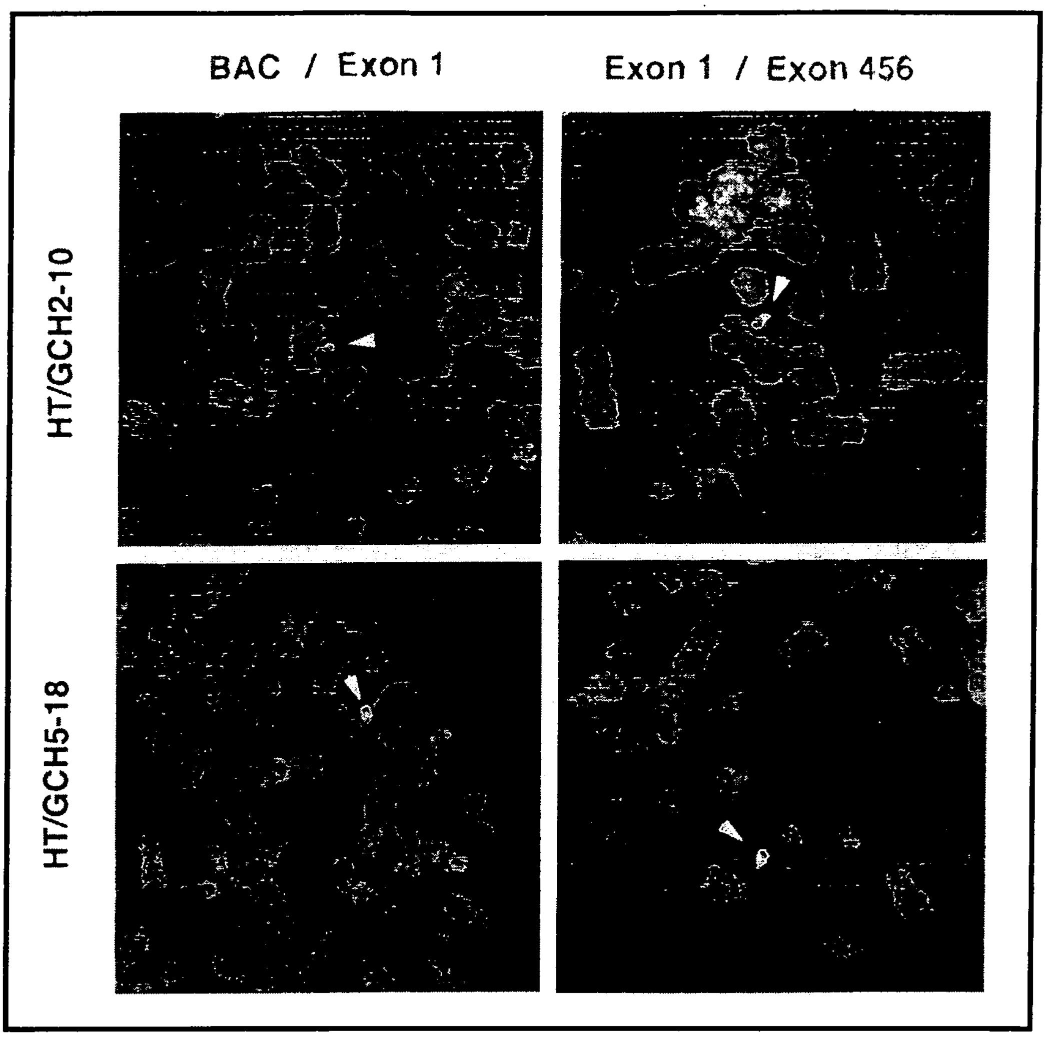

Fig.5

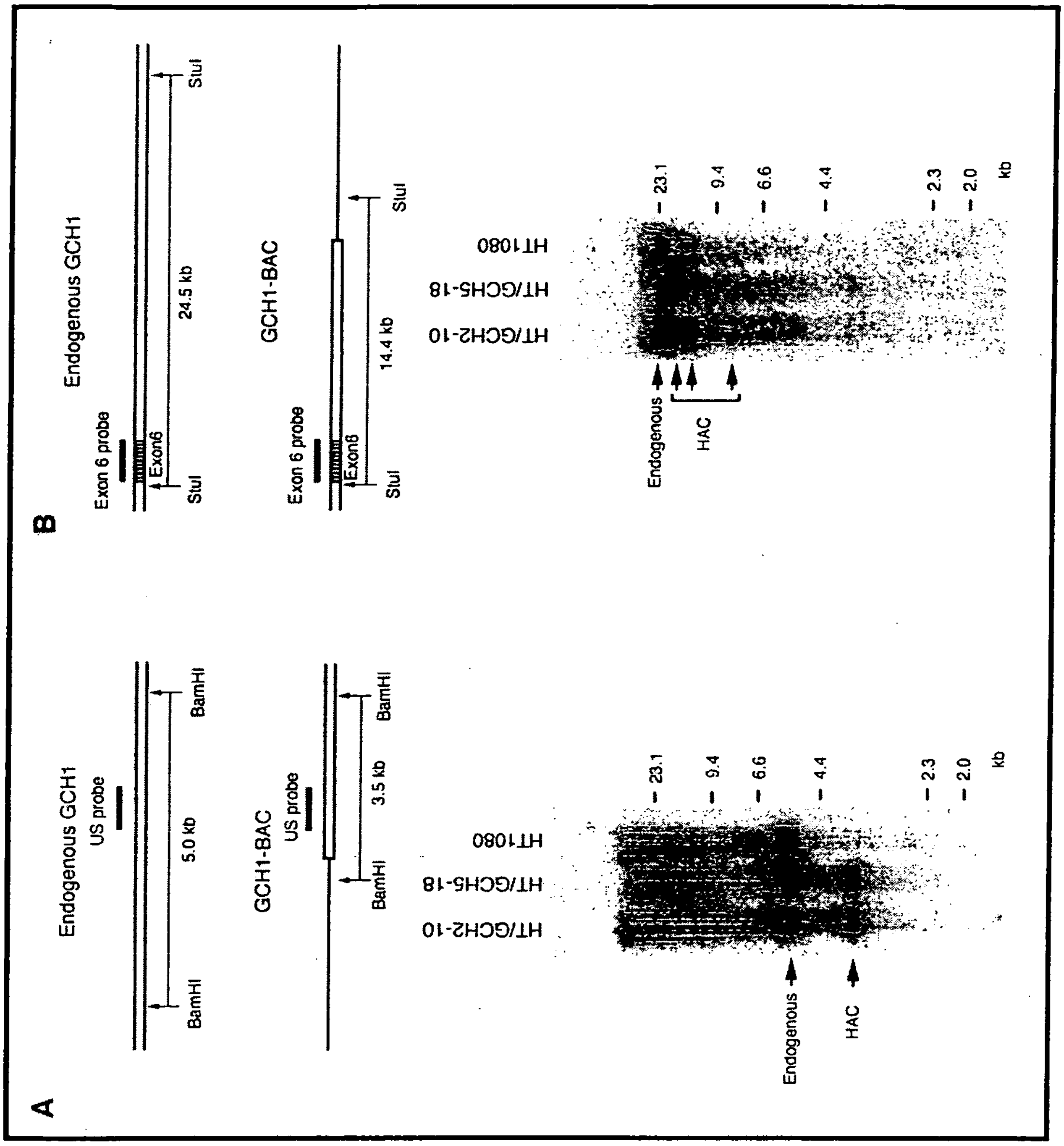
A
Endogenous GCH1
US probe
BamHI
5.0 kb
BamHI
GCH1-BAC
US probe
BamHI
3.5 kb
BamHI
HT/GCH2-10
HT/GCH5-18
HT1080
23.1
9.4
6.6
4.4
2.3
2.0
kb
Endogenous
HAC
B
Endogenous GCH1
Exon 6 probe
Exon6
StuI
24.5 kb
StuI
GCH1-BAC
Exon 6 probe
Exon6
StuI
14.4 kb
StuI
HT/GCH2-10
HT/GCH5-18
HT1080
23.1
9.4
6.6
4.4
2.3
2.0
kb
Endogenous
HAC

Fig.6

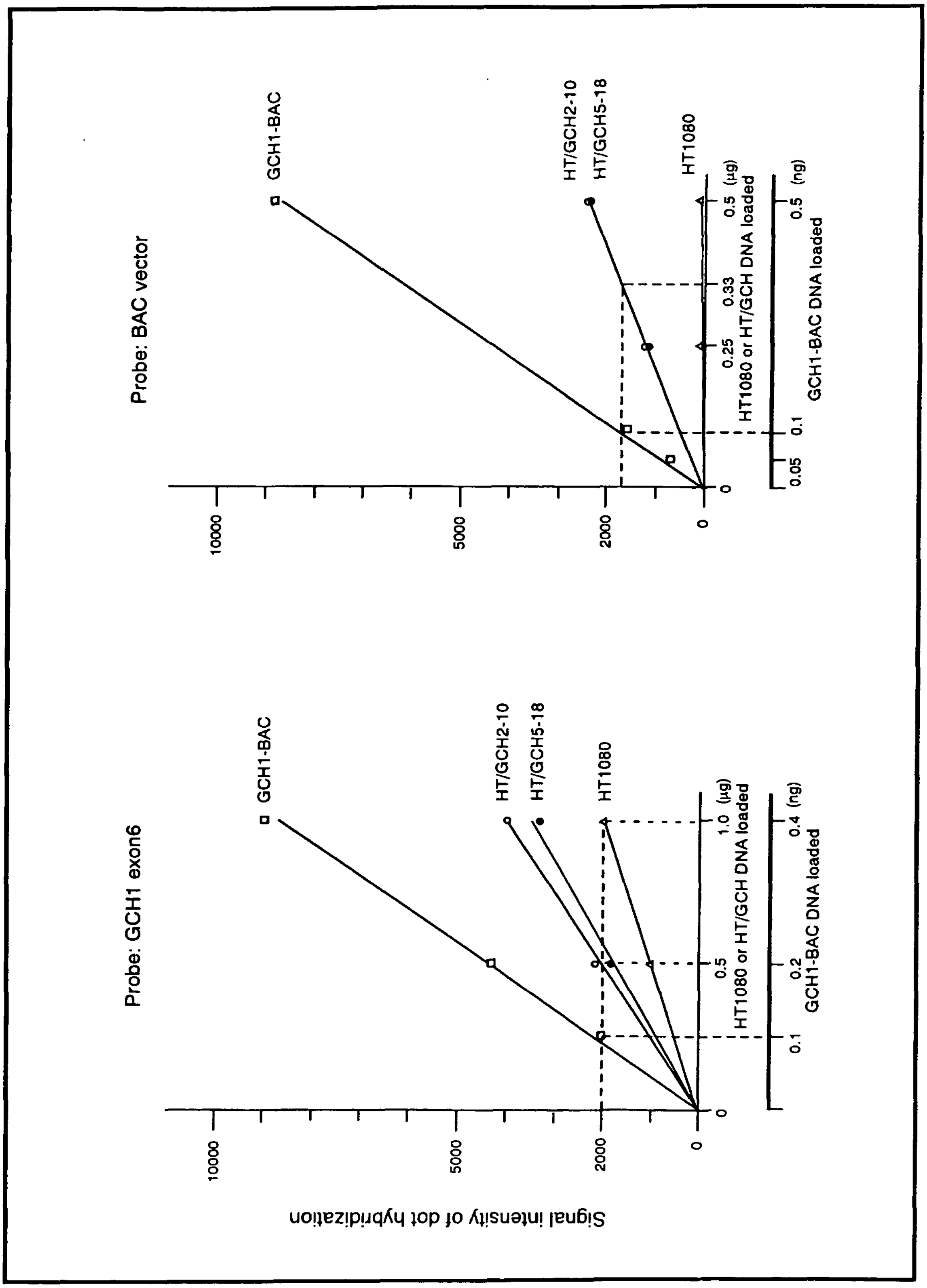

Probe: GCH1 exon6
Probe: BAC vector
Signal intensity of dot hybridization
10000
5000
2000
0
GCH1-BAC
HT/GCH2-10
HT/GCH5-18
HT1080
HT1080 or HT/GCH DNA loaded
GCH1-BAC DNA loaded
0.1
0.5
1.0 (µg)
0.2
0.4 (ng)
0.05
0.25
0.33
0.5 (µg)
0.5 (ng)

Fig.7

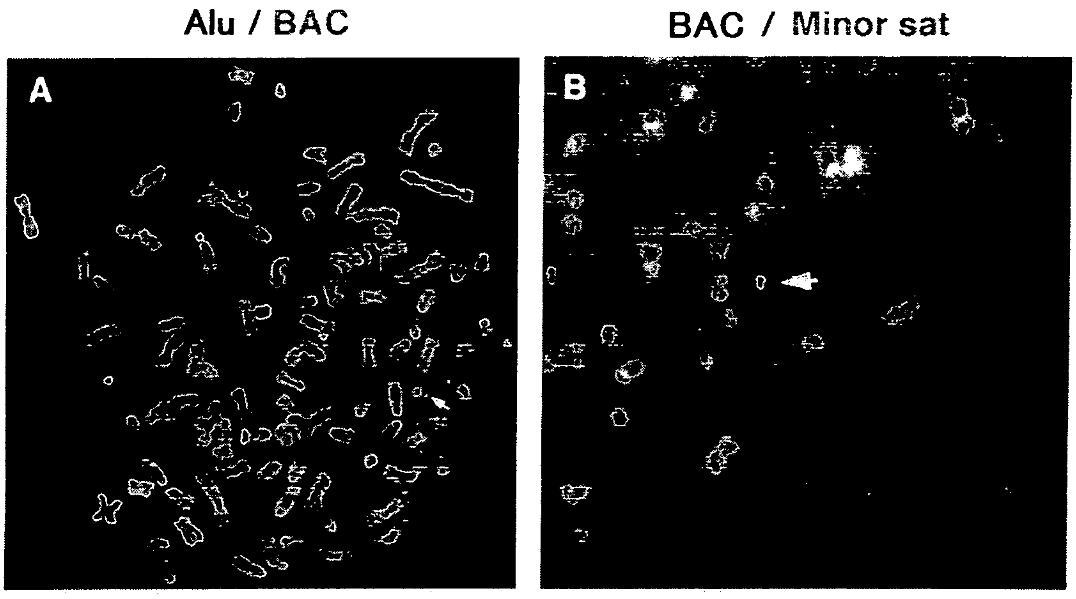

Fig.8

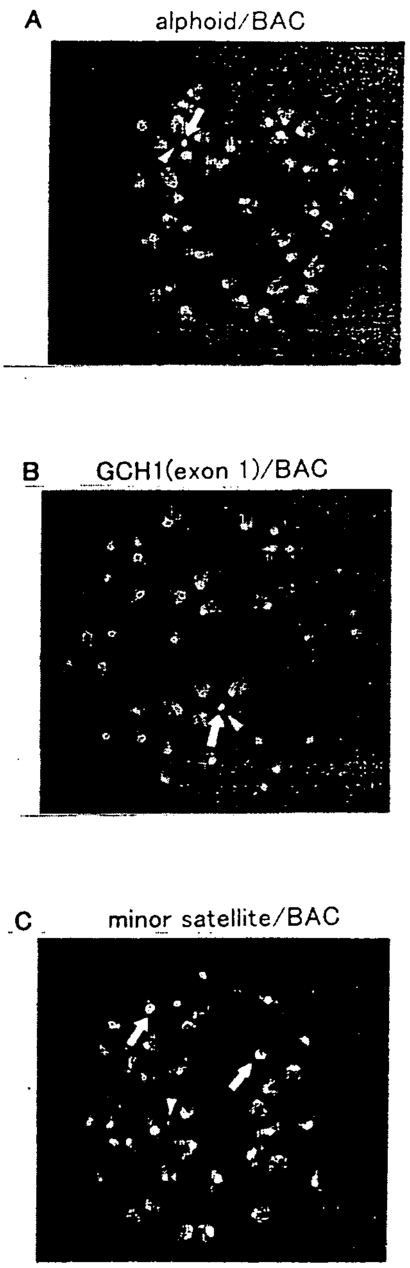

Fig.9

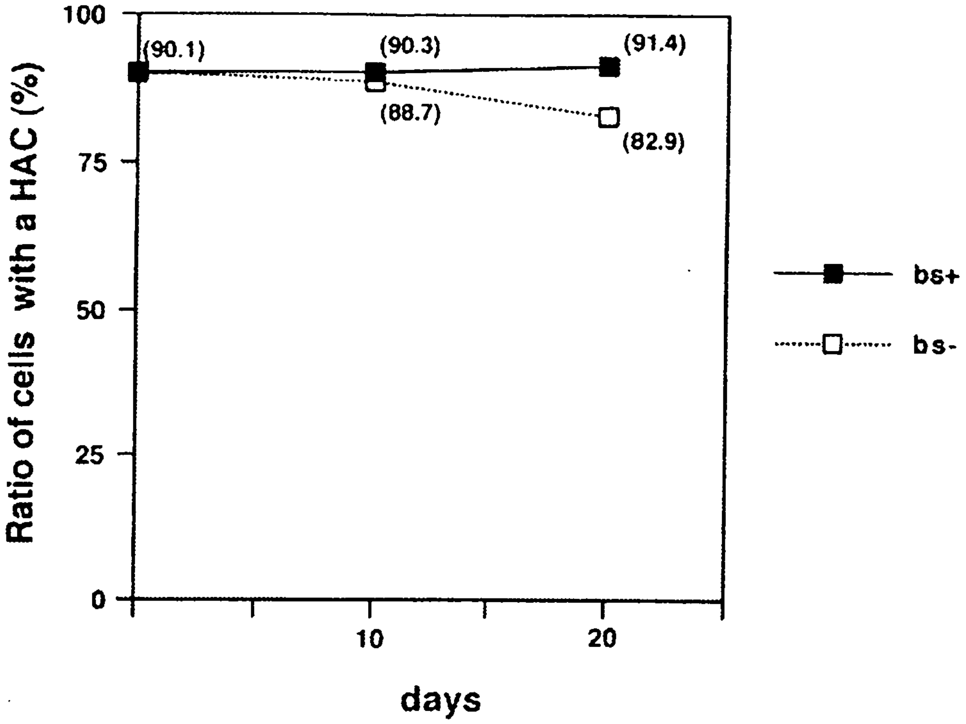
100
75
50
25
0
Ratio of cells with a HAC (%)
(90.1)
(90.3)
(91.4)
(88.7)
(82.9)
10
20
days
bs+
bs-

Fig.10

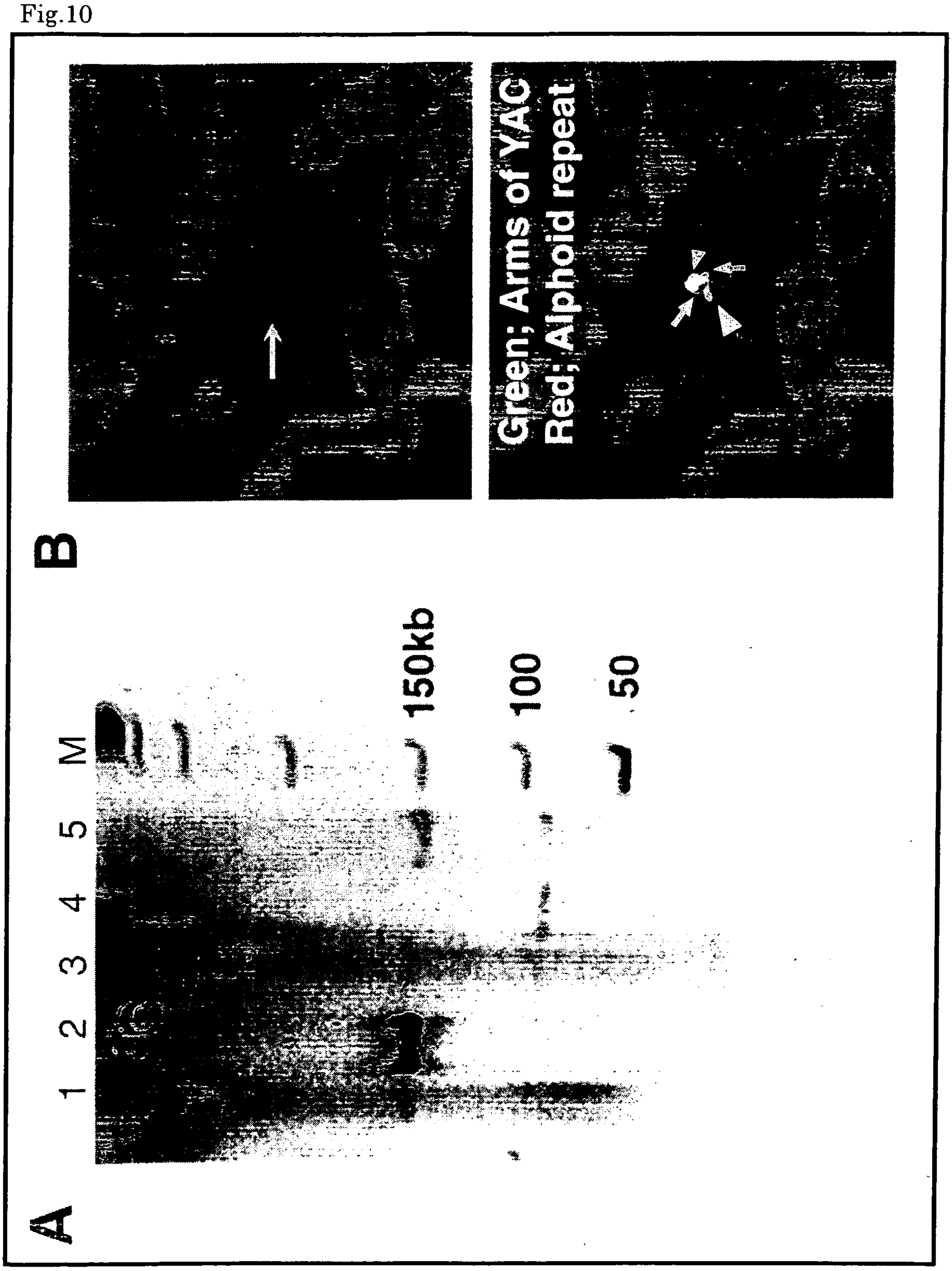

Fig.11

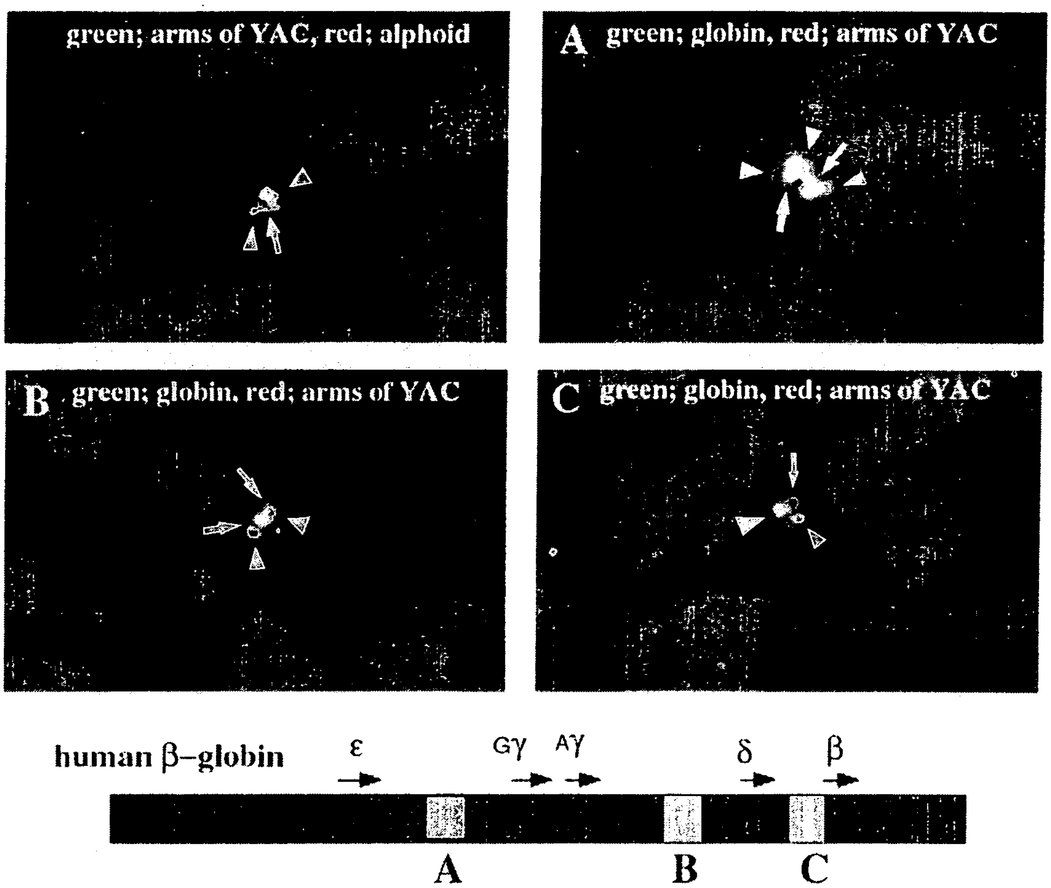
green; arms of YAC, red; alphoid
A green; globin, red; arms of YAC
B green; globin, red; arms of YAC
C green; globin, red; arms of YAC
human β–globin
ε
Gγ
Aγ
δ
β
A
B
C

Fig.12

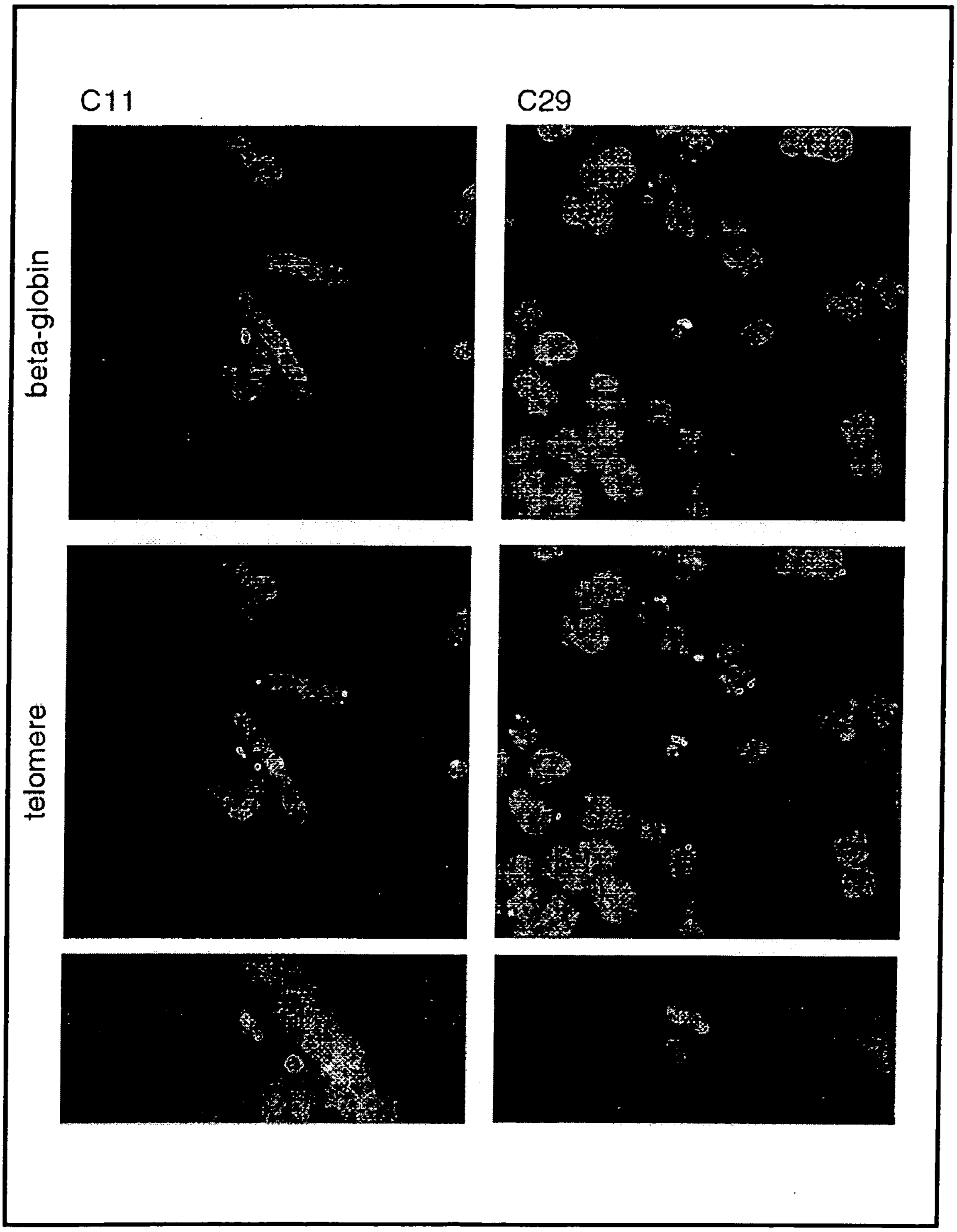

Fig.13

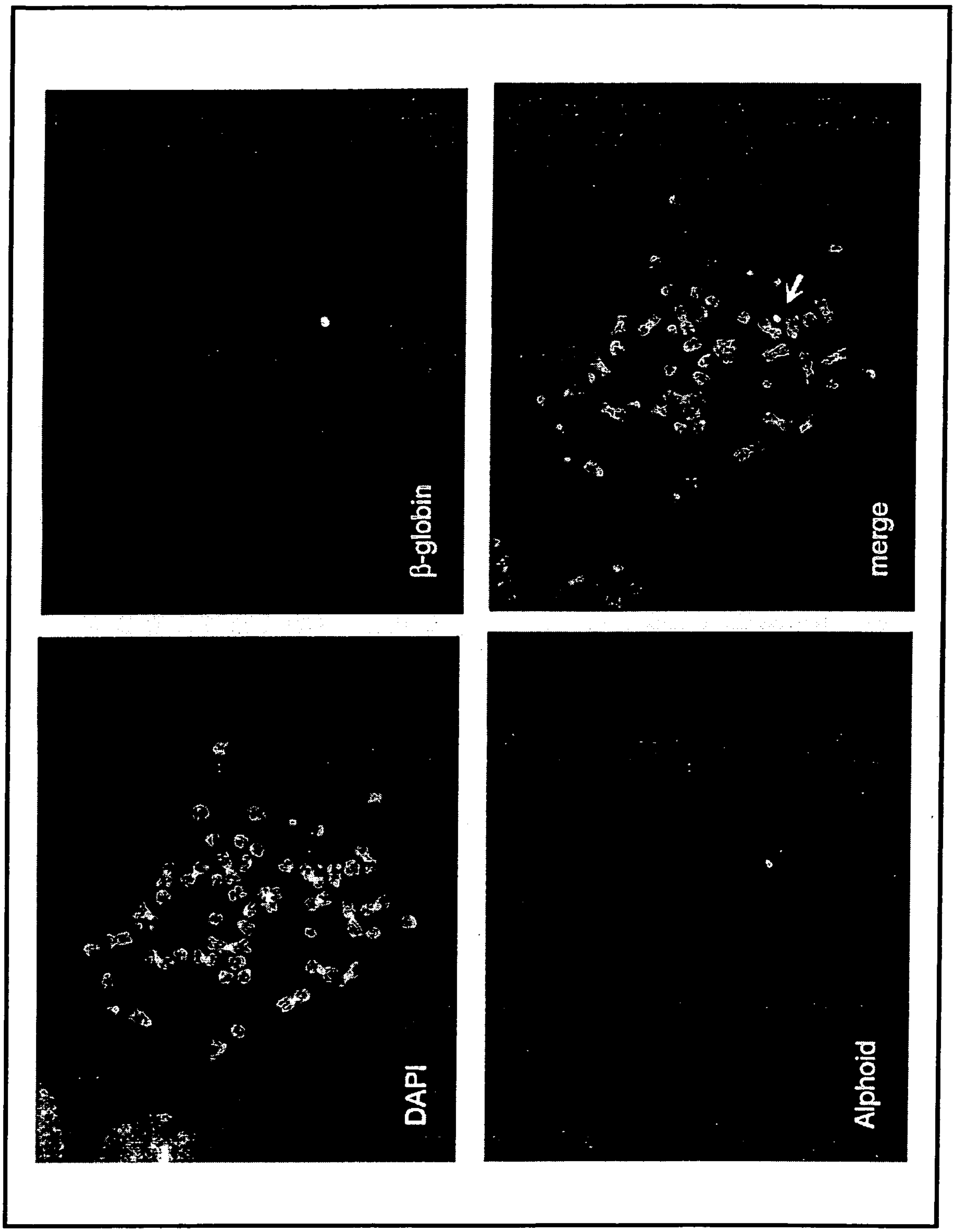

Fig.14

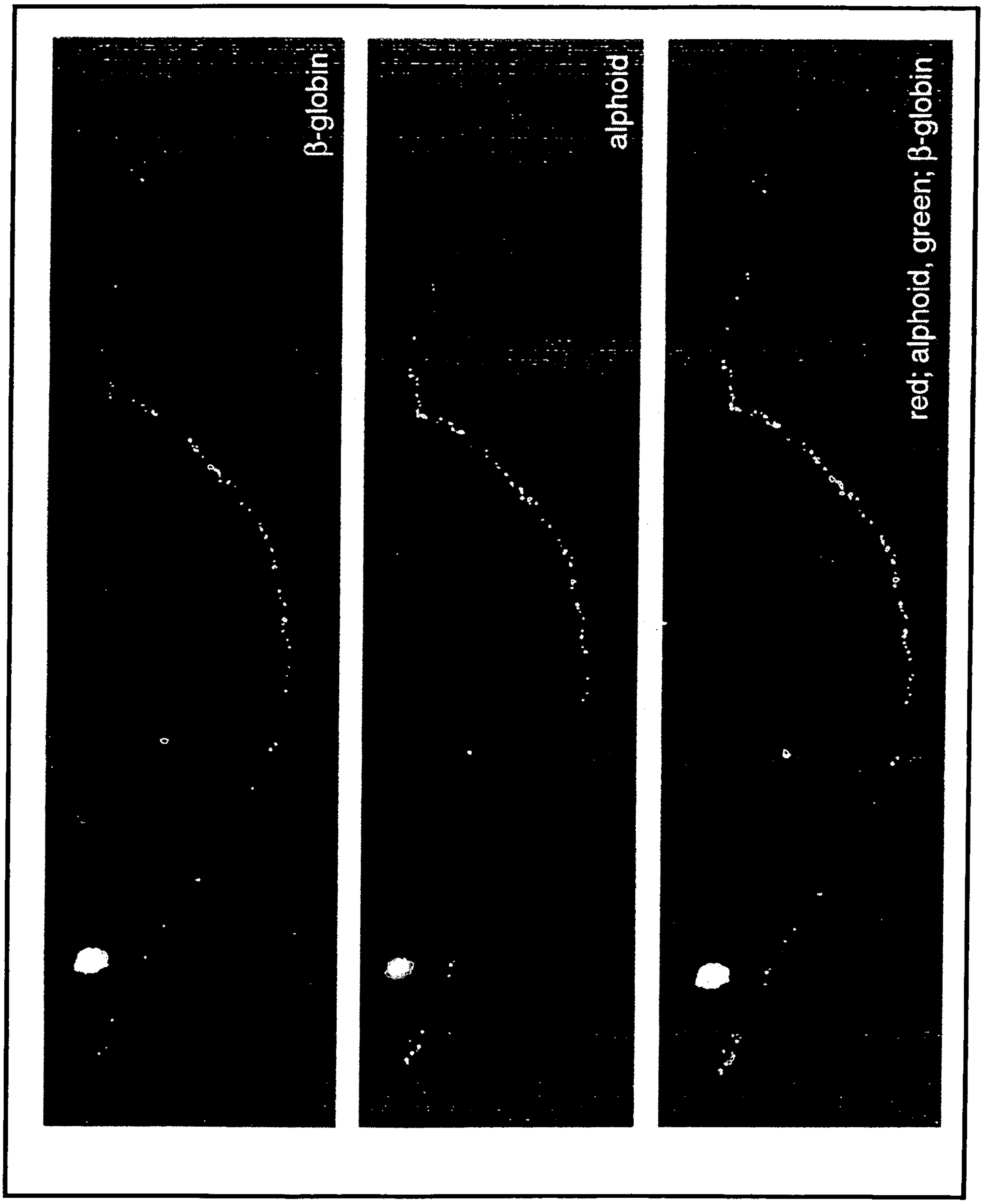

Fig.15

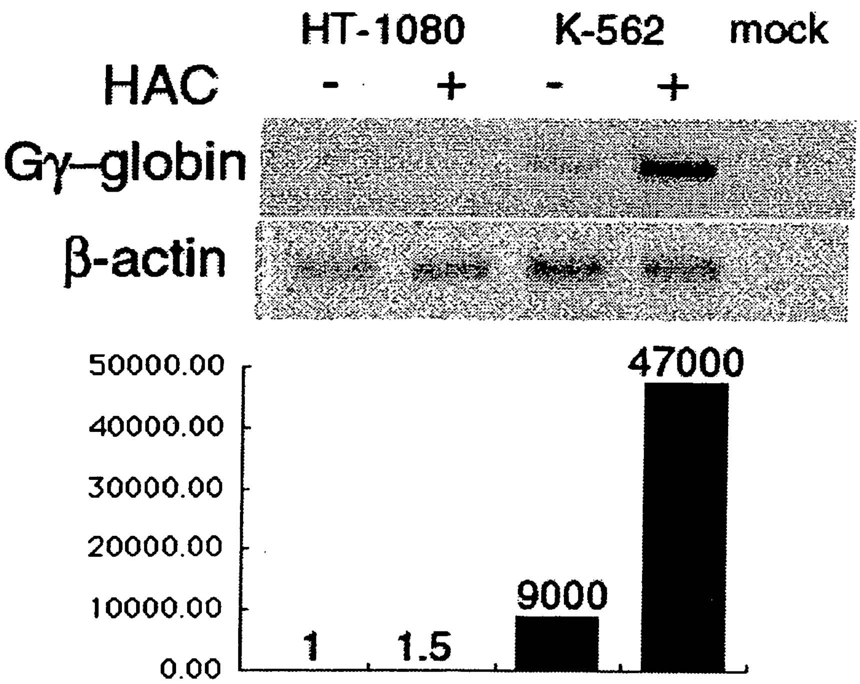
HT-1080
K-562
mock
HAC
-
+
-
+
Gγ–globin
β-actin
50000.00
40000.00
30000.00
20000.00
10000.00
0.00
1
1.5
9000
47000

Fig.16

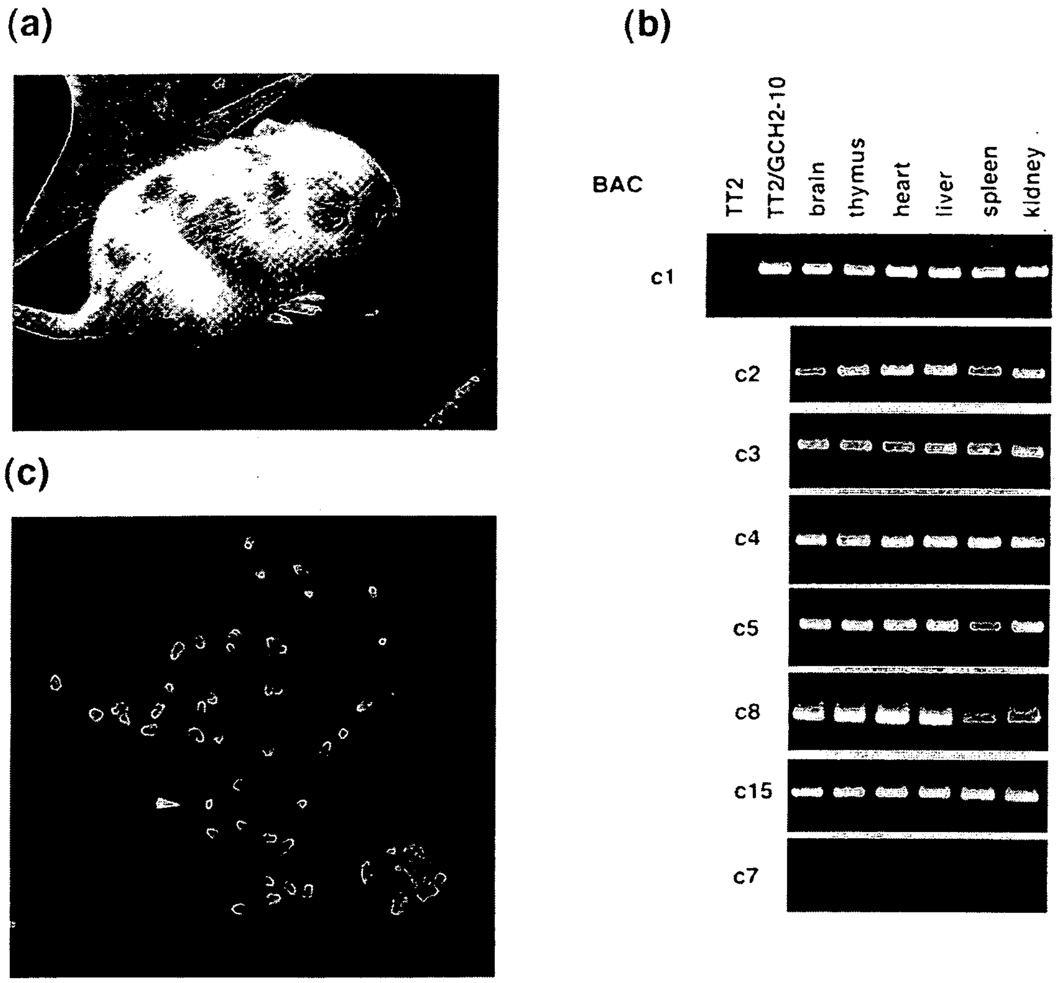
(a)
(b)
(c)
BAC
TT2
TT2/GCH2-10
brain
thymus
heart
liver
spleen
kidney
c1
c2
c3
c4
c5
c8
c15
c7

Fig.17

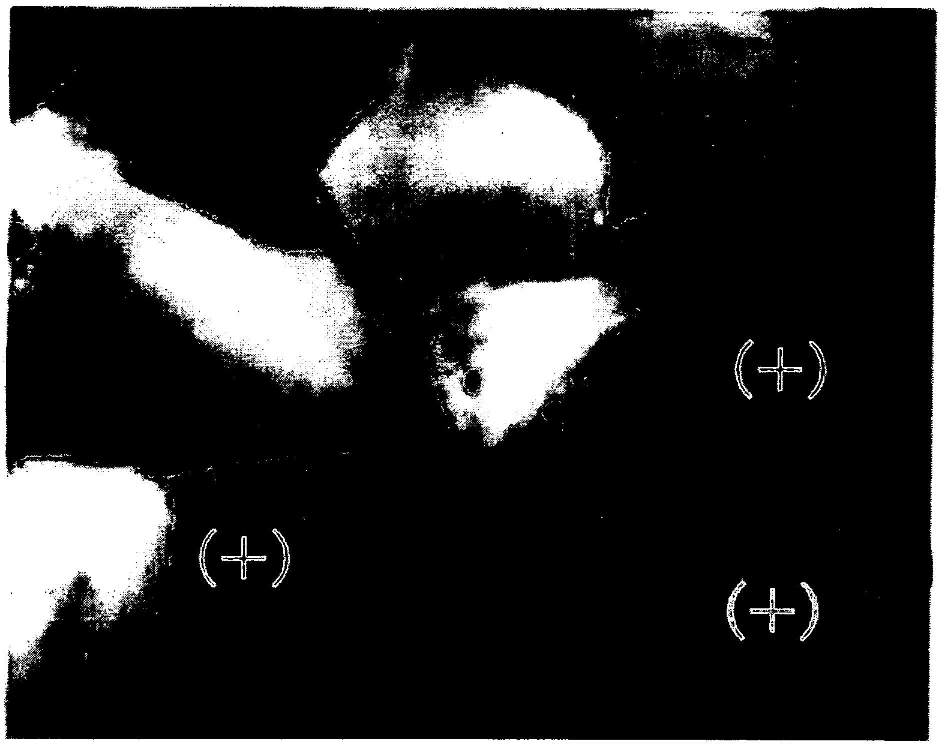

Fig.18

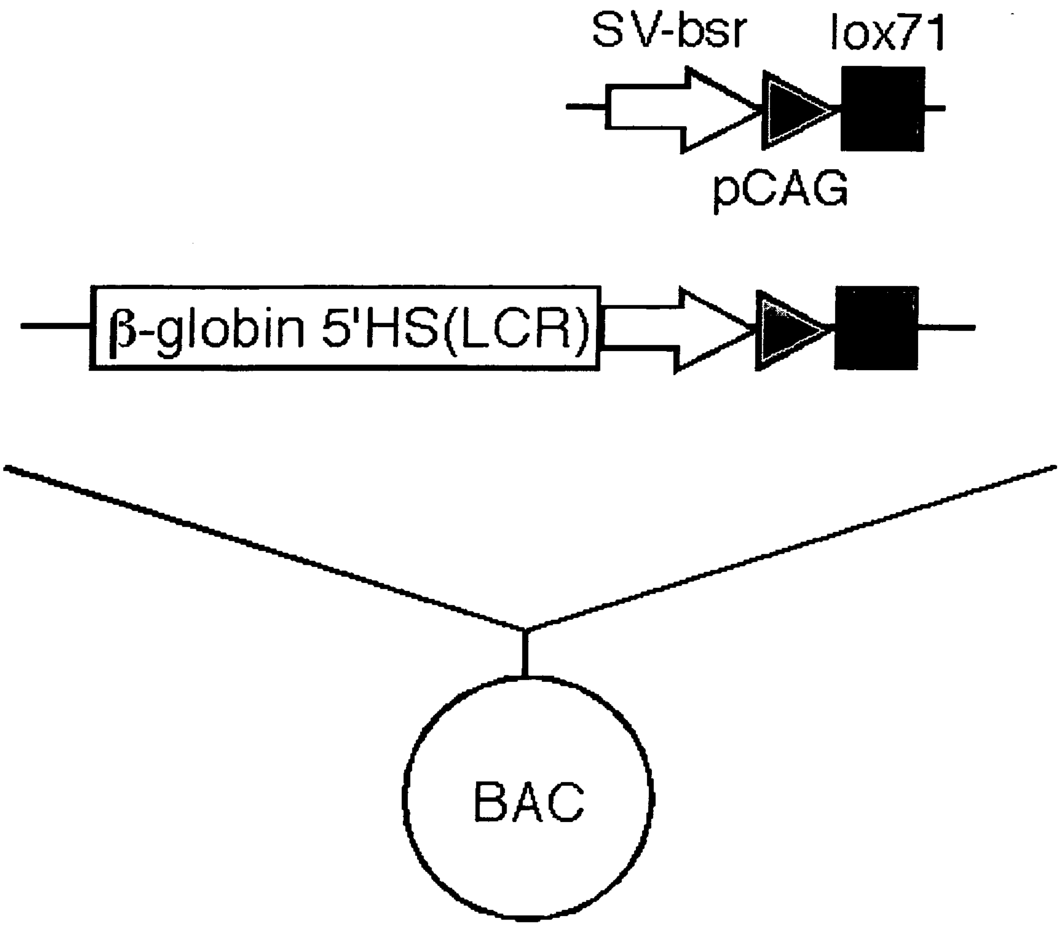

Fig.19

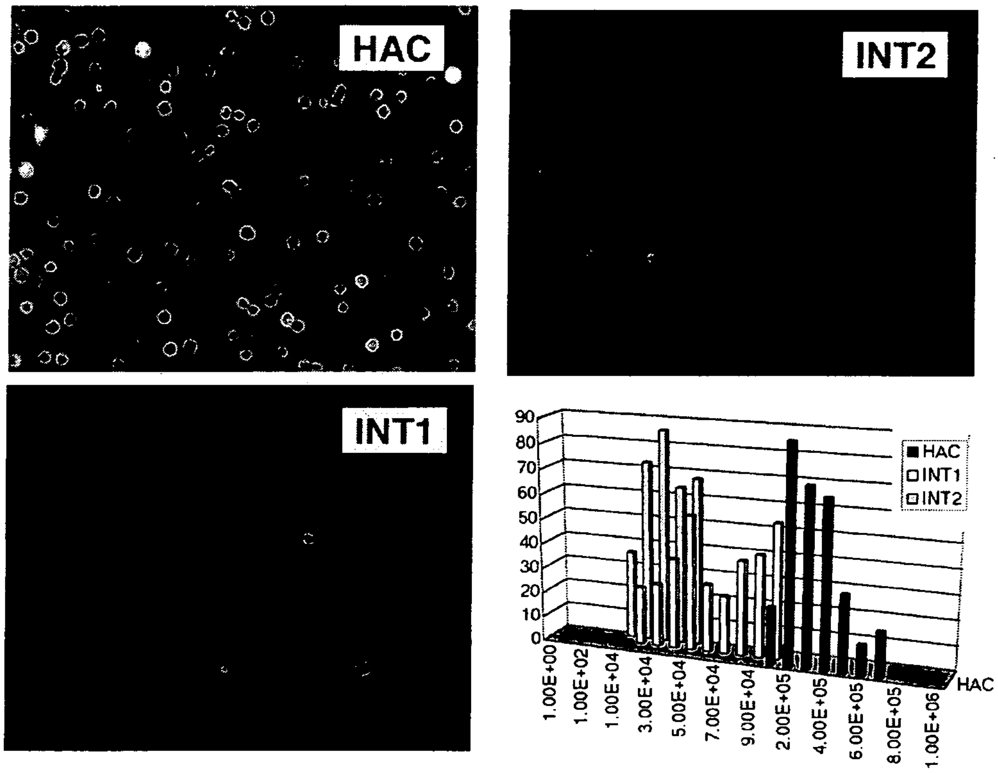
HAC
INT2
INT1
90
80
70
60
50
40
30
20
10
0
HAC
INT1
INT2
1.00E+00
1.00E+02
1.00E+04
3.00E+04
5.00E+04
7.00E+04
9.00E+04
2.00E+05
4.00E+05
6.00E+05
8.00E+05
1.00E+06
HAC

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3058566 B, Tsukuba-shi, Ibaraki-ken
- JP 2000517182 W
- JP 2002258114 P
- JP 2002338865 P


### Non-patent literature cited in the description


- Belo-BAC. New England Biolabs inc,
- **MASUMOTO et al.** NATO ASI Series. Springer-Verlag, 1993, vol. H72, 31-43
- **YODA et al.** *Mol. Cell. Biol.,* 1996, vol. 16, 5169-5177
- **IKENO et al.** *Human Mol. Genet.,* 1994, vol. 3, 1245-1247
- **FELGNER, P.L. et al.** *Proc. Natl. Acad. Sci.,* 1984, vol. 84, 7413-7417
- **GRAESSMANN,M. ; GRAESSMANN,A.** *Proc. Natl. Acad. Sci.,* 1976, vol. 73, 366-370
- **POTTER,H. et al.** *Proc. Natl. Acad. Sci.,* 1984, vol. 81, 7161-7165
- **LAWRENCE, J. B. et al.** *Cell,* 1998, vol. 52, 51-61
- **TAKAHASHI, E. et al.** *Jpn. J. Hum. Genet.,* 1989, vol. 34, 307-311
- **KEIJI TANIMONO ; DOUGLAS ENGEL.** *Nucleic Acid Research,* vol. 27, 3130-3137
- 10% FBS. Trace Scientific Ltd,
- D-MEM: Dulbecco's Modified Eagle Medium. Invitrogen Corp,
- HeLa cells. *Mol. Cell. Biol.,* vol. 22, 2229-2241
- **BANNISTER, A.J. ; ZEGERMAN, P. ; PARTRIDGE, J.F. et al.** Selective recognition of methylated lysine 9 on histone H3 by the HP1 chromo domain. *Nature,* 2001, vol. 410, 120-124
- **EBERSOLE, T.A. ; ROSS, A. ; CLARK, E. et al.** Mammalian artificial chromosome formation from circular alphoid input DNA does not require telomere repeats. *Hum. Mo. Genet.,* 2000, vol. 9, 1623-1631
- **DONG, J.Y ; FAN, P.D. ; FRIZZELL, R.A.** Quantitative analysis of the packaging capacity of recombinant adeno-associated virus. *Hum. Gene Ther.,* 1996, vol. 7, 2101-2112
- **FESTENTEIN, R. ; TOLAINI, M. ; CORBELLA, P. et al.** Locus control region function and heterochromatin-induced position effect variegation. *Science,* 1996, vol. 271, 1123-1125
- **FISHER, K.J. ; JOOSS, K. ; ALSTON, J. ; YANG, Y. ; HAEKER, S.E. ; HIGH, K. ; PATHAK, R. ; RAPER, S.E. ; WILSON, J.M.** Recombinant adeno-associated virus for muscle directed gene therapy. *Nature Med.,* 1997, vol. 3, 306-312
- **FOURNIER, R.E.K. ; RUDDLE, F.H.** Micro-cell-mediated transfer of murine chromosomes into mouse, Chinese hamster, and human somatic cells. *Proc. Natl. Acad. Sci.,* 1977, vol. 74, 319-323
- **GRIMES, B.R. ; SCHINDELHAUER, D. ; MCGILL, N.I. et al.** Stable gene expression from a mammalian artificial chromosome. *EMBO Rep.,* 2001, vol. 2, 910-914
- **HENNING, K.A. ; NOVOTNY, E.A. ; COMPTON, S.T. et al.** Human artificial chromosomes generated by modification of a yeast artificial chromosome containing both human alpha-satellite and single-copy DNA sequences. *Proc. Natl. Acad. Sci.,* 1999, vol. 96, 592-597
- **HIBIYA, M. ; ICHINOSE, H. ; OZAKI, N. et al.** Normal values and age-dependent changes in GTP cyclohydrolase I activity in stimulated mononuclear blood cells measured by high-performance liquid chromatography. *J. Chromatogr B. Biomed. Sci. Appl.,* 2000, vol. 740, 35-42
- **HOWMAN, E.V. ; FOWLER, K.J. ; NEWSON, A.J. et al.** Early disruption of centromeric chromatin organization in centromere protein A (Cenpa) null mice. *Proc. Natl. Acad. Sci.,* 2000, vol. 97, 1148-1153
- **HUBBARD, T. ; BARKER, D. ; BIRNEY, E. et al.** The Ensembl genome database project. *Nucl. Acids Res.,* 2002, vol. 30, 38-41
- **ICHINOSE, H. ; OHYE, T. ; TAKAHASHI, E. et al.** Hereditary progressive dystonia with marked diurnal fluctuation caused by mutations in the GTP cyclohydrolase I gene. *Nature. Genet.,* 1994, vol. 8, 236-242
- **ICHINOSE, H. ; OHYE, T. ; MATSUDA, Y. et al.** Characterization of mouse and human GTP cyclohydrolase I genes. Mutations in patients with GTP cyclohydrolase I deficiency. *J. Biol. Chem.,* 1995, vol. 270, 10062-10071

- **ICHINOSE, H. ; SUZUKI, T. ; INAGAKI, H. ; OHYE, T. ; NAGATSU, T.** Molecular genetics of dopa-responsive dystonia. *Biol. Chem.,* 1999, vol. 380, 1355-1364
- **IKENO, M. ; MASUMOTO, H. ; OKAZAKI, T.** Distribution of CENP-B boxes reflected in CREST centromere antigenic sites on long-range alpha-satellite DNA arrays of human chromosome 21. *Hum. Mol. Genet.,* 1994, vol. 3, 1245-1257
- **IKENO, M. ; GRIMES, B. ; OKAZAKI, T. et al.** Construction of YAC-based mammalian artificial chromosomes. *Nature Biotechnol.,* 1998, vol. 16, 431-439
- **KARPEN, G.H.** Position-effect variegation and the new biology ofheterochromatin. *Curr. Opin .Genet. Dev.,* 1994, vol. 4, 281-291
- **KARPEN, G.H. ; ALLSHIRE, R.C.** The case for epigenetic effects on centromere identity and function. *Trends Genet.,* 1997, vol. 13, 489-496
- **KAUFMAN, S.** New tetrahydrobiopterin-dependent systems. *Annu. Rev. Nutr.,* 1993, vol. 13, 261-286
- **LACHNER, M. ; O'CARROLL, D. ; REA, S. ; MECHTLER, K. ; JENUWEIN, T.** Methylation of histone H3 lysine 9 creates a binding site for HP1 proteins. *Nature,* 2001, vol. 410, 116-120
- **MEJIA, J.E. ; WILLMOTT, A. ; LEVY, E. ; EARNSHAW, W.C. ; LARIN, Z.** Functional complementation of a genetic deficiency with human artificial chromosomes. *Am. J. Hum. Genet.,* 2001, vol. 69, 315-326
- **MILOT, E. ; STROUBOULIS, J. ; TRIMBORN, T. et al.** Heterochromatin effects on the frequency and duration of LCR-mediated gene transcription. *Cell,* 1996, vol. 87, 105-114
- **MINETA, T. ; RABKIN, S.D. ; YAZAKI, T. ; HUNTER, W.D. ; MARTUZA, R.L.** Attenuated multi-mutated herpes simplex virus-1 for the treatment of malignant gliomas. *Nature Med.,* 1995, vol. 1, 938-943
- **MURAMATSU, S. ; FUJIMOTO, K. ; IKEGUCHI, K. et al.** Behavioral recovery in a primate model of Parkinson's disease by triple transduction of striatal cells with adeno-associated viral vectors expressing dopamine-synthesizing enzymes. *Hum. Gene Ther.,* 2002, vol. 13, 345-354
- **NICHOL, C.A. ; SMITH, G.K. ; DUCH, D.S.** Biosynthesis and metabolism of tetrahydrobiopterin and molybdopterin. *Annu. Rev. Biochem.,* 1985, vol. 54, 729-764
- **OTA, A. ; YOSHIDA, S. ; NOMURA, T. ; MATSUI, S. ; HAGINO, Y. ; UMEZAWA, K. ; KATOH, S. ; NAGATSU, T.** Tetrahydrobiopterin biosynthesis enhanced by lipopolysaccharide stimulation in murine neuroblastoma cell line N1E-115. *J. Neurochem.,* 1996, vol. 67, 2540-2548
- **PALMER, D.K. ; O'DAY, K. ; TRONG, H.L. ; CHARBONNEAU, H. ; MARGOLIS, R.L.** Purification of the centromere-specific protein CENP-A and demonstration that it is a distinctive histone. *Proc. Natl. Acad. Sci.,* 1991, vol. 88, 3734-3738
- **PFEIFER, A. ; VERMA, I.M.** Gene therapy: promises and problems. *Annu. Rev. Genomics Hum. Genet.,* 2001, vol. 2, 177-211
- **PLATERO, J. S. ; HARTNETT, T. ; EISSENBERG, J. C.** Functional analysis of the chromo domain of HP1. *EMBO J.,* 1995, vol. 14, 3977-3986
- **SHEN, M.H. ; YANG, J. ; LOUPART, M.L. ; SMITH, A. ; BROWN, W.** Human mini-chromosomes in mouse embryonal stem cells. *Hum. Mol. Genet.,* 1997, vol. 6, 1375-82
- **TANAKA, K. ; KAUFMAN, S. ; MILSTEIN, S.** Tetrahydrobiopterin, the cofactor for aromatic amino acid hydroxylases, is synthesized by and regulates proliferation of erythroid cells. *Proc. Natl. Acad. Sci.,* 1989, vol. 86, 5864-5867
- **WADE-MARTINS, R. ; WHITE, R.E. ; KIMURA, H. ; COOK, P.R. ; JAMES M.R.** Stable correction of a genetic deficiency in human cells by an episome carrying a 115 kb of genomic transgene. *Nature Biotechnol.,* 2000, vol. 18, 1311-1314
- **WERNER, E.R. ; WERNER-FELMAYER ,G. ; FUCHS ,D. et al.** Tetrahydrobiopterin biosynthetic activities in human macrophages, fibroblasts, THP-1, and T 24 cells. GTP-cyclohydrolase I is stimulated by interferon-gamma, and 6-pyruvoyl tetrahydropterin synthase and sepiapterin reductase are constitutively present. *J. Biol. Chem.,* 1990, vol. 265, 3189-3192
- **WERNER, E.R. ; WERNER-FELMAYER, G. ; WACHTER, H.** Tetrahydrobiopterin and cytokines. *Proc. Soc. Exp. Biol. Med.,* 1993, vol. 203, 1-12
- **YEN, T.J. ; COMPTON, D.A. ; WISE, D. et al.** CENP-E, a novel human centromere-associated protein required for progression from metaphase to anaphase. *EMBO J.,* 1991, vol. 10, 1245-1254
- **KIMI ARAKI ; MASATAKE ARAKI ; KEN-ICHI YAMAMURA.** Target integration of DNA using munatn lox sites in embryonic stem cells. *Nucleic Acids Research,* 1997, vol. 25 (4), 868-872